# EUROPEAN PATENT APPLICATION

(11) **EP 4 563 161 A1**
(43) Date of publication of application: **04.06.2025**
(21) Application number: 23846251.9
(22) Date of filing: 13.07.2023
(51) Int. Cl.: A61K 47/32, A61K 8/02, A61K 8/34, A61K 8/41, A61K 8/73, A61K 8/81, A61K 9/06, A61K 9/08, A61K 9/107, A61K 9/12, A61K 31/045, A61K 31/165, A61K 31/192, A61K 45/00, A61K 47/10, A61K 47/18, A61K 47/36, A61K 47/38, A61P 17/00, A61P 17/04

(54) **COMPOSITION FOR EXTERNAL PREPARATION**

(30) Priority: 28.07.2022 JP 2022120146; 29.05.2023 JP 2023087463
(71) Applicant: Kao Corporation, Tokyo 103-8210 (JP)
(72) Inventor: MATSUI Shogo, Tokyo 131-8501 (JP); OKUBO Emi, Tokyo 131-8501 (JP); SAKAGUCHI Daishi, Tokyo 131-8501 (JP)
(74) Representative: Hoffmann Eitle
(86) International application number: PCT/JP2023/025839
(87) International publication number: WO 2024/024520

(57) **Abstract**

Provided is a composition for external preparation, containing the following components (A) to (D): (A) a medicinal ingredient; (B) a water-insoluble polymer; (C) a non-volatile base; and (D) a volatile solvent. The component (B) contains two or more selected from the group consisting of (B1) a cellulosic polymer, (B2) an acrylic polymer, and (B3) a vinyl polymer. The composition for external preparation has a viscosity at 25°C of 1.0 mPa·s or more and 10,000 mPa·s or less.

## Description

### Field of the Invention

The present invention relates to a composition for external preparation.

### Background of the Invention

A liquid skin external preparation and a patch containing a medicinal ingredient such as an anti-inflammatory analgesic component and a bactericidal disinfectant component are known. However, existing liquid skin external preparations have problems that they are easily rubbed off by clothes after being applied to the skin, and it is difficult to sustainably release the medicinal ingredients into the skin. In the case of a patch containing a medicinal ingredient, when the patch does not easily follow the skin and is easily peeled off, it is difficult to sustainably release the medicinal ingredient to the skin. On the other hand, a patch having high adhesiveness to the skin also has a problem that a burden on the skin increases when the patch is peeled off from the skin. In addition, a patch has problems in practical use such as a tendency to cause rash and a concern about the appearance when the patch is attached to the skin.

Therefore, a film-forming formulation capable of sustainably releasing a medicinal ingredient into the skin has been investigated. Since a film-forming formulation can form a coating film when applied to the skin, it is less likely to rub off than a liquid skin external preparation. In addition, even in a case where the film-forming formulation is applied to the skin, it does not stand out as in a patch, and it is preferable from the viewpoint that the burden on the skin is small in a case where the film-forming formulation is removed from the skin.

For example, JP 2021-6522 A (PTL 1) discloses that a film-forming skin external composition containing a film-forming polymer containing a cellulosic polymer and a vinyl polymer, a polyhydric alcohol, and a volatile solvent can form a film that protects an affected part and firmly adheres to the skin, has high flexibility, thereby has excellent feeling of use without uncomfortable feeling, is resistant to movement and rubbing, and is difficult to peel off, and also has excellent application property.

WO 2022/059618 (PTL 2) discloses that a film-forming aerosol composition containing a stock solution containing a film-forming agent and an organic solvent having a predetermined boiling point, and a propellant, in which an alkyl acrylate-vinyl acetate copolymer is contained as the film-forming agent, has excellent skin protection properties, and can form a film which is excellent in both removability after application and water resistance against sweat on the skin.

### Summary of the Invention

The present invention relates to the following.
[1] A composition for external preparation, containing the following components (A) to (D):
   (A) a medicinal ingredient;
   (B) a water-insoluble polymer;
   (C) a non-volatile base; and
   (D) a volatile solvent,
   in which the component (B) contains two or more selected from the group consisting of (B1) a cellulosic polymer, (B2) an acrylic polymer, and (B3) a vinyl polymer, and the composition for external preparation has a viscosity at 25°C of 1.0 mPa·s or more and 10,000 mPa·s or less.
[2] A method for using the composition for external preparation as set forth in [1], including the following steps (I) and (II) in this order:
   step (I): applying the composition for external preparation to an object and then drying the composition to form a coating film composed of the composition for external preparation; and
   step (II): bringing the coating film into contact with a cleaning agent and then washing with water to remove the coating film.

### Detailed Description of the Invention

As described above, PTL 1 describes the durability of the coating film to be formed, and PTL 2 describes the resistance of the coating film to sweat, but the durability and moisture resistance of the coating film by these conventional techniques are not sufficient for practical use, and there is room for further improvement.

The present invention relates to a composition for external preparation containing a medicinal ingredient and capable of forming a coating film having excellent durability and moisture resistance.

The present inventors have found that a composition for external preparation containing a medicinal ingredient, two or more predetermined water-insoluble polymers, and a predetermined solvent component, and having a predetermined viscosity can solve the above problems.

According to the present invention, it is possible to provide a composition for external preparation which contains a medicinal ingredient and is capable of forming a coating film having excellent durability and moisture resistance, and a method for using the same.

### [Composition for External Preparation]

The present invention relates to a composition for external preparation, containing the following components (A) to (D):
(A) a medicinal ingredient;
(B) a water-insoluble polymer;
(C) a non-volatile base; and
(D) a volatile solvent,
in which the component (B) contains two or more selected from the group consisting of (B1) a cellulosic polymer, (B2) an acrylic polymer, and (B3) a vinyl polymer, and the composition for external preparation has a viscosity at 25°C of 1.0 mPa·s or more and 10,000 mPa·s or less. Hereinafter, the composition is also referred to as "the composition of the present invention" as appropriate.

### <Definitions>

In the description herein, the term "composition for external preparation" refers to a composition mainly applied to skin surfaces.

In the description herein, the term "containing X" includes those in which X is blended.

In the description herein, the term "water-insoluble polymer" refers to a polymer in which 1 g of the polymer is immersed in 10 g of ion-exchange water under an environment of 23°C and 1 atmosphere, and after 24 hours have elapsed, more than 0.5 g of the immersed polymer does not dissolve.

In the description herein, the term "non-volatile base" refers to a base that is in a liquid state at 70°C, which has a mass loss rate of less than 1% when 1 g of the base is spread on a glass Petri dish having a diameter of 48 mm and allowed to stand at 25°C and atmospheric pressure for 24 hours.

The term "volatile solvent" refers to a component other than water that is liquid at 25°C and has a mass loss rate of 1% or more when 1 g of the solvent is spread on a glass Petri dish having a dimeter of 48 mm and allowed to stand at 25°C and atmospheric pressure for 24 hours.

In addition, in the description herein, when a composition for external preparation is applied to an object (the skin of a subject) to form a coating film, and the coating film is not easily rubbed off even when the subject performs daily living activities, it is referred to as "the durability of the coating film is high". In addition, the fact that wrinkling or peeling of the coating film is less likely to occur even when rubbing occurs under high temperature and high humidity is referred to as "the moisture resistance of the coating film is high". Specifically, the durability and moisture resistance of the coating film can be evaluated by the method described in Examples.

The composition of the present invention contains the component (A): a medicinal ingredient, the component (B): a water-insoluble polymer, the component (C): a non-volatile base, and the component (D): a volatile solvent, in which the component (A) is a component not contained in any of the other component (B), component (C), and component (D), and the component (B) is a component not contained in any of the component (C) and the component (D).

By having the above-described configuration, the composition of the present invention can form a coating film which contains a medicinal ingredient and has excellent durability and moisture resistance. The reason therefor is not clear, but is considered as follows.

The composition of the present invention is a composition having film-forming properties, which contains, as coating film-forming components, a water-insoluble polymer which is a component (B) and a non-volatile base which is a component (C). When the composition of the present invention is applied to the skin and then dried, the component (D) is volatilized to form a coating film containing the components (A) to (C).

Since the component (B) is water-insoluble and can form a highly hydrophobic coating film, it is considered that the durability and moisture resistance of the coating film are likely to be improved, and the stickiness of the coating film can also be suppressed. On the other hand, when only the component (B) is used as the coating film-forming component, there is a concern that the coating film becomes brittle and the durability decreases. In the present invention, it is considered that these problems can be suppressed by using the component (B) and the component (C) in combination.

Furthermore, it has been found that when only one kind of water-insoluble polymer is used as the component (B), or when only the same kind of polymers are used even when two or more kinds of water-insoluble polymers are used, it is difficult to realize not only the durability of the coating film but also the moisture resistance at a high level at which wrinkling and peeling hardly occur even when the coating film is rubbed under high temperature and high humidity. In the present invention, it is considered that the above problems can be solved by using two or more kinds of specific and different water-insoluble polymers as the component (B).

It is considered that the component (D) contributes to improvement of quick-drying property of the composition for external preparation, and an effect of suppressing crystallization of the component (A). By suppressing the crystallization of the component (A) in the coating film, the component (A), which is a medicinal ingredient, can be effectively sustained-released to an object. Furthermore, when the composition for external preparation contains the component (D), the viscosity can be easily adjusted to a predetermined range. As a result, it is considered that the application property and the film-forming properties of the composition for external preparation are improved, and the durability and the moisture resistance of the coating film to be formed are also further improved.

In addition, the coating film formed from the composition of the present invention is less sticky, has excellent durability and moisture resistance during use, and also has effects of capable of being easily removed by washing with water using a cleaning agent after use (hereinafter, also referred to as "removability of coating film").

### <Component (A): Medicinal Ingredient>

The composition of the present invention contains a medicinal ingredient as a component (A). From the viewpoint of use in the composition for external preparation, it is preferable that the component (A) is a skin external drug ingredient that can be administered transdermally. Specifically, it is preferable that the component (A) contains one or more selected from the group consisting of an anti-inflammatory analgesic component, a local irritation component, a blood circulation promoting component, an antihistaminic component, a crude drug component, an antipruritic component, a local anesthetic component, a keratin softening component, a bactericidal component, an antibacterial and antifungal component, an immunosuppressive agent, an antiviral component, a hair growth and hair restoration component, a sebum inhibitory component, an antiperspirant component, and a whitening component, and it is more preferable that the component (A) contains one or more selected from the group consisting of an anti-inflammatory analgesic component, an antihistaminic component, an antipruritic component, a bactericidal component, an antibacterial and antifungal component, an immunosuppressive agent, an antiviral component, a hair growth and hair restoration component, a sebum inhibitory component, an antiperspirant component, and a whitening component.

Examples of the anti-inflammatory analgesic component include glycol salicylate, methyl salicylate, aspirin, sulpyrine hydrate, acetaminophen, diclofenac sodium, fenbufen, ibuprofen, alminoprofen, loxoprofen sodium hydrate, naproxen, oxaprofen, ketoprofen, tiaprofenic acid, sulindac, flufenamate aluminum, felbinac, mefenamic acid, indometacin, indometacin farnesil, acemetacin, proglumetacin maleate, bendazac, piroxicam, ampiroxicam, lornoxicam, tenoxicam, meloxicam, etodolac, tiaramide hydrochloride, bucolome, flurbiprofen, esflurbiprofen, lysozyme hydrochloride, bromelain, diphenhydramine hydrochloride, dibucaine, dimethylisopropylazulene, benzethonium chloride, glycyrrhizic acid, dipotassium glycyrrhizinate, zinc oxide, allantoin, heparinoid, glycyrrhetinic acid, ibuprofen piconol, fluocinolone acetonide, difluprednate, clobetasol propionate, and betamethasone valerate. Among these, one or more selected from the group consisting of glycol salicylate, methyl salicylate, diclofenac sodium, loxoprofen sodium hydrate, ketoprofen, indometacin, piroxicam, flurbiprofen, esflurbiprofen, glycyrrhizic acid, dipotassium glycyrrhizinate, zinc oxide, allantoin, heparinoid, glycyrrhetinic acid, ibuprofen piconol, fluocinolone acetonide, difluprednate, clobetasol propionate, and betamethasone valerate are preferable.

Examples of the local irritation component include l-menthol, dl-camphor, nonylic acid vanillylamide, ammonia, and peppermint oil. Among these, one or more selected from the group consisting of l-menthol, dl-camphor, and nonylic acid vanillylamide are preferable.

Examples of the blood circulation promoting component include benzyl nicotinate, sodium polyethylenesulfonate, tocopherol acetate, and carpronium chloride hydrate. Among these, one or more selected from the group consisting of tocopherol acetate and carpronium chloride hydrate are preferable.

Examples of the antihistaminic component include diphenhydramine, diphenhydramine hydrochloride, diphenhydramine salicylate, and chlorpheniramine maleate. Among these, one or more selected from the group consisting of diphenhydramine, diphenhydramine hydrochloride, and chlorpheniramine maleate are preferable.

Examples of the crude drug component include capsicum, arnica tincture, eucalyptus oil, Phellodendron Bark, arnica tincture, Houttuynia herb, Chinese peony, Artemisia princeps, Chinese cinnamon, Cnidium officinale, Cyperus Rhizome, Atractylodes lancea rhizome, Illicium verum, Schisandra repanda, Citrus Unshiu Peel, ginseng, magnolol, clove, ginger, gardeniae fructus, Glycyrrhiza, Fennel, akebia stem, thistle saffron, Magnolia Bark, bitter orange peel, and Artemisiae capillaris flos. Among these, one or more selected from the group consisting of capsicum and arnica tincture are preferable.

Examples of the antipruritic component include crotamiton, cortisone acetate, isothipendyl hydrochloride, benzalkonium chloride, calamine, d-borneol, ammonium water, hydrocortisone, hydrocortisone acetate, hydrocortisone butyrate, dexamethasone, dexamethasone acetate, prednisolone, prednisolone acetate, prednisolone valerate acetate, and ufenamate. Among these, one or more selected from the group consisting of crotamiton, benzalkonium chloride, hydrocortisone, hydrocortisone acetate, hydrocortisone butyrate, dexamethasone, dexamethasone acetate, prednisolone, prednisolone valerate acetate, and ufenamate are preferable.

Examples of the local anesthetic component include lidocaine, mepivacaine, bupivacaine, ropivacaine, levobupivacaine, dibucaine, dibucaine hydrochloride, and ethyl aminobenzoate. Among these, one or more selected from the group consisting of lidocaine and dibucaine hydrochloride are preferable.

Examples of the keratin softening component include urea, sulfur, and salicylic acid.

Examples of the bactericidal component include chlorhexidine gluconate, sodium copper chlorophyllin, isopropylmethylphenol, cetylpyridinium chloride hydrate, benzethonium chloride, benzalkonium chloride, resorcin, acrinol hydrate, chlorhexidine gluconate, homosulfamine, homosulfamine hydrochloride, povidone iodine, iodine-potassium iodide, mercurochrome, oxydol, cresol, iodoform, thymol, chlorhexidine hydrochloride, adapalene, and trichlorocarbanilide. Among these, one or more selected from the group consisting of isopropylmethylphenol, benzethonium chloride, resorcin, homosulfamine, homosulfamine hydrochloride, chlorhexidine hydrochloride, adapalene, and trichlorocarbanilide are preferable.

Examples of the antibacterial and antifungal component include undecylenic acid, zinc undecylenate, phenyl-11-iodo-10-undecynoate, exalamide, clotrimazole, econazole nitrate, miconazole nitrate, tioconazole, zinc diethyldithiocarbamate, ciclopiroxolamine, siccanin, trichomycin, pyrrolnitrin, thianthol, 2,4,6-tribromophenyl caproate, trimethylcetylammonium pentachlorophenate, tolciclate, tolnaftate, haloprogin, althea bark, berberine benzoate, dequalinium chloride, chlorhexidine hydrochloride, chlorhexidine gluconate solution, dequalinium acetate, hinokitiol, resorcin, benzoic acid, chlorobutanol, acetic acid, phenol, iodine tincture, diphenylpyraline hydrochloride, diphenhydramine salicylate, diphenylimidazole, chlorpheniramine maleate, dibucaine hydrochloride, procaine hydrochloride, lidocaine hydrochloride, aldioxa, glycyrrhizic acid and its salts, lithospermum root, Angelica acutiloba, borneol, diethyl phthalate, chlorohydroxy aluminum, penicillins, cephems, carbapenems, monobactams, penams, aminoglycosides, fosfomycins, chloramphenicols, macrolides, glycopeptides, quinolones, new quinolones, sulfa drug, fradiomycin sulfate, gentamicin sulfate, pentamidine isethionate, silver sulfadiazine, oxiconazole nitrate, sulconazole nitrate, bifonazole, neticonazole hydrochloride, lanoconazole, amorolfine hydrochloride, terbinafine hydrochloride, butenafine hydrochloride, polymixin B sulfate, colistin sulfate, bacitracin, fradiomycin sulfate, benzoyl peroxide, nadifroxacin, levofloxacin, and clindamycin phosphate. Among these, one or more selected from the group consisting of clotrimazole, miconazole nitrate, pyrrolnitrin, tolnaftate, hinokitiol, fradiomycin sulfate, oxiconazole nitrate, bifonazole, lanoconazole, terbinafine hydrochloride, and butenafine hydrochloride are preferable.

Examples of the immunosuppressive agent include tacrolimus.

Examples of the antiviral component include acyclovir and vidarabine.

Examples of the hair growth and hair restoration component include minoxidil, panthenol, and pantothenyl ethyl ether.

Examples of the sebum inhibitory component include pyridoxine hydrochloride.

Examples of the antiperspirant component include aluminum chloride, glycopyrronium tosilate hydrate, and sofpironium bromide.

Examples of the whitening component include tranexamic acid, tretinoin, and hydroquinone.

As the component (A), one or more of the above-mentioned medicinal ingredients can be used.

Among the above, it is more preferable that the component (A) contains one or more selected from the group consisting of glycol salicylate, methyl salicylate, diclofenac sodium, loxoprofen sodium hydrate, ketoprofen, indometacin, flurbiprofen, dipotassium glycyrrhizinate, zinc oxide, allantoin, heparinoid, glycyrrhetinic acid, Ibuprofen piconol, fluocinolone acetonide, l-menthol, dl-camphor, nonylic acid vanillylamide, tocopherol acetate, carpronium chloride hydrate, diphenhydramine, diphenhydramine hydrochloride, chlorpheniramine maleate, capsicum, crotamiton, benzalkonium chloride, hydrocortisone, hydrocortisone butyrate, dexamethasone acetate, prednisolone valerate acetate, betamethasone valerate, ufenamate, lidocaine, dibucaine hydrochloride, urea, sulfur, salicylic acid, isopropylmethylphenol, benzethonium chloride, resorcin, homosulfamine, homosulfamine hydrochloride, chlorhexidine hydrochloride, trichlorocarbanilide, clotrimazole, miconazole nitrate, tolnaftate, hinokitiol, oxiconazole nitrate, bifonazole, lanoconazole, terbinafine hydrochloride, butenafine hydrochloride, tacrolimus, acyclovir, vidarabine, minoxidil, panthenol, pantothenyl ethyl ether, pyridoxine hydrochloride, tranexamic acid, tretinoin, homosulfamine hydrochloride, aluminum chloride, glycopyrronium tosilate hydrate, and sofpironium bromide.

### <Component (B): Water-Insoluble Polymer>

The composition of the present invention contains a water-insoluble polymer as a component (B). The component (B) is a film-forming component, and is considered to improve the film-forming properties of the composition and enable the formation of a film having high durability and moisture resistance. Furthermore, even in a case where the component (A) having high crystallinity is used, an effect is also exerted that the crystallization of the component (A) in the coating film can be suppressed, and the formed film is less sticky and can be easily removed by washing with water using a cleaning agent.

From the viewpoint of improving the film-forming properties, it is preferable that the component (B) is a film-forming water-insoluble polymer. The definition of "water-insoluble" of the component (B) is as described above.

The component (B) contains two or more selected from the group consisting of (B1) a cellulosic polymer, (B2) an acrylic polymer, and (B3) a vinyl polymer.

In the description herein, the phrase "the component (B) contains two or more selected from the group consisting of (B1) a cellulosic polymer, (B2) an acrylic polymer, and (B3) a vinyl polymer" specifically means any of the following aspects (i) to (iv):
(i) the component (B) contains the component (B1) and the component (B2) and does not contain the component (B3);
(ii) the component (B) contains the component (B1) and the component (B3) and does not contain the component (B2);
(iii) the component (B) contains the component (B2) and the component (B3) and does not contain the component (B 1); and
(iv) the component (B) contains the component (B1), the component (B2), and the component (B3).

Therefore, for example, a case where the component (B) contains two or more kinds of the component (B1) but does not contain any of the component (B2) and the component (B3) is not included in the scope of the present invention.

Among the above aspects, from the viewpoint of improving the durability and moisture resistance of the coating film, from the viewpoint of improving the effect of suppressing crystallization of the component (A), and from the viewpoint of suppressing the stickiness of the coating film, the aspect (i) or (iv) is preferable, and the aspect (i) is more preferable.

### (Component (B 1): Cellulosic Polymer)

Examples of the cellulosic polymer used as the component (B1) include water-insoluble polymers among polymers having a cellulose skeleton.

Specific examples of the component (B1) include water-insoluble polymers among methyl cellulose, ethyl cellulose, hypromellose, carboxymethyl cellulose, carboxymethyl ethyl cellulose, hydroxyethyl cellulose, hydroxypropyl cellulose, hypromellose phthalate, hydrophobized (C16-C18) hydroxypropyl methyl cellulose, hypromellose acetate succinate, and cellulose acetate phthalate, and one kind or two or more kinds thereof can be used.

From the viewpoint of improving the durability and the moisture resistance of the coating film, from the viewpoint of improving the effect of suppressing crystallization of the component (A), and from the viewpoint of suppressing the stickiness of the coating film, as the component (B1), one or more kinds selected from the group consisting of ethyl cellulose, hypromellose phthalate, and hydrophobized (C16-C18) hydroxypropyl methyl cellulose are preferable, and hypromellose phthalate is more preferable.

As the component (B1), a commercially available product can also be used. For example, examples of the ethyl cellulose used as the component (B1) include "ETHOCEL 100P" manufactured by Nisshin Kasei Co., Ltd., and examples of the hypromellose phthalate include "HPMCP HP-55", "HPMCP HP-55S", and "HPMCP HP-50" manufactured by Shin-Etsu Chemical Co., Ltd.

### (Component (B2): Acrylic Polymer)

Examples of the acrylic polymer used as the component (B2) include water-insoluble polymers among polymers containing at least a constituent unit derived from a monomer having a (meth)acrylic group. In the description herein, the term "(meth)acryl" means both acryl and methacryl.

Specific examples of the component (B2) include water-insoluble polymers among acrylates/diacetoneacrylamide copolymer, acrylamide-methoxypolyethylene glycol methacrylate copolymer, alkyl acrylate copolymer, acrylate-vinyl acetate copolymer, 2-ethylhexyl acrylate-methyl acrylate-acrylic acid-glycidyl methacrylate copolymer, 2-ethylhexyl acrylate-vinyl acetate-hydroxyethyl acrylate-glycidyl methacrylate copolymer, 2-ethylhexyl acrylate-diacetoneacrylamide-acetoacetoxyethyl methacrylate-methyl methacrylate copolymer, 2-ethylhexyl acrylate-vinylpyrrolidone copolymer, 2-ethylhexyl acrylate-2-ethylhexyl methacrylate-dodecyl methacrylate copolymer, ethyl acrylate-methyl methacrylate copolymer, ethyl acrylate-methyl methacrylate-trimethylammonium ethyl methacrylate chloride copolymer, and methyl methacrylate-butyl methacrylate-dimethylaminoethyl methacrylate copolymer, and one kind or two or more kinds thereof can be used.

Among the above, as the acrylates/diacetoneacrylamide copolymer, the cosmetic ingredient label name "AMP-acrylates/diacetoneacrylamide copolymer" can be used; as the acrylamide-methoxypolyethylene glycol methacrylate copolymer, "acrylamide-methoxypolyethylene glycol methacrylate copolymer solution" described in Japanese Pharmaceutical Excipients 2018 (Pharmaceutical Evaluation and Licensing Division, Pharmaceutical Safety and Environmental Health Bureau, Ministry of Health, Labor and Welfare) can be used; as the alkyl acrylate copolymer, a cosmetic ingredient label name "alkyl acrylate copolymer ammonium" can be used; as the 2-ethylhexyl acrylate-vinylpyrrolidone copolymer, "2-ethylhexyl acrylate-vinylpyrrolidone copolymer solution" described in Japanese Pharmaceutical Excipients 2018 (Pharmaceutical Evaluation and Licensing Division, Pharmaceutical Safety and Environmental Health Bureau, Ministry of Health, Labor and Welfare) can be used; as the 2-ethylhexyl acrylate-2-ethylhexyl methacrylate-dodecyl methacrylate copolymer, "2-ethylhexyl acrylate-2-ethylhexyl methacrylate-dodecyl methacrylate copolymer solution" can be used; as the ethyl acrylate-methyl methacrylate copolymer, "ethyl acrylate-methyl methacrylate copolymer dispersion liquid" can be used; and as the methyl methacrylate-butyl methacrylate-dimethylaminoethyl methacrylate copolymer, "aminoalkyl methacrylate copolymer E" can be used.

In addition, as the ethyl acrylate-methyl methacrylate-trimethylammoniumethyl methacrylate chloride copolymer, "Aminoalkyl Methacrylate Copolymer RS" known as a pharmaceutical additive can be used.

From the viewpoint of improving the durability and the moisture resistance of the coating film, from the viewpoint of improving the effect of suppressing crystallization of the component (A), and from the viewpoint of suppressing the stickiness of the coating film, the component (B2) preferably contains one or more kinds selected from the group consisting of acrylates/diacetoneacrylamide copolymer, alkyl acrylate copolymer, and acrylate-vinyl acetate copolymer, and the acrylates/diacetone acrylamide copolymer is more preferable.

As the component (B2), a commercially available product can also be used. Examples of the acrylates/diacetoneacrylamide copolymer used as the component (B2) include "Plascize L-53" manufactured by Goo Chemical Co., Ltd., examples of the alkyl acrylate-vinyl acetate copolymer include "VINYSOL 2140L" manufactured by Daido Chemical Corporation, and examples of the alkyl acrylate copolymer include "VINYSOL 1086WP" manufactured by Daido Chemical Corporation.

### (Component (B3): Vinyl Polymer)

Examples of the vinyl polymer used as the component (B3) include water-insoluble polymers among polymers which are polymers containing at least a constituent unit derived from a monomer having a vinyl group and do not belong to the acrylic polymer. Examples of the vinyl polymer include water-insoluble polymers among polyvinyl alcohol, polyvinyl acetate, and polyvinyl butyral.

From the viewpoint of improving the durability and the moisture resistance of the coating film, from the viewpoint of improving the effect of suppressing crystallization of the component (A), and from the viewpoint of suppressing the stickiness of the coating film, polyvinyl butyral is preferable as the component (B3).

As the component (B3), a commercially available product can also be used. Examples of the polyvinyl butyral used as the component (B3) include "S-LEC B·K" series manufactured by Sekisui Chemical Co., Ltd.

In a case where (i) the component (B) contains the component (B1) and the component (B2) and does not contain the component (B3), the component (B1) is preferably one or more selected from the group consisting of ethyl cellulose, hypromellose phthalate, and hydrophobized (C16-18) hydroxypropyl methyl cellulose, and more preferably hypromellose phthalate, and the component (B2) is preferably acrylates/diacetoneacrylamide copolymer.

In a case where (ii) the component (B) contains the component (B1) and the component (B3) and does not contain the component (B2), the component (B1) is preferably hypromellose phthalate, and the component (B3) is preferably polyvinyl butyral.

In a case where (iii) the component (B) contains the component (B2) and the component (B3) and does not contain the component (B1), the component (B2) is preferably acrylates/diacetoneacrylamide copolymer and the component (B3) is preferably polyvinyl butyral.

In a case where (iv) the component (B) contains the component (B1), the component (B2), and the component (B3), the component (B1) is preferably hypromellose phthalate, the component (B2) is preferably acrylates/diacetoneacrylamide copolymer, and the component (B3) is preferably polyvinyl butyral.

In a case where the component (B) contains the component (B1) and the component (B2), the mass ratio [(B2)/(B1)] of the component (B2) to the component (B1) is preferably 0.05 or more, more preferably 0.1 or more, still more preferably 0.2 or more, yet still more preferably 0.5 or more, and yet still more preferably 1.0 or more from the viewpoint of improving the durability and the moisture resistance of the coating film, and is preferably 30 or less, more preferably 20 or less, still more preferably 15 or less, yet still more preferably 12 or less, and yet still more preferably 10 or less from the viewpoint of improving the durability and the moisture resistance of the coating film and from the viewpoint of suppressing the stickiness of the coating film. Then, the mass ratio [(B2)/(B1)] of the component (B2) to the component (B1) is preferably 0.05 or more and 30 or less, more preferably 0.1 or more and 20 or less, still more preferably 0.2 or more and 15 or less, yet still more preferably 0.5 or more and 12 or less, and yet still more preferably 1.0 or more and 10 or less.

In a case where the component (B) contains the component (B1) and the component (B3), the mass ratio [(B3)/(B1)] of the component (B3) to the component (B1) is preferably 0.1 or more, more preferably 0.2 or more, still more preferably 0.5 or more, yet still more preferably 1.0 or more, and yet still more preferably 2.0 or more from the viewpoint of improving the durability and the moisture resistance of the coating film, and is preferably 20 or less, more preferably 15 or less, still more preferably 12 or less, yet still more preferably 10 or less, and yet still more preferably 8 or less from the viewpoint of improving the durability and the moisture resistance of the coating film and from the viewpoint of suppressing the stickiness of the coating film. Then, the mass ratio [(B3)/(B1)] of the component (B3) to the component (B1) is preferably 0.1 or more and 20 or less, more preferably 0.2 or more and 15 or less, still more preferably 0.5 or more and 12 or less, yet still more preferably 1.0 or more and 10 or less, and yet still more preferably 2.0 or more and 8 or less.

In a case where the component (B) contains the component (B2) and the component (B3), the mass ratio [(B2)/(B3)] of the component (B2) to the component (B3) is preferably 0.05 or more, more preferably 0.1 or more, still more preferably 0.2 or more, yet still more preferably 0.5 or more, and yet still more preferably 1.0 or more from the viewpoint of improving the durability and the moisture resistance of the coating film, and is preferably 30 or less, more preferably 20 or less, still more preferably 15 or less, yet still more preferably 12 or less, and yet still more preferably 10 or less from the viewpoint of improving the durability and the moisture resistance of the coating film and from the viewpoint of suppressing the stickiness of the coating film. Then, the mass ratio [(B2)/(B3)] of the component (B2) to the component (B3) is preferably 0.05 or more and 30 or less, more preferably 0.1 or more and 20 or less, still more preferably 0.2 or more and 15 or less, yet still more preferably 0.5 or more and 12 or less, and yet still more preferably 1.0 or more and 10 or less, from the viewpoint of the durability and the moisture resistance of the coating film.

The component (B) may contain a water-insoluble polymer other than the component (B1), the component (B2), and the component (B3) as long as the effects of the present invention are not impaired. However, the total content of the component (B1), the component (B2), and the component (B3) in the component (B) is preferably 50% by mass or more, more preferably 60% by mass or more, still more preferably 70% by mass or more, yet still more preferably 80% by mass or more, and yet still more preferably 90% by mass or more, and is 100% by mass or less, from the viewpoint of improving the durability and the moisture resistance of the coating film, from the viewpoint of improving the effect of suppressing crystallization of the component (A), and from the viewpoint of suppressing the stickiness of the coating film.

### <Component (C): Non-Volatile Base>

The composition of the present invention contains a non-volatile base (excluding those corresponding to the component (A)) as a component (C). It is considered that the combined use of the component (C) and the component (B) contributes to the improvement of the durability and the moisture resistance of the coating film and the improvement of the effect of suppressing crystallization of the component (A).

The definition of the "non-volatile base" of the component (C) is as described above.

Examples of the component (C) include a lipophilic base, a hydrophilic base, and an amphiphilic base, and one or more of these can be used.

### (Lipophilic Base)

Preferred examples of the lipophilic base include a polar oil and a non-polar oil.

Specific examples of the polar oil include a non-volatile synthetic ester oil, and more preferred examples thereof include one or more selected from the group consisting of (i) a fatty acid monoester composed of a fatty acid and a monohydric alcohol, (ii) a fatty acid monoester or diester composed of a fatty acid and a dihydric alcohol, (iii) a dicarboxylic acid diester composed of a dicarboxylic acid and a monohydric alcohol, (iv) a tricarboxylic acid triester composed of a tricarboxylic acid and a monohydric alcohol, and (v) a glycerol fatty acid triester.

Examples of (i) the fatty acid monoester composed of a fatty acid and a monohydric alcohol include monoesters of a saturated fatty acid having 8 or more and 22 or less carbon atoms and an aliphatic or aromatic ring-containing monohydric alcohol having 1 or more and 24 or less carbon atoms, and examples thereof include cetyl octanoate, cetyl 2-ethylhexanoate, ethyl laurate, hexyl laurate, isopropyl myristate, myristyl myristate, hexadecyl myristate, 2-hexyldecyl myristate, octyldodecyl myristate, isopropyl palmitate, ethylhexyl palmitate, hexadecyl palmitate, 2-hexyldecyl palmitate, 2-heptylundecyl palmitate, butyl stearate, 2-ethylhexyl stearate, isocetyl stearate, isocetyl isostearate, isononyl isononanoate, isotridecyl isononanoate, and hexyldecyl dimethyloctanoate.

Examples of (ii) the fatty acid monoester or diester composed of a fatty acid and a dihydric alcohol include monoesters or diesters of a saturated fatty acid having 8 or more and 22 or less carbon atoms and an aliphatic or aromatic dihydric alcohol having 1 or more and 12 or less carbon atoms, and examples thereof include propylene glycol monocaprate, ethylene glycol di-2-ethylhexanoate, ethylene glycol dilaurate, ethylene glycol distearate, neopentyl glycol dicaprate, neopentyl glycol diethylhexanoate, propanediol di(caprylate/caprate), and propanediol diisostearate.

Examples of (iii) the dicarboxylic acid diester composed of a dicarboxylic acid and a monohydric alcohol include diesters of an aliphatic or aromatic dicarboxylic acid having 4 or more and 18 or less carbon atoms and an aliphatic or aromatic ring-containing monohydric alcohol having 1 or more and 24 or less carbon atoms, and examples thereof include 2-ethylhexyl succinate, diisopropyl adipate, dibutyl adipate, diisobutyl adipate, 2-hexyldecyl adipate, di-2-heptylundecyl adipate, diethyl sebacate, diisopropyl sebacate, di-2-ethylhexyl sebacate, diisononyl phthalate, and diisostearyl malate.

Examples of (iv) the tricarboxylic acid triester composed of a tricarboxylic acid and a monohydric alcohol include triesters of an aliphatic or aromatic tricarboxylic acid having 5 or more and 12 or less carbon atoms and an aliphatic or aromatic ring-containing monohydric alcohol having 1 or more and 24 or less carbon atoms, and examples thereof include triisodecyl trimellitate.

Examples of (v) the glycerol fatty acid triester include triesters of glycerol and a saturated fatty acid having 8 or more and 22 or less carbon atoms, and examples thereof include glycerol tri-2-ethylhexanoate, glycerol trimyristate, glycerol tri-2-heptylundecanoate, and tristearin.

As the ester oil other than the above-mentioned (i) to (v), alkyl benzoate, cetyl lactate, myristyl lactate, lanolin acetate, dipentaerythritol fatty acid ester, glycerol di-2-heptylundecanoate, castor oil fatty acid methyl ester, or N-lauroyl-L-glutamic acid-2-octyldodecyl can be used.

Among the above, the ester oil is more preferably one or more selected from the group consisting of (i) a fatty acid monoester composed of a fatty acid and a monohydric alcohol, and (ii) a fatty acid monoester or diester composed of a fatty acid and a dihydric alcohol, still more preferably one or more selected from the group consisting of a fatty acid monoester composed of a fatty acid and a monohydric alcohol having 1 or more and 24 or less carbon atoms, and a monoester or diester composed of a saturated fatty acid having 8 or more and 22 or less carbon atoms and an aliphatic or aromatic dihydric alcohol having 1 or more and 12 or less carbon atoms, and yet still more preferably one or more selected from the group consisting of isopropyl myristate and propylene glycol monocaprate.

Specific examples of the non-polar oil include non-volatile hydrocarbon oils, silicone oils, and fluorine oils. Examples of the hydrocarbon oil include liquid paraffin and squalane; examples of the silicone oil include dimethylpolysiloxane, dimethylcyclopolysiloxane, methylphenylpolysiloxane, methylhydrogenpolysiloxane, and higher alcohol-modified organopolysiloxane; and examples of the fluorine oil include fluoropolyether and perfluoroalkyl ether silicone.

### (Hydrophilic Base)

Examples of the hydrophilic base preferably include one or more selected from the group consisting of a polyol and a lower amine.

Specific examples of the polyol include non-volatile, alkylene glycols, polyalkylene glycols, and glycerols. Examples of the alkylene glycols include ethylene glycol, propylene glycol, 1,3-propanediol, 1,3-butylene glycol (1,3-butanediol), and 1,2-pentanediol; examples of the polyalkylene glycols include diethylene glycol, dipropylene glycol, polyethylene glycol, polypropylene glycol, and polyoxyethylene-polyoxypropylene glycol; and examples of the glycerols include glycerol, diglycerol, and triglycerol. The polyethylene glycol, polypropylene glycol, and polyoxyethylene-polyoxypropylene glycol preferably have a weight-average molecular weight of less than 10,000.

In the description herein, the term "lower amine" refers to an amine having preferably 9 or less carbon atoms, and more preferably 2 or more and 9 or less carbon atoms. The lower amine is preferably an alkanolamine from the viewpoint of nonvolatility, from the viewpoint of improving the durability and the moisture resistance of the coating film, from the viewpoint of improving the effect of suppressing crystallization of the component (A), and from the viewpoint of suppressing the stickiness of the coating film.

Specific examples of the alkanolamine include triethanolamine, diethanolamine, monoethanolamine, triisopropanolamine, diisopropanolamine, monoisopropanolamine, and 2-amino-2-methylpropanol.

### (Amphiphilic Base)

Examples of the amphiphilic base preferably include an ionic or nonionic surfactant, and a nonionic surfactant is preferable.

Specific examples of the nonionic surfactant include polyoxyethylene alkyl ether, polyoxyethylene alkenyl ether, polyoxyethylene sorbitan fatty acid ester, polyoxyethylene sorbit fatty acid ester, polyoxyethylene fatty acid ester, alkyl glucoside, alkyl glyceryl ether, fatty acid sucrose ester, polyoxyethylene hydrogenated castor oil, alkyl saccharide, alkylamine oxide, and alkylamidoamine oxide.

The alkyl group in the polyoxyethylene alkyl ether, alkyl glucoside, alkyl glyceryl ether, alkyl saccharide, alkylamine oxide, and alkylamidoamine oxide, the alkenyl group in the polyoxyethylene alkenyl ether, and the fatty acid in the polyoxyethylene sorbitan fatty acid ester, polyoxyethylene fatty acid ester, and fatty acid sucrose ester preferably have 8 or more and 22 or less carbon atoms, more preferably 10 or more and 20 or less carbon atoms, and still more preferably 12 or more and 18 or less carbon atoms.

Among the above, as the nonionic surfactant, one or more selected from the group consisting of polyoxyethylene sorbitan fatty acid ester, polyoxyethylene sorbit fatty acid ester, polyoxyethylene fatty acid ester, alkyl glucoside, and alkyl glyceryl ether are preferable, and polyoxyethylene sorbitan fatty acid ester is more preferable.

Specific examples of the polyoxyethylene sorbitan fatty acid ester include polyoxyethylene sorbitan monolaurate (20E.O.) (Polysorbate 20), polyoxyethylene sorbitan monostearate (20E.O.) (Polysorbate 60), polyoxyethylene sorbitan tristearate (20E.O.) (Polysorbate 65), and polyoxyethylene sorbitan oleate (20E.O.) (Polysorbate 80), and preferably polyoxyethylene sorbitan oleate (20E.O.) (Polysorbate 80).

As the component (C), one or two or more can be used.

Among the above, the component (C) is preferably one or more selected from the group consisting of a polar oil, a non-polar oil, a polyol, an alkanolamine, and a nonionic surfactant, more preferably one or more selected from the group consisting of isopropyl myristate, propylene glycol monocaprate, squalane, 1,3-butylene glycol, dipropylene glycol, polyethylene glycol having a weight-average molecular weight of less than 1,000, triethanolamine, diisopropanolamine, 2-amino-2-methylpropanol, and polyoxyethylene sorbitan fatty acid ester, and still more preferably contains one or more selected from the group consisting of isopropyl myristate, 1,3-butylene glycol, diisopropanolamine, 2-amino-2-methylpropanol, and polyoxyethylene sorbitan fatty acid ester.

The amine which is the component (C) also includes an amine constituting a neutralizing salt of the water-insoluble polymer which is the component (B).

For example, in the composition of the present invention, as a commercially available product containing the component (B) and alkanolamine as the component (C), "Plascize L-53P" manufactured by Goo Chemical Co., Ltd., which contains a partial neutralization product of acrylates/diacetoneacrylamide copolymer with 2-amino-2-methylpropanol can be used.

### <Component (D): Volatile Solvent>

The composition of the present invention contains a volatile solvent (excluding those corresponding to the component (A)) as a component (D). The component (D) contributes to improving the quick-drying property of the composition, and the effect of suppressing crystallization of the component (A). Furthermore, it is considered that the viscosity of the composition for external preparation is easily adjusted to a predetermined range, so that the application property and the film-forming properties are improved, and the durability and the moisture resistance of the coating film are also further improved.

The definition of the "volatile solvent" of the component (D) is as described above.

Examples of the component (D) include an alcohol, a ketone, an ester, a hydrocarbon, and a silicone, having volatility.

The alcohol used as the component (D) is preferably a lower alcohol. The "lower alcohol" is preferably an alcohol having 4 or less carbon atoms, and examples thereof include methanol, ethanol, n-propyl alcohol, isopropyl alcohol, n-butyl alcohol, isobutyl alcohol, sec-butyl alcohol, and tert-butyl alcohol.

Examples of the ketone used as the component (D) include acetone, ethyl methyl ketone, and methyl isobutyl ketone; examples of the ester include methyl acetate, ethyl acetate, and butyl acetate; examples of the hydrocarbon include volatile liquid paraffin; and examples of the silicone include linear polydimethylsiloxane and cyclic siloxane.

As the component (D), one kind or two or more kinds can be used.

From the viewpoint of improving the application property and the quick-drying property of the composition, from the viewpoint of the solubility or dispersibility of the components (A) to (C), and from the viewpoint of improving the effect of suppressing crystallization of the component (A), the component (D) is preferably an alcohol having 4 or less carbon atoms, and more preferably one or more selected from the group consisting of ethanol and isopropyl alcohol.

### <Water>

The composition for external preparation preferably further contains water from the viewpoint of improving the effect of suppressing crystallization of the component (A) and from the viewpoint of improving the removability of the coating film. The water used in the present invention is not particularly limited, and for example, ion-exchanged water, pure water, and distilled water can be used.

### <Water-Soluble Polymer>

The composition for external preparation may further contain a water-soluble polymer as long as the effects of the present invention are not impaired. The water-soluble polymer is a water-soluble polymer that does not correspond to any of the components (A) to (D), and examples thereof include a carboxyvinyl polymer, polyacrylic acid, and polyvinylpyrrolidone.

However, from the viewpoint of improving the durability and the moisture resistance of the coating film and from the viewpoint of suppressing the stickiness of the coating film, the mass ratio of the water-soluble polymer to the component (B) [water-soluble polymer/component (B)] is preferably low. Specifically, the mass ratio of the water-soluble polymer to the component (B) [water-soluble polymer/component (B)] in the composition for external preparation is preferably 2 or less, more preferably 1.5 or less, still more preferably 1.3 or less, yet still more preferably 1 or less, yet still more preferably 0.5 or less, yet still more preferably 0.1 or less, and yet still more preferably 0.

### <Other Components>

The composition for external preparation of the present invention may appropriately contain, in addition to the above-mentioned components, other components usually blended in a composition for external preparation within a range not impairing the object of the present invention. Examples of the component include an organic acid or an inorganic acid, an antioxidant, an ultraviolet absorber, a preservative, a pH adjuster other than an organic acid or an inorganic acid, a perfume, and a pigment.

### <Content>

The content of each component in the composition for external preparation is preferably as follows from the viewpoint of improving the durability and the moisture resistance of the coating film, from the viewpoint of improving the effect of suppressing crystallization of the component (A), and from the viewpoint of suppressing the stickiness of the coating film.

The content of the component (A) in the composition for external preparation is preferably 0.001% by mass or more, more preferably 0.005% by mass or more, still more preferably 0.01% by mass or more, and yet still more preferably 0.02% by mass or more from the viewpoint of sufficiently exhibiting the efficacy of the component (A), and is preferably 30% by mass or less, more preferably 20% by mass or less, still more preferably 15% by mass or less, and yet still more preferably 10% by mass or less from the viewpoint of improving the effect of suppressing crystallization of the component (A). Then, the content of the component (A) in the composition for external preparation is preferably 0.001% by mass or more and 30% by mass or less, more preferably 0.005% by mass or more and 20% by mass or less, still more preferably 0.01% by mass or more and 15% by mass or less, and yet still more preferably 0.02% by mass or more and 10% by mass or less.

When the component (A) contains an anti-inflammatory analgesic component, the content of the anti-inflammatory analgesic component in the composition for external preparation is preferably 0.01% by mass or more and 30% by mass or less, more preferably 0.02% by mass or more and 20% by mass or less, still more preferably 0.05% by mass or more and 15% by mass or less, yet still more preferably 0.05% by mass or more and 10% by mass or less, and yet still more preferably 0.05% by mass or more and 6.0% by mass or less.

When the component (A) contains a local irritation component, the content of the local irritation component in the composition for external preparation is preferably 0.001% by mass or more and 20% by mass or less, more preferably 0.005% by mass or more and 15% by mass or less, still more preferably 0.01% by mass or more and 10% by mass or less, and yet still more preferably 0.01% by mass or more and 6.0% by mass or less.

When the component (A) contains a blood circulation promoting component, the content of the blood circulation promoting component in the composition for external preparation is preferably 0.1% by mass or more and 30% by mass or less, more preferably 0.5% by mass or more and 20% by mass or less, still more preferably 1.0% by mass or more and 15% by mass or less, and yet still more preferably 1.0% by mass or more and 5.0% by mass or less.

When the component (A) contains an antihistaminic component, the content of the antihistaminic component in the composition for external preparation is preferably 0.01% by mass or more and 30% by mass or less, more preferably 0.02% by mass or more and 20% by mass or less, still more preferably 0.05% by mass or more and 15% by mass or less, yet still more preferably 0.05% by mass or more and 10% by mass or less, yet still more preferably 0.1% by mass or more and 5.0% by mass or less, and yet still more preferably 0.1% by mass or more and 3.0% by mass or less.

When the component (A) contains a crude drug component, the content of the crude drug component in the composition for external preparation is preferably 0.01% by mass or more and 30% by mass or less, more preferably 0.02% by mass or more and 20% by mass or less, still more preferably 0.05% by mass or more and 15% by mass or less, yet still more preferably 0.05% by mass or more and 10% by mass or less, yet still more preferably 0.1% by mass or more and 5.0% by mass or less, and yet still more preferably 0.1% by mass or more and 3.0% by mass or less.

When the component (A) contains an antipruritic component, the content of the antipruritic component in the composition for external preparation is preferably 0.01% by mass or more and 30% by mass or less, more preferably 0.02% by mass or more and 20% by mass or less, still more preferably 0.02% by mass or more and 15% by mass or less, and yet still more preferably 0.02% by mass or more and 10% by mass or less.

When the component (A) contains a local anesthetic component, the content of the local anesthetic component in the composition for external preparation is preferably 0.01% by mass or more and 30% by mass or less, more preferably 0.02% by mass or more and 20% by mass or less, still more preferably 0.02% by mass or more and 15% by mass or less, yet still more preferably 0.02% by mass or more and 10% by mass or less, and yet still more preferably 0.02% by mass or more and 5.0% by mass or less.

When the component (A) contains a keratin softening component, the content of the keratin softening component in the composition for external preparation is preferably 0.1% by mass or more and 30% by mass or less, more preferably 0.2% by mass or more and 20% by mass or less, and still more preferably 0.5% by mass or more and 20% by mass or less.

When the component (A) contains a bactericidal component, the content of the bactericidal component in the composition for external preparation is preferably 0.001% by mass or more and 20% by mass or less, more preferably 0.005% by mass or more and 15% by mass or less, still more preferably 0.01% by mass or more and 10% by mass or less, yet still more preferably 0.01% by mass or more and 5.0% by mass or less, and yet still more preferably 0.01% by mass or more and 3.0% by mass or less.

When the component (A) contains a antibacterial and antifungal component, the content of the antibacterial and antifungal component in the composition for external preparation is preferably 0.001% by mass or more and 20% by mass or less, more preferably 0.005% by mass or more and 15% by mass or less, still more preferably 0.01% by mass or more and 10% by mass or less, yet still more preferably 0.02% by mass or more and 5.0% by mass or less, and yet still more preferably 0.05% by mass or more and 3.0% by mass or less.

When the component (A) contains an immunosuppressive agent, the content of the immunosuppressive agent in the composition for external preparation is preferably 0.01% by mass or more and 1% by mass or less, and more preferably 0.03% by mass or more and 0.5% by mass or less.

When the component (A) contains an antiviral component, the content of the antiviral component in the composition for external preparation is preferably 0.1% by mass or more and 20% by mass or less, more preferably 0.5% by mass or more and 15% by mass or less, still more preferably 1.0% by mass or more and 10% by mass or less, and yet still more preferably 1.0% by mass or more and 5.0% by mass or less.

When the component (A) contains a hair growth and hair restoration component, the content of the hair growth and hair restoration component in the composition for external preparation is preferably 0.1% by mass or more and 20% by mass or less, more preferably 0.5% by mass or more and 15% by mass or less, still more preferably 1.0% by mass or more and 10% by mass or less, and yet still more preferably 1.0% by mass or more and 5.0% by mass or less.

When the component (A) contains a sebum inhibitory component, the content of the sebum inhibitory component in the composition for external preparation is preferably 0.001% by mass or more and 20% by mass or less, more preferably 0.005% by mass or more and 15% by mass or less, still more preferably 0.01% by mass or more and 10% by mass or less, yet still more preferably 0.01% by mass or more and 5.0% by mass or less, and yet still more preferably 0.01% by mass or more and 3.0% by mass or less.

When the component (A) contains an antiperspirant component, the content of the antiperspirant component in the composition for external preparation is preferably 0.1% by mass or more and 20% by mass or less, more preferably 0.5% by mass or more and 15% by mass or less, still more preferably 1.0% by mass or more and 10% by mass or less, and yet still more preferably 2.0% by mass or more and 5.0% by mass or less.

When the component (A) contains a whitening component, the content of the whitening component in the composition for external preparation is preferably 0.01% by mass or more and 30% by mass or less, more preferably 0.02% by mass or more and 20% by mass or less, still more preferably 0.05% by mass or more and 15% by mass or less, yet still more preferably 0.1% by mass or more and 10% by mass or less, and yet still more preferably 0.2% by mass or more and 5.0% by mass or less.

The content of the component (B) in the composition for external preparation is preferably 0.01% by mass or more, more preferably 0.05% by mass or more, still more preferably 0.1% by mass or more, yet still more preferably 0.5% by mass or more, and yet still more preferably 1.0% by mass or more, from the viewpoint of improving the film-forming properties and from the viewpoint of improving the durability and the moisture resistance of the coating film, and is preferably 30% by mass or less, more preferably 25% by mass or less, still more preferably 20% by mass or less, yet still more preferably 15% by mass or less, and yet still more preferably 10% by mass or less, from the viewpoint of improving the durability of the coating film and from the viewpoint of suppressing the stickiness of the coating film. Then, the content of the component (B) in the composition for external preparation is preferably 0.01% by mass or more and 30% by mass or less, more preferably 0.05% by mass or more and 25% by mass or less, still more preferably 0.1% by mass or more and 20% by mass or less, yet still more preferably 0.5% by mass or more and 15% by mass or less, and yet still more preferably 1.0% by mass or more and 10% by mass or less.

When the component (B) contains a component (B1), the content of the component (B1) in the composition for external preparation is preferably 0.002% by mass or more, more preferably 0.005% by mass or more, still more preferably 0.01% by mass or more, yet still more preferably 0.02% by mass or more, yet still more preferably 0.05% by mass or more, yet still more preferably 0.1% by mass or more, and yet still more preferably 0.2% by mass or more, and is preferably 20% by mass or less, more preferably 15% by mass or less, still more preferably 10% by mass or less, yet still more preferably 8% by mass or less, and yet still more preferably 5% by mass or less. Then, the content of the component (B1) in the composition for external preparation is preferably 0.002% by mass or more and 20% by mass or less, more preferably 0.005% by mass or more and 15% by mass or less, still more preferably 0.01% by mass or more and 10% by mass or less, yet still more preferably 0.02% by mass or more and 8% by mass or less, yet still more preferably 0.05% by mass or more and 5% by mass or less, yet still more preferably 0.1% by mass or more and 5% by mass or less, and yet still more preferably 0.2% by mass or more and 5% by mass or less.

When the component (B) contains a component (B2), the content of the component (B2) in the composition for external preparation is preferably 0.003% by mass or more, more preferably 0.005% by mass or more, still more preferably 0.01% by mass or more, yet still more preferably 0.02% by mass or more, yet still more preferably 0.05% by mass or more, yet still more preferably 0.1% by mass or more, yet still more preferably 0.2% by mass or more, yet still more preferably 0.4% by mass or more, yet still more preferably 0.5% by mass or more, and yet still more preferably 0.6% by mass or more, and is preferably 25% by mass or less, more preferably 20% by mass or less, still more preferably 15% by mass or less, yet still more preferably 10% by mass or less, and yet still more preferably 8% by mass or less. Then, the content of the component (B2) in the composition for external preparation is preferably 0.003% by mass or more and 25% by mass or less, more preferably 0.005% by mass or more and 25% by mass or less, still more preferably 0.01% by mass or more and 20% by mass or less, yet still more preferably 0.02% by mass or more and 20% by mass or less, yet still more preferably 0.05% by mass or more and 15% by mass or less, yet still more preferably 0.1% by mass or more and 15% by mass or less, yet still more preferably 0.2% by mass or more and 10% by mass or less, yet still more preferably 0.4% by mass or more and 10% by mass or less, yet still more preferably 0.5% by mass or more and 8% by mass or less, and yet still more preferably 0.6% by mass or more and 8% by mass or less.

When the component (B) contains a component (B3), the content of the component (B3) in the composition for external preparation is preferably 0.002% by mass or more, more preferably 0.005% by mass or more, still more preferably 0.01% by mass or more, yet still more preferably 0.02% by mass or more, yet still more preferably 0.05% by mass or more, yet still more preferably 0.1% by mass or more, yet still more preferably 0.2% by mass or more, yet still more preferably 0.4% by mass or more, yet still more preferably 0.5% by mass or more, and yet still more preferably 0.6% by mass or more, and is preferably 20% by mass or less, more preferably 15% by mass or less, still more preferably 10% by mass or less, and yet still more preferably 8% by mass or less. Then, the content of the component (B3) in the composition for external preparation is preferably 0.002% by mass or more and 20% by mass or less, more preferably 0.005% by mass or more and 20% by mass or less, still more preferably 0.01% by mass or more and 15% by mass or less, yet still more preferably 0.02% by mass or more and 15% by mass or less, yet still more preferably 0.05% by mass or more and 15% by mass or less, yet still more preferably 0.1% by mass or more and 10% by mass or less, yet still more preferably 0.2% by mass or more and 10% by mass or less, yet still more preferably 0.4% by mass or more and 10% by mass or less, yet still more preferably 0.5% by mass or more and 8% by mass or less, and yet still more preferably 0.6% by mass or more and 8% by mass or less.

The content of the component (C) in the composition for external preparation is preferably 0.001% by mass or more, more preferably 0.005% by mass or more, still more preferably 0.01% by mass or more, yet still more preferably 0.05% by mass or more, and yet still more preferably 0.1% by mass or more, from the viewpoint of improving the film-forming properties and from the viewpoint of improving the durability and the moisture resistance of the coating film, and is preferably 40% by mass or less, more preferably 30% by mass or less, still more preferably 20% by mass or less, and yet still more preferably 15% by mass or less, from the viewpoint of suppressing the stickiness of the coating film. Then, the content of the component (C) in the composition for external preparation is preferably 0.001% by mass or more and 40% by mass or less, more preferably 0.005% by mass or more and 30% by mass or less, still more preferably 0.01% by mass or more and 20% by mass or less, yet still more preferably 0.05% by mass or more and 20% by mass or less, and yet still more preferably 0.1% by mass or more and 15% by mass or less.

The content of the component (D) in the composition for external preparation is preferably 10% by mass or more, more preferably 20% by mass or more, still more preferably 30% by mass or more, and yet still more preferably 40% by mass or more, from the viewpoint of improving the application property and the quick-drying property of the composition, the durability and the moisture resistance of the coating film, and the effect of suppressing crystallization of the component (A), and is preferably 95% by mass or less, more preferably 90% by mass or less, still more preferably 80% by mass or less, and yet still more preferably 70% by mass or less, from the viewpoint of maintaining the application property and the film-forming properties of the composition. Then, the content of the component (D) in the composition for external preparation is preferably 10% by mass or more and 95% by mass or less, more preferably 20% by mass or more and 90% by mass or less, still more preferably 30% by mass or more and 80% by mass or less, and yet still more preferably 40% by mass or more and 70% by mass or less.

The total content of the components (A) to (D) in the composition for external preparation is preferably 20% by mass or more, more preferably 30% by mass or more, still more preferably 50% by mass or more, and yet still more preferably 60% by mass or more, and is 100% by mass or less, preferably 97% by mass or less, more preferably 95% by mass or less, and still more preferably 90% by mass or less, from the viewpoint of improving the durability and the moisture resistance of the coating film, from the viewpoint of improving the effect of suppressing crystallization of the component (A), and from the viewpoint of suppressing the stickiness of the coating film. Then, the total content of the components (A) to (D) in the composition for external preparation is preferably 20% by mass or more and 100% by mass or less, more preferably 30% by mass or more and 97% by mass or less, still more preferably 50% by mass or more and 95% by mass or less, and yet still more preferably 60% by mass or more and 90% by mass or less.

When the composition for external preparation contains water, the content of water in the composition for external preparation is preferably 1% by mass or more, more preferably 2% by mass or more, still more preferably 3% by mass or more, yet still more preferably 5% by mass or more, yet still more preferably 7% by mass or more, yet still more preferably 8% by mass or more, yet still more preferably 10% by mass or more, and yet still more preferably 15% by mass or more, from the viewpoint of improving the application property and the film-forming properties of the composition for external preparation, and the durability and the moisture resistance of the coating film, from the viewpoint of improving the effect of suppressing crystallization of the component (A), from the viewpoint of suppressing the stickiness of the coating film, and from the viewpoint of improving the removability of the coating film, and is preferably 70% by mass or less, more preferably 60% by mass or less, still more preferably 50% by mass or less, yet still more preferably 45% by mass or less, yet still more preferably 40% by mass or less, yet still more preferably 38% by mass or less, and yet still more preferably 35% by mass or less, from the viewpoint of improving the quick-drying property of the composition, from the viewpoint of improving the durability and the moisture resistance of the coating film, from the viewpoint of improving the effect of suppressing crystallization of the component (A), and from the viewpoint of suppressing the stickiness of the coating film. Then, the content of water in the composition for external preparation is preferably 1% by mass or more and 70% by mass or less, more preferably 2% by mass or more and 60% by mass or less, still more preferably 3% by mass or more and 50% by mass or less, yet still more preferably 5% by mass or more and 45% by mass or less, yet still more preferably 7% by mass or more and 40% by mass or less, yet still more preferably 8% by mass or more and 38% by mass or less, yet still more preferably 10% by mass or more and 35% by mass or less, and yet still more preferably 15% by mass or more and 35% by mass or less.

The mass ratio [(A)/(B)] of the component (A) to the component (B) in the composition for external preparation is preferably 0.001 or more, more preferably 0.002 or more, and still more preferably 0.003 or more, from the viewpoint of sufficiently exhibiting the efficacy of the component (A) and from the viewpoint of improving the effect of suppressing crystallization of the component (A), and is preferably 300 or less, more preferably 250 or less, still more preferably 200 or less, yet still more preferably 100 or less, yet still more preferably 50 or less, yet still more preferably 30 or less, yet still more preferably 20 or less, yet still more preferably 10 or less, yet still more preferably 5 or less, and yet still more preferably 2 or less, from the viewpoint of improving the application property and the film-forming properties of the composition, from the viewpoint of improving the durability and the moisture resistance of the coating film, and from the viewpoint of improving the effect of suppressing crystallization of the component (A). Then, the mass ratio [(A)/(B)] of the component (A) to the component (B) in the composition for external preparation is preferably 0.001 or more and 300 or less, more preferably 0.001 or more and 250 or less, still more preferably 0.002 or more and 200 or less, yet still more preferably 0.002 or more and 100 or less, yet still more preferably 0.002 or more and 50 or less, yet still more preferably 0.002 or more and 30 or less, yet still more preferably 0.002 or more and 20 or less, yet still more preferably 0.002 or more and 10 or less, yet still more preferably 0.002 or more and 5 or less, and yet still more preferably 0.003 or more and 2 or less.

The mass ratio [(A)/(C)] of the component (A) to the component (C) in the composition for external preparation is preferably 0.001 or more, more preferably 0.002 or more, still more preferably 0.003 or more, yet still more preferably 0.005 or more, and yet still more preferably 0.01 or more, from the viewpoint of sufficiently exhibiting the efficacy of the component (A) and from the viewpoint of improving the effect of suppressing crystallization of the component (A), and is preferably 100 or less, more preferably 80 or less, still more preferably 70 or less, yet still more preferably 60 or less, yet still more preferably 50 or less, and yet still more preferably 45 or less, from the viewpoint of improving the application property and the film-forming properties of the composition, from the viewpoint of improving the durability and the moisture resistance of the coating film, and from the viewpoint of improving the effect of suppressing crystallization of the component (A). Then, the mass ratio [(A)/(C)] of the component (A) to the component (C) in the composition for external preparation is preferably 0.001 or more and 100 or less, more preferably 0.002 or more and 80 or less, still more preferably 0.003 or more and 70 or less, yet still more preferably 0.005 or more and 60 or less, yet still more preferably 0.01 or more and 50 or less, and yet still more preferably 0.01 or more and 45 or less.

The mass ratio [(C)/(B)] of the component (C) to the component (B) in the composition for external preparation is preferably 0.01 or more, more preferably 0.02 or more, and still more preferably 0.03 or more, from the viewpoint of improving the durability and the moisture resistance of the coating film, and is preferably 50 or less, more preferably 35 or less, still more preferably 20 or less, yet still more preferably 10 or less, and yet still more preferably 5.0 or less, from the viewpoint of improving the durability and the moisture resistance of the coating film, and from the viewpoint of suppressing the stickiness of the coating film. Then, the mass ratio [(C)/(B)] of the component (C) to the component (B) in the composition for external preparation is preferably 0.01 or more and 50 or less, more preferably 0.01 or more and 35 or less, still more preferably 0.02 or more and 20 or less, yet still more preferably 0.02 or more and 10 or less, and yet still more preferably 0.03 or more and 5.0 or less.

The mass ratio [(A)/{(B) + (C)}] of the component (A) to the total content of the component (B) and the component (C) in the composition for external preparation is preferably 0.001 or more, more preferably 0.005 or more, still more preferably 0.01 or more, yet still more preferably 0.02 or more, and yet still more preferably 0.03 or more, from the viewpoint of sufficiently exhibiting the efficacy of the component (A), and is preferably 20 or less, more preferably 15 or less, still more preferably 10 or less, yet still more preferably 5.0 or less, yet still more preferably 4.0 or less, and yet still more preferably 3.0 or less, from the viewpoint of improving the durability and the moisture resistance of the coating film, and from the viewpoint of improving the effect of suppressing crystallization of the component (A). Then, the mass ratio [(A)/{(B) + (C)}] of the component (A) to the total content of the component (B) and the component (C) in the composition for external preparation is preferably 0.001 or more and 20 or less, more preferably 0.005 or more and 15 or less, still more preferably 0.01 or more and 10 or less, yet still more preferably 0.02 or more and 5.0 or less, yet still more preferably 0.03 or more and 4.0 or less, and yet still more preferably 0.03 or more and 3.0 or less.

When the composition for external preparation contains water, the mass ratio [water/(D)] of water to the component (D) in the composition for external preparation is preferably 0.005 or more, more preferably 0.05 or more, still more preferably 0.10 or more, yet still more preferably 0.20 or more, and yet still more preferably 0.50 or more, from the viewpoint of improving the durability and the moisture resistance of the coating film, from the viewpoint of improving the effect of suppressing crystallization of the component (A), from the viewpoint of suppressing the stickiness of the coating film, and from the viewpoint of improving the removability of the coating film, and is preferably 10 or less, more preferably 8.0 or less, still more preferably 5.0 or less, yet still more preferably 3.0 or less, yet still more preferably 2.0 or less, yet still more preferably 1.0 or less, and yet still more preferably 0.7 or less, from the viewpoint of improving the quick-drying property of the composition, from the viewpoint of improving the durability and the moisture resistance of the coating film, from the viewpoint of improving the effect of suppressing crystallization of the component (A), and from the viewpoint of suppressing the stickiness of the coating film. Then, the mass ratio [water/(D)] of water to the component (D) in the composition for external preparation is preferably 0.005 or more and 10 or less, more preferably 0.05 or more and 8.0 or less, still more preferably 0.10 or more and 5.0 or less, yet still more preferably 0.20 or more and 3.0 or less, yet still more preferably 0.50 or more and 3.0 or less, yet still more preferably 0.50 or more and 2.0 or less, yet still more preferably 0.50 or more and 1.0 or less, and yet still more preferably 0.50 or more and 0.7 or less.

The mass ratio [(B)/(D)] of the component (B) to the component (D) in the composition for external preparation is preferably 0.0001 or more, more preferably 0.0002 or more, still more preferably 0.0005 or more, and yet still more preferably 0.001 or more, from the viewpoint of improving the film-forming properties, and is preferably 1.0 or less, more preferably 0.50 or less, still more preferably 0.30 or less, yet still more preferably 0.20 or less, and yet still more preferably 0.10 or less, from the viewpoint of improving the application property and the quick-drying property of the composition. Then, the mass ratio [(B)/(D)] of the component (B) to the component (D) in the composition for external preparation is preferably 0.0001 or more and 1.0 or less, more preferably 0.0002 or more and 0.50 or less, still more preferably 0.0005 or more and 0.30 or less, yet still more preferably 0.001 or more and 0.20 or less, and yet still more preferably 0.001 or more and 0.10 or less.

### <Viscosity>

The viscosity of the composition for external preparation at 25°C is 1.0 mPa·s or more, preferably 1.5 mPa·s or more, more preferably 2.0 mPa·s or more, and still more preferably 3.0 mPa·s or more, from the viewpoint of improving the application property of the composition. On the other hand, from the viewpoint of improving the quick-drying property of the composition, and from the viewpoint of improving the durability and the moisture resistance of the coating film, the viscosity of the composition for external preparation at 25°C is 200,000 mPa·s or less, 150,000 mPa·s or less, 130,000 mPa·s or less, 100,000 mPa·s or less, 50,000 mPa·s or less, 30,000 mPa·s or less, 20,000 mPa·s or less, 10,000 mPa·s or less, preferably 7,500 mPa·s or less, more preferably 5,000 mPa·s or less, still more preferably 3,000 mPa·s or less, yet still more preferably 2,000 mPa·s or less, yet still more preferably 1,000 mPa·s or less, yet still more preferably 700 mPa·s or less, yet still more preferably 500 mPa·s or less, yet still more preferably 200 mPa·s or less, yet still more preferably 100 mPa·s or less, and yet still more preferably 50 mPa·s or less. Then, the viscosity of the composition for external preparation at 25°C is 1.0 mPa·s or more and 10,000 mPa·s or less, preferably 1.0 mPa·s or more and 7,500 mPa·s or less, more preferably 1.5 mPa·s or more and 5,000 mPa·s or less, still more preferably 1.5 mPa·s or more and 3,000 mPa·s or less, yet still more preferably 1.5 mPa·s or more and 2,000 mPa·s or less, yet still more preferably 2.0 mPa·s or more and 1,000 mPa·s or less, yet still more preferably 2.0 mPa·s or more and 700 mPa·s or less, yet still more preferably 2.0 mPa·s or more and 500 mPa·s or less, yet still more preferably 3.0 mPa·s or more and 200 mPa·s or less, yet still more preferably 3.0 mPa·s or more and 100 mPa·s or less, and yet still more preferably 3.0 mPa·s or more and 50 mPa·s or less.

The viscosity of the composition for external preparation at 25°C is measured by a vibration type viscometer for a composition having a viscosity of 1,000 mPa·s or less, and by a B-type rotational viscometer for a composition having a viscosity of more than 1,000 mPa·s, and can be measured specifically by the method described in Examples.

### <Contact Angle>

From the viewpoint of improving the application property of the composition, the film-forming properties, and the durability of the coating film, the contact angle of the composition for external preparation with respect to the artificial leather after 10 seconds after droplet deposition is preferably 5° or more, more preferably 10° or more, still more preferably 15° or more, yet still more preferably 20° or more, and yet still more preferably 25° or more, and is preferably 80° or less, more preferably 75° or less, still more preferably 70° or less, yet still more preferably 60° or less, and yet still more preferably 55° or less. Then, the contact angle of the composition for external preparation with respect to the artificial leather after 10 seconds after droplet deposition is preferably 5° or more and 80° or less, more preferably 10° or more and 75° or less, still more preferably 15° or more and 70° or less, yet still more preferably 20° or more and 60° or less, and yet still more preferably 25° or more and 55° or less. Further, the contact angle of the composition with respect to the artificial leather after 60 seconds after droplet deposition is preferably 5° or more, more preferably 10° or more, still more preferably 15° or more, yet still more preferably 18° or more, and yet still more preferably 20° or more, and is preferably 70° or less, more preferably 65° or less, still more preferably 60° or less, yet still more preferably 55° or less, and yet still more preferably 50° or less. Then, the contact angle of the composition for external preparation with respect to the artificial leather after 60 seconds after droplet deposition is preferably 5° or more and 70° or less, more preferably 10° or more and 65° or less, still more preferably 15° or more and 60° or less, yet still more preferably 18° or more and 55° or less, and yet still more preferably 20° or more and 50° or less.

In addition, the rate of change in the contact angle of the composition for external preparation with respect to the artificial leather from after 10 seconds after droplet deposition to after 60 seconds after droplet deposition (1 - ((contact angle after 60 sec after droplet deposition)/(contact angle after 10 sec after droplet deposition))) is preferably 0.6 or less, more preferably 0.5 or less, still more preferably 0.4 or less, yet still more preferably 0.3 or less, and yet still more preferably 0.25 or less from the viewpoint of improving the application property of the composition, the film-forming properties, and the durability of the coating film. The contact angle can be measured at a temperature of 25°C by the method described in Examples.

### <Dosage Form>

The composition for external preparation of the present invention is preferably used as a composition for skin external preparation.

The dosage form of the composition for external preparation may be in any form as long as it has a viscosity within the above range and can be applied to the skin. Examples thereof include a lotion formulation, a gel formulation, an ointment formulation, a cream formulation, or a foam formulation made of the composition for external preparation of the present invention; and an aerosol formulation or a pump spray type formulation using the composition for external preparation of the present invention. Examples of the foam formulation include formulations used by filling a pump foamer with the composition for external preparation of the present invention and ejecting it in a foam form.

The compositions of the present invention can also be electrostatically sprayed onto the skin using an electrostatic spray device. The electrostatic spraying will be described later.

The aerosol formulation contains the composition for external preparation and a propellant. The aerosol formulation using the composition for external preparation of the present invention can provide cool feeling by spraying and applying the composition to the skin.

In the aerosol formulation, the composition for external preparation used as an aerosol stock solution and the suitable aspect thereof are the same as described above. That is, the content of the components (A) to (D) in the aerosol stock solution and the viscosity of the aerosol stock solution are preferably in the ranges described above.

Examples of the propellant used in the aerosol formulation include liquefied petroleum gas (LPG) which is ethane, propane, normal butane, isobutane, isopentane, and a mixture thereof; ethers such as dimethyl ether (DME); and compressed gases such as nitrogen and carbon dioxide, and one or more of these can be used.

In the aerosol formulation, the quantitative ratio between the composition for external preparation which is the aerosol stock solution and the propellant is not particularly limited as long as the composition for external preparation can be sprayed to the skin, but from the viewpoint of aerosol performances and from the viewpoint of the stability of the aerosol formulation, the mass ratio of the composition for external preparation to the propellant is preferably in the range of 1:0.01 to 1:10, and more preferably in the range of 1:0.05 to 1:7.5.

When the propellant is LPG, the mass ratio of the composition for external preparation to LPG is still more preferably 1:0.5 to 1:5, when the propellant is dimethyl ether, the mass ratio of the composition for external preparation to dimethyl ether is still more preferably 1:0.1 to 1:5, and when the propellant is carbon dioxide, the mass ratio of the composition for external preparation to carbon dioxide is still more preferably 1:0.1 to 1:0.5.

Examples of an aerosol container used in the aerosol formulation include a known pressure-resistant container made of metal or plastic, and a double-structured container in which an inner bag is accommodated inside the pressure-resistant container. In the double-structured container, it is preferable that the inner bag is filled with a composition for external preparation as an aerosol stock solution, and a propellant is filled between the pressure-resistant container and the inner bag.

The method for preparing the aerosol formulation is not particularly limited. For example, the aerosol formulation can be prepared by filling the aerosol container with a composition for external preparation which is an aerosol stock solution, attaching a valve to the container, and then filling a propellant from the valve portion.

In addition, the pump spray type formulation is a formulation used by filling the composition for external preparation of the present invention into a pump spray container.

### [Method of Use]

The present invention also provides a method for using a composition for external preparation, which includes the following steps (I) and (II) in this order:
step (I): applying the composition for external preparation to an object and then drying the composition to form a coating film composed of the composition for external preparation; and
step (II): bringing the coating film into contact with a cleaning agent and then washing with water to remove the coating film.

According to the method of the present invention, in step (I), the composition for external preparation is applied to an object and then dried, whereby a coating film containing the component (A) which is a medicinal ingredient can be formed on the surface of the object. Since the coating film is excellent in durability and moisture resistance, the component (A) can be sustainedly released to an object. In addition, since the coating film can be washed away with water using a cleaning agent, the coating film formed in the step (I) can be easily removed from the object by performing the step (II).

### <Step (I)>

In the step (I), the method for applying the composition for external preparation to an object can be appropriately selected depending on the dosage form of the composition for external preparation, and examples thereof include a method for applying by coating, casting, or spraying. Among these, it is preferable to apply by coating or spraying. After the composition for external preparation is applied, the component (D) is volatilized by natural drying, and a coating film containing the components (A) to (C) is formed on the surface of the object.

When the composition for external preparation is directly applied to the skin, the composition for external preparation can be applied by hand or using an application tool such as a metal roller, a plastic roller, a sponge-like porous body, or a brush.

When the composition for external preparation is sprayed on the skin, examples of the spraying method of the composition include a method using an aerosol, a pump spray, or an electrostatic spray device. Regarding the aerosol and the pump spray, the aerosol formulation and the pump spray formulation are prepared using the composition for external preparation as a stock solution, and the composition for external preparation can be sprayed to an object using the formulation.

The electrostatic spray device is specifically provided with a storage section capable of storing the composition for external preparation, a nozzle for ejecting the composition for external preparation, a power source for applying a voltage to the nozzle, and a means for sending the composition for external preparation from the storage section to the nozzle. As an embodiment thereof, a handy-type electrostatic spray device having a size capable of being gripped by one hand is mentioned.

As the electrostatic spray device, those exemplified in WO 2018/194140 can be used.

The object to which the composition for external preparation is applied is preferably a skin surface.

The application site of the composition for external preparation is not particularly limited, but preferred examples thereof include a skin surface of a head, a face, a neck, a hand, an arm, a foot, a leg, a buttock, or a trunk.

The amount of the composition for external preparation applied is not particularly limited, but from the viewpoint of effectively exerting the efficacy of the component (A), it is preferably 0.5 µL/cm² or more, more preferably 1 µL/cm² or more, and still more preferably 3 µL/cm² or more. On the other hand, from the viewpoint of the appearance (transparency) of the formed coating film, the amount is preferably 100 µL/cm² or less, and more preferably 50 µL/cm² or less.

The drying method after applying the composition for external preparation to an object is preferably natural drying, but a device such as a dryer can also be used as appropriate.

The thickness of the coating film formed in the step (I) is preferably 0.005 mm or more, more preferably 0.01 mm or more, still more preferably 0.015 mm or more, and yet still more preferably 0.02 mm or more, and is preferably 0.3 mm or less, more preferably 0.1 mm or less, still more preferably 0.07 mm or less, and yet still more preferably 0.06 mm or less, from the viewpoint of ease of formation of the coating film and from the viewpoint of improvement of durability. Then, the thickness of the coating film formed in the step (I) is preferably 0.005 mm or more and 0.3 mm or less, more preferably 0.01 mm or more and 0.1 mm or less, still more preferably 0.015 mm or more and 0.07 mm or less, and yet still more preferably 0.02 mm or more and 0.06 mm or less. The thickness of the coating film can be measured by the method described in Examples.

### <Step (II)>

In the step (II), the coating film formed in the step (I) is brought into contact with a cleaning agent and then washed with water to remove the coating film.

The step (II) is preferably performed after a certain period of time has elapsed since the formation of the coating film on the surface of the object in the step (I), that is, after the efficacy of the component (A) has been exhibited. From this viewpoint, the time after the step (I) until the step (II) is performed is preferably 0.5 hours or more and 72 hours or less, and more preferably 1 hour or more and 48 hours or less.

The cleaning agent used in the step (II) may be a cleaning agent for cleaning an object. Examples thereof include a cleaning agent containing an anionic, cationic, or nonionic surface-active component and a cleaning agent containing an organic solvent. Specific examples thereof include a synthetic detergent and soap. When the skin as the object is a scalp, examples of the cleaning agent include a shampoo, and when the skin as the object is a face, examples of the cleaning agent include a facial cleanser. Examples of cleaning agents for skin other than these include soap, hand soap, and body soap. Examples of the pH of the cleaning agent include less than pH3 (acidic), pH3 or more and 6 or less (weakly acidic), and more than pH6. From the viewpoint of removability of the coating film and from the viewpoint of less irritation to the skin, the cleaning agent is preferably a cleaning agent of pH3 or more and 6 or less (weakly acidic), or more than pH6, and more preferably a cleaning agent of more than pH6.

The pH of the cleaning agent refers to the pH of a 5% aqueous solution at 20°C.

In the step (II), one embodiment of a method for removing a coating film formed on a skin surface will be described. First, the cleaning agent is brought into contact with a coating film formed on the skin surface by application, casting, or spraying. When the cleaning agent is a solid cleaning agent such as soap, it is preferable to wet the coating film on the skin surface or the solid cleaning agent with water in advance and then bring them into contact with each other. In addition, the cleaning agent may be foamed in advance and then brought into contact with the coating film.

After the cleaning agent brought into contact with the coating film is applied to the coating film, the coating film is washed with water. At the time of washing with water, it is preferable to rub the coating film. By this operation, the coating film can be easily removed.

### Examples

Hereinafter, the present invention will be described with reference to Examples, but the present invention is not limited to the scope of Examples. In these Examples, various measurements and evaluations were performed by the following methods.

### (Viscosity)

The viscosity of the composition for external preparation at 25°C was measured using a vibration type viscometer ("VM-10AL" manufactured by SEKONIC CORPORATION). In addition, the viscosity of the composition having a viscosity of more than 1,000 mPa·s was measured using a B-type rotational viscometer ("TVB-10" manufactured by Toki Sangyo Co., Ltd., SPINDLE: M3, rotation speed: 12 rpm).

### (Presence or absence of crystals in coating film)

The composition for external preparation was applied in an amount of 18 µL to an area of 1.5 cm × 2 cm on a slide glass using MICROMAN (manufactured by GILSON), and dried for 1 hour on a hot plate (a "ceramic hot plate" manufactured by AS ONE Corporation) set at 32°C. The presence or absence of crystals in the formed coating film was evaluated by observation with a polarizing microscope and judged according to the following criteria. A higher score means that the deposition of the crystalline medicinal ingredient is less likely to occur.
5: No crystals are observed at all.
4: Crystals of less than about 10 µm are observed.
3: Crystals of about 10 µm or more and less than 50 µm are observed.
2: Crystals of about 50 µm or more and less than 100 µm are observed.
1: Crystals of about 100 µm or more are observed.

### (Sticky feeling in coating film)

An expert panelist applied 18 µL of the composition for external preparation to an area of 1.5 cm × 2 cm on the inner side of the forearm using MICROMAN (manufactured by GILSON), and evaluated the sticky feeling after 2 minutes on a 5-point scale. The average value of the scores of the 6 expert panelists (rounded off to the second decimal place) is shown in the table. A higher score means a better evaluation result.
5: Not sticky at all.
4: Slightly sticky.
3: Somewhat sticky.
2: Sticky.
1: Very sticky.

### (Durability of coating film)

An expert panelist applied 18 µL of the composition for external preparation to an area of 1.5 cm × 2 cm on the inner side of the forearm using MICROMAN (manufactured by GILSON). After the application, a living action with an exercise intensity of about 3 mets or less was performed in a room at 30°C or lower, and the formed coating film was visually observed after a certain period of time to confirm the presence or absence of the remaining coating film. The longest time during which 90% or more of the coating film remained was scored based on the following criteria.
5: After 8 hours.
4: After 4 hours.
3: After 1 hour.
2: After 30 minutes.
1: After 10 minutes.

### (Moisture resistance of coating film)

An expert panelist applied 50 µL of the composition for external preparation to an area of 2 cm × 2 cm on the inner side of the forearm using MICROMAN (manufactured by GILSON), and the composition was naturally dried for 10 minutes to obtain a coating film. Thereafter, the panelist was allowed to stay for 10 minutes in a constant temperature and humidity room having a temperature of 40°C and a humidity of 75%, and the state of the coating film remaining when the coated portion was rubbed with a finger 10 times was visually observed, and the moisture resistance of the coating film was determined based on the following criteria.
5: No wrinkling or peeling, and 100% of the coating film remained.
4: Almost no wrinkling or peeling, and 90% or more of the coating film remained.
3: Slight wrinkling or peeling, and 80% or more of the coating film remained.
2: Wrinkling or peeling, and 50% or more and less than 80% of the coating film remained.
1: Significant wrinkling or peeling, and less than 50% of the coating film remained.

### Examples 1 to 102 and Comparative Examples 1 to 8 (Preparation and Evaluation of Compositions for External Preparation)

The component (B) and other components described in the table were mixed with the component (C), the component (D), and an appropriate amount of water, and then the component (A) was mixed therewith. Thereafter, the remaining water was mixed to prepare a composition for external preparation.

Using the obtained composition for external preparation, evaluation was performed by the method described above. The main components used are shown in Table 1, and the compositions and evaluation results of the compositions for external preparation are shown in Tables 2 to 11. The blending amount described in each table is the active ingredient amount (% by mass) of each component.

**Table 1**

| Component names described in the tables of Examples | | Acquisition source, product name |
|---|---|---|
| (A) | Loxoprofen sodium hydrate | FUJIFILM Wako Pure Chemical Corporation |
| | 1-Menthol | TAKASAGO INTERNATIONAL CORPORATION |
| (B) | (B1) Hypromellose phthalate | "HPMCP HP-55" manufactured by Shin-Etsu Chemical Co., Ltd. |
| | (B1) Ethyl cellulose | "ETHOCEL 100P" manufactured by Nisshin Kasei Co., Ltd. |
| | (B2) Acrylates/diacetoneacrylamide copolymer | "Plascize L-53" manufactured by GOO CHEMICAL CO., LTD., nonvolatile content: 50% by mass |
| | (B2) Alkyl acrylate-vinyl acetate copolymer | "VINYSOL 2140L" manufactured by Daido Chemical Corporation, nonvolatile content: 45% by mass |
| | (B2) Alkyl acrylate copolymer | "VINYSOL 1086WP" manufactured by Daido Chemical Corporation, nonvolatile content: 40% by mass |
| | (B3) Polyvinyl butyral | "S-LEC BM-1" manufactured by SEKISUI CHEMICAL CO., LTD. |
| | Isopropyl myristate | "EXCEPARL IPM" manufactured by Kao Corporation |
| | Dipropylene glycol | "DPG-RF" manufactured by ADEKA CORPORATION |
| | Polysorbate 80 | "TO-10MV" manufactured by Nikko Chemicals Co., Ltd. |
| | Squalane | manufactured by Nikko Chemicals Co., Ltd. |
| (C) | Diisopropanolamine | manufactured by Mitsui Fine Chemicals, Inc. |
| | Triethanolamine | Merck & Co., Inc. |
| | 1,3-Butylene glycol | "1,3-butylene glycol-P" manufactured by KH Neochem Co., Ltd. |
| | Propylene glycol monocaprate | "SEFSOL-218" manufactured by Nikko Chemicals Co., Ltd. |
| | Polyethylene glycol 4000 | manufactured by FUJIFILM Wako Pure Chemical Corporation |
| Others | Polyvinylpyrrolidone | manufactured by BASF |
| | Carboxyvinyl polymer | "HIVISWAKO 103" manufactured by FUJIFILM Wako Pure Chemical Corporation |

**Table 2**

| (% by volume) | | | Examples | | | | |
|---|---|---|---|---|---|---|---|
| | | | 1 | 2 | 3 | 4 | 5 |
| (A) | Loxoprofen sodium hydrate | | 1.13 | 1.13 | 1.13 | 1.13 | 1.13 |
| | l-Menthol | | 3 | 3 | 3 | 3 | 3 |
| (B) | (B1) Hypromellose phthalate | Cellulosic-type | 1 | | 0.01 | 0.3 | 0.5 |
| | (B1) Ethyl cellulose | Cellulosic-type | | 0.2 | | | |
| | (B2) Acrylates/diacetoneacrylamide copolymer | Acrylic-type | 2 | 2 | 0.01 | 0.6 | 1 |
| | (B3) Polyvinyl butyral | Vinyl-type | | | | | |
| | Isopropyl myristate | Ester oil | 1 | 1 | 1 | 1 | 1 |
| | Dipropylene glycol | Polyol | | | | | |
| | Polyethylene glycol 400 | Polyol | | | | | |
| | Polysorbate 80 | Nonionic surfactant | | | | | |
| | Squalane | Non-polar oil | | | | | |
| (C) | Diisopropanolamine | Alkanolamine | | | | | |
| | Triethanolamine | Alkanolamine | | | | | |
| | 2-Amino-2-methylpropanol | Alkanolamine | 0.3 | 0.3 | 0.001 | 0.1 | 0.1 |
| | 1,3-Butylene glycol | Polyol | | | | | |
| | Propylene glycol monocaprate | Ester oil | | | | | |
| | Polyethylene glycol 4000 | Polyol | | | | | |
| Others | Polyvinylpyrrolidone | Water-soluble polymer | | | | | |
| | Lactic acid | | 0.18 | | | | |
| (D) | Ethanol | | 57 | 57 | 57 | 57 | 57 |
| | Isopropanol | | | | | | |
| Water | Purified water | | 34.42 | 35.40 | 37.85 | 36.89 | 36.24 |
| Total | | | 100 | 100 | 100 | 100 | 100 |
| Component (A) content (% by mass) | | | 4.13 | 4.13 | 4.13 | 4.13 | 4.13 |
| Component (B) content (% by mass) | | | 3 | 2.2 | 0.02 | 0.9 | 1.5 |
| Component (B1) content (% by mass) | | | 1 | 0.2 | 0.01 | 0.3 | 0.5 |
| Component (B2) content (% by mass) | | | 2 | 2 | 0.01 | 0.6 | 1 |
| Component (B3) content (% by mass) | | | 0 | 0 | 0 | 0 | 0 |
| Component (C) content (% by mass) | | | 1.27 | 1.27 | 1.00 | 1.08 | 1.13 |
| Component (D) content (% by mass) | | | 57 | 57 | 57 | 57 | 57 |
| Components (A) to (D) total content (% by mass) | | | 65.40 | 64.60 | 62.15 | 63.11 | 63.76 |
| A/B | | | 1.38 | 1.88 | 206.50 | 4.59 | 2.75 |
| A/C | | | 3.26 | 3.26 | 4.12 | 3.82 | 3.64 |
| C/B | | | 0.42 | 0.58 | 50.07 | 1.20 | 0.76 |
| A/(B + C) | | | 0.97 | 1.19 | 4.04 | 2.09 | 1.57 |
| Water/D | | | 0.60 | 0.62 | 0.66 | 0.65 | 0.64 |
| B/D | | | 0.05 | 0.04 | 0.00 | 0.02 | 0.03 |
| Acrylic/cellulosic B2/B1 | | | 2 | 10 | 1 | 2 | 2 |
| Vinyl/cellulosic B3/B1 | | | 0 | 0 | 0 | 0 | 0 |
| Acrylic/vinyl B2/B3 | | | - | - | - | - | - |
| Viscosity (mPa·s) 25°C | | | 6.08 | 5.51 | 1.56 | 3.07 | 5.45 |
| Evaluation | Presence or absence of crystals in coating film | | 5.0 | 5.0 | 3.0 | 3.0 | 4.0 |
| | Sticky feeling in coating film | | 5.0 | 4.2 | 5.0 | 5.0 | 5.0 |
| | Durability of coating film | | 5.0 | 4.0 | 3.0 | 5.0 | 5.0 |
| | Moisture resistance of coating film | | 5.0 | 5.0 | 3.0 | 5.0 | 5.0 |

**Table 2 (continued)**

| (% by mass) | | | Examples | | | | |
|---|---|---|---|---|---|---|---|
| | | | 6 | 7 | 8 | 9 | 10 |
| (A) | Loxoprofen sodium hydrate | | 1.13 | 1.13 | 1.13 | 1.13 | 1.13 |
| | 1-Menthol | | 3 | 3 | 3 | 3 | 3 |
| (B) | (B1) Hypromellose phthalate | Cellulosic-type | 1.5 | 2 | 3 | 2 | 5 |
| | (B1) Ethyl cellulose | Cellulosic-type | | | | | |
| | (B2) Acrylates/diacetoneacrylamide copolymer | Acrylic-type | 3 | 4 | 6 | 8 | 5 |
| | (B3) Polyvinyl butyral | Vinyl-type | | | | | |
| | Isopropyl myristate | Ester oil | 1 | 1 | 1 | 1 | 1 |
| | Dipropylene glycol | Polyol | | | | | |
| | Polyethylene glycol 400 | Polyol | | | | | |
| | Polysorbate 80 | Nonionic surfactant | | | | | |
| | Squalane | Non-polar oil | | | | | |
| (C) | Diisopropanolamine | Alkanolamine | | | | | |
| | Triethanolamine | Alkanolamine | | | | | |
| | 2-Amino-2-methylpropanol | Alkanolamine | 0.4 | 0.5 | 0.8 | 1.1 | 0.7 |
| | 1,3-Butylene glycol | Polyol | | | | | |
| | Propylene glycol monocaprate | Ester oil | | | | | |
| | Polyethylene glycol 4000 | Polyol | | | | | |
| Others | Polyvinylpyrrolidone | Water-soluble polymer | | | | | |
| | Lactic acid | | | | | | |
| (D) | Ethanol | | 57 | 57 | 57 | 57 | 57 |
| | Isopropanol | | | | | | |
| Water | Purified water | | 32.97 | 31.34 | 28.07 | 26.80 | 27.20 |
| Total | | | 100 | 100 | 100 | 100 | 100 |
| Component (A) content (% by mass) | | | 4.13 | 4.13 | 4.13 | 4.13 | 4.13 |
| Component (B) content (% by mass) | | | 4.5 | 6 | 9 | 10 | 10 |
| Component (B1) content (% by mass) | | | 1.5 | 2 | 3 | 2 | 5 |
| Component (B2) content (% by mass) | | | 3 | 4 | 6 | 8 | 5 |
| Component (B3) content (% by mass) | | | 0 | 0 | 0 | 0 | 0 |
| Component (C) content (% by mass) | | | 1.40 | 1.53 | 1.80 | 2.07 | 1.67 |
| Component (D) content (% by mass) | | | 57 | 57 | 57 | 57 | 57 |
| Components (A) to (D) total content (% by mass) | | | 67.03 | 68.66 | 71.93 | 73.20 | 72.80 |
| A/B | | | 0.92 | 0.69 | 0.46 | 0.41 | 0.41 |
| A/C | | | 2.95 | 2.69 | 2.29 | 2.00 | 2.48 |
| C/B | | | 0.31 | 0.26 | 0.20 | 0.21 | 0.17 |
| A/(B + C) | | | 0.70 | 0.55 | 0.38 | 0.34 | 0.35 |
| Water/D | | | 0.58 | 0.55 | 0.49 | 0.47 | 0.48 |
| B/D | | | 0.08 | 0.11 | 0.16 | 0.18 | 0.18 |
| Acrylic/cellulosic B2/B1 | | | 2 | 2 | 2 | 4 | 1 |
| Vinyl/cellulosic B3/B1 | | | 0 | 0 | 0 | 0 | 0 |
| Acrylic/vinyl B2/B3 | | | - | - | - | - | - |
| Viscosity (mPa·s) 25°C | | | 7.35 | 11.2 | 17.4 | 24 | 36.1 |
| Evaluation | Presence or absence of crystals in coating film | | 4.0 | 4.0 | 5.0 | 5.0 | 4.0 |
| | Sticky feeling in coating film | | 5.0 | 5.0 | 5.0 | 5.0 | 5.0 |
| | Durability of coating film | | 5.0 | 5.0 | 5.0 | 5.0 | 5.0 |
| | Moisture resistance of coating film | | 5.0 | 5.0 | 5.0 | 5.0 | 5.0 |

**Table 2 (continued)**

| (% by mass) | | | Examples | | | | |
|---|---|---|---|---|---|---|---|
| | | | 11 | 12 | 13 | 14 | 15 |
| (A) | Loxoprofen sodium hydrate | | 1.13 | 1.13 | 1.13 | 1.13 | 1.13 |
| | 1-Menthol | | 3 | 3 | 3 | 3 | 3 |
| (B) | (B1) Hypromellose phthalate | Cellulosic-type | 8 | 10 | 1 | 1 | 1 |
| | (B1) Ethyl cellulose | Cellulosic-type | | | | | |
| | (B2) Acrylates/diacetoneacrylamide copolymer | Acrylic-type | 2 | 2 | 12 | 15 | 20 |
| | (B3) Polyvinyl butyral | Vinyl-type | | | | | |
| | Isopropyl myristate | Ester oil | 1 | 1 | 1 | 1 | 1 |
| | Dipropylene glycol | Polyol | | | | | |
| | Polyethylene glycol 400 | Polyol | | | | | |
| | Polysorbate 80 | Nonionic surfactant | | | | | |
| | Squalane | Non-polar oil | | | | | |
| (C) | Diisopropanolamine | Alkanolamine | | | | | |
| | Triethanolamine | Alkanolamine | | | | | |
| | 2-Amino-2-methylpropanol | Alkanolamine | 0.3 | 0.3 | 1.6 | 2.0 | 2.7 |
| | 1,3-Butylene glycol | Polyol | | | | | |
| | Propylene glycol monocaprate | Ester oil | | | | | |
| | Polyethylene glycol 4000 | Polyol | | | | | |
| Others | Polyvinylpyrrolidone | Water-soluble polymer | | | | | |
| | Lactic acid | | | | | | |
| (D) | Ethanol | | 57 | 57 | 57 | 57 | 57 |
| | Isopropanol | | | | | | |
| Water | Purified water | | 27.60 | 25.60 | 23.27 | 19.87 | 14.20 |
| Total | | | 100 | 100 | 100 | 100 | 100 |
| Component (A) content (% by mass) | | | 4.13 | 4.13 | 4.13 | 4.13 | 4.13 |
| Component (B) content (% by mass) | | | 10 | 12 | 13 | 16 | 21 |
| Component (B1) content (% by mass) | | | 8 | 10 | 1 | 1 | 1 |
| Component (B2) content (% by mass) | | | 2 | 2 | 12 | 15 | 20 |
| Component (B3) content (% by mass) | | | 0 | 0 | 0 | 0 | 0 |
| Component (C) content (% by mass) | | | 1.27 | 1.27 | 2.60 | 3.00 | 3.67 |
| Component (D) content (% by mass) | | | 57 | 57 | 57 | 57 | 57 |
| Components (A) to (D) total content (% by mass) | | | 72.40 | 74.40 | 76.73 | 80.13 | 85.80 |
| A/B | | | 0.41 | 0.34 | 0.32 | 0.26 | 0.20 |
| A/C | | | 3.26 | 3.26 | 1.59 | 1.38 | 1.13 |
| C/B | | | 0.13 | 0.11 | 0.20 | 0.19 | 0.17 |
| A/(B + C) | | | 0.37 | 0.31 | 0.26 | 0.22 | 0.17 |
| Water/D | | | 0.48 | 0.45 | 0.41 | 0.35 | 0.25 |
| B/D | | | 0.18 | 0.21 | 0.23 | 0.28 | 0.37 |
| Acrylic/cellulosic B2/B1 | | | 0.25 | 0.2 | 12 | 15 | 20 |
| Vinyl/cellulosic B3/B1 | | | 0 | 0 | 0 | 0 | 0 |
| Acrylic/vinyl B2/B3 | | | - | - | - | - | - |
| Viscosity (mPa·s) 25°C | | | 80.4 | 121 | 82.5 | 102 | 135 |
| Evaluation | Presence or absence of crystals in coating film | | 5.0 | 5.0 | 5.0 | 5.0 | 5.0 |
| | Sticky feeling in coating film | | 5.0 | 5.0 | 3.8 | 3.2 | 3.0 |
| | Durability of coating film | | 4.0 | 3.0 | 4.0 | 4.0 | 3.0 |
| | Moisture resistance of coating film | | 4.0 | 3.0 | 5.0 | 5.0 | 5.0 |

**Table 3**

| (% by mass) | | | Examples | | | | |
|---|---|---|---|---|---|---|---|
| | | | 16 | 17 | 18 | 19 | 20 |
| (A) | Loxoprofen sodium hydrate | | 1.13 | 1.13 | 1.13 | 1.13 | 1.13 |
| | 1-Menthol | | 3 | 3 | 3 | 3 | 3 |
| (B) | (B1) Hypromellose phthalate | Cellulosic-type | 0.01 | 0.3 | 2 | 1 | 1 |
| | (B1) Ethyl cellulose | Cellulosic-type | | | | | |
| | (B2) Acrylates/diacetoneacrylamide copolymer | Acrylic-type | | | | | |
| | (B3) Polyvinyl butyral | Vinyl-type | 0.01 | 0.6 | 1 | 2 | 6 |
| | Isopropyl myristate | Ester oil | 1 | 1 | 1 | 1 | 1 |
| | Dipropylene glycol | Polyol | | | | | |
| | Polyethylene glycol 400 | Polyol | | | | | |
| | Polysorbate 80 | Nonionic surfactant | | | | | |
| | Squalane | Non-polar oil | | | | | |
| (C) | Diisopropanolamine | Alkanolamine | | | | | |
| | Triethanolamine | Alkanolamine | | | | | |
| | 2-Amino-2-methylpropanol | Alkanolamine | | | | | |
| | 1,3-Butylene glycol | Polyol | | | | | |
| | Propylene glycol monocaprate | Ester oil | | | | | |
| | Polyethylene glycol 4000 | Polyol | | | | | |
| Others | Polyvinylpyrrolidone | Water-soluble polymer | | | | | |
| | Lactic acid | | | | | | |
| (D) | Ethanol | | 57 | 61.75 | 61.75 | 57 | 63 |
| | Isopropanol | | | | | | |
| Water | Purified water | | 37.85 | 32.22 | 30.12 | 34.87 | 24.87 |
| Total | | | 100 | 100 | 100 | 100 | 100 |
| Component (A) content (% by mass) | | | 4.13 | 4.13 | 4.13 | 4.13 | 4.13 |
| Component (B) content (% by mass) | | | 0.02 | 0.9 | 3 | 3 | 7 |
| Component (B1) content (% by mass) | | | 0.01 | 0.3 | 2 | 1 | 1 |
| Component (B2) content (% by mass) | | | 0 | 0 | 0 | 0 | 0 |
| Component (B3) content (% by mass) | | | 0.01 | 0.6 | 1 | 2 | 6 |
| Component (C) content (% by mass) | | | 1.00 | 1.00 | 1.00 | 1.00 | 1.00 |
| Component (D) content (% by mass) | | | 57 | 61.75 | 61.75 | 57 | 63 |
| Components (A) to (D) total content (% by mass) | | | 62.15 | 67.78 | 69.88 | 65.13 | 75.13 |
| A/B | | | 206.50 | 4.59 | 1.38 | 1.38 | 0.59 |
| A/C | | | 4.13 | 4.13 | 4.13 | 4.13 | 4.13 |
| C/B | | | 50.00 | 1.11 | 0.33 | 0.33 | 0.14 |
| A/(B + C) | | | 4.05 | 2.17 | 1.03 | 1.03 | 0.52 |
| Water/D | | | 0.66 | 0.52 | 0.49 | 0.61 | 0.39 |
| B/D | | | 0.00 | 0.01 | 0.05 | 0.05 | 0.11 |
| Acrylic/cellulosic B2/B1 | | | 0 | 0 | 0 | 0 | 0 |
| Vinyl/cellulosic B3/B1 | | | 1 | 2 | 0.5 | 2 | 6 |
| Acrylic/vinyl B2/B3 | | | 0 | 0 | 0 | 0 | 0 |
| Viscosity (mPa·s) 25°C | | | 1.23 | 4.25 | 6.24 | 8.26 | 42.1 |
| Evaluation | Presence or absence of crystals in coating film | | 3.0 | 3.0 | 5.0 | 5.0 | 5.0 |
| | Sticky feeling in coating film | | 5.0 | 5.0 | 5.0 | 5.0 | 5.0 |
| | Durability of coating film | | 3.0 | 5.0 | 5.0 | 5.0 | 5.0 |
| | Moisture resistance of coating film | | 3.0 | 5.0 | 4.0 | 5.0 | 5.0 |

**Table 3 (continued)**

| (% by mass) | | | Examples | | | |
|---|---|---|---|---|---|---|
| | | | 21 | 22 | 23 | 24 |
| (A) | Loxoprofen sodium hydrate | | 1.13 | 1.13 | 1.13 | 1.13 |
| | l-Menthol | | 3 | 3 | 3 | 3 |
| (B) | (B1) Hypromellose phthalate | Cellulosic-type | 1 | 1 | | |
| | (B1) Ethyl cellulose | Cellulosic-type | | | | |
| | (B2) Acrylates/diacetoneacrylamide copolymer | Acrylic-type | | | 10 | 10 |
| | (B3) Polyvinyl butyral | Vinyl-type | 8 | 10 | 0.5 | 1 |
| | Isopropyl myristate | Ester oil | 1 | 1 | 1 | 1 |
| | Dipropylene glycol | Polyol | | | | |
| | Polyethylene glycol 400 | Polyol | | | | |
| | Polysorbate 80 | Nonionic surfactant | | | | |
| | Squalane | Non-polar oil | | | | |
| (C) | Diisopropanolamine | Alkanolamine | | | | |
| | Triethanolamine | Alkanolamine | | | | |
| | 2-Amino-2-methylpropanol | Alkanolamine | | | 1.3 | 1.3 |
| | 1,3-Butylene glycol | Polyol | | | | |
| | Propylene glycol monocaprate | Ester oil | | | | |
| | Polyethylene glycol 4000 | Polyol | | | | |
| Others | Polyvinylpyrrolidone | Water-soluble polymer | | | | |
| | Lactic acid | | | | | |
| (D) | Ethanol | | 63 | 63 | 61.75 | 61.75 |
| | Isopropanol | | | | | |
| Water | Purified water | | 22.87 | 20.87 | 21.29 | 20.79 |
| Total | | | 100 | 100 | 100 | 100 |
| Component (A) content (% by mass) | | | 4.13 | 4.13 | 4.13 | 4.13 |
| Component (B) content (% by mass) | | | 9 | 11 | 10.5 | 11 |
| Component (B1) content (% by mass) | | | 1 | 1 | 0 | 0 |
| Component (B2) content (% by mass) | | | 0 | 0 | 10 | 10 |
| Component (B3) content (% by mass) | | | 8 | 10 | 0.5 | 1 |
| Component (C) content (% by mass) | | | 1.00 | 1.00 | 2.33 | 2.33 |
| Component (D) content (% by mass) | | | 63 | 63 | 61.75 | 61.75 |
| Components (A) to (D) total content (% by mass) | | | 77.13 | 79.13 | 78.71 | 79.21 |
| A/B | | | 0.46 | 0.38 | 0.39 | 0.38 |
| A/C | | | 4.13 | 4.13 | 1.77 | 1.77 |
| C/B | | | 0.11 | 0.09 | 0.22 | 0.21 |
| A/(B + C) | | | 0.41 | 0.34 | 0.32 | 0.31 |
| Water/D | | | 0.36 | 0.33 | 0.34 | 0.34 |
| B/D | | | 0.14 | 0.17 | 0.17 | 0.18 |
| Acrylic/cellulosic B2/B1 | | | 0 | 0 | - | - |
| Vinyl/cellulosic B3/B1 | | | 8 | 10 | - | - |
| Acrylic/vinyl B2/B3 | | | 0 | 0 | 20 | 10 |
| Viscosity (mPa·s) 25°C | | | 124 | 234 | 10.8 | 12.4 |
| Evaluation | Presence or absence of crystals in coating film | | 5.0 | 5.0 | 4.0 | 4.0 |
| | Sticky feeling in coating film | | 5.0 | 5.0 | 3.0 | 3.8 |
| | Durability of coating film | | 4.0 | 3.0 | 4.0 | 4.0 |
| | Moisture resistance of coating film | | 4.0 | 3.0 | 5.0 | 5.0 |

**Table 3 (continued)**

| (% by mass) | | | Examples | | | |
|---|---|---|---|---|---|---|
| | | | 25 | 26 | 27 | 28 |
| (A) | Loxoprofen sodium hydrate | | 1.13 | 1.13 | 1.13 | 1.13 |
| | 1-Menthol | | 3 | 3 | 3 | 3 |
| (B) | (B1) Hypromellose phthalate | Cellulosic-type | | | | |
| | (B1) Ethyl cellulose | Cellulosic-type | | | | |
| | (B2) Acrylates/diacetoneacrylamide copolymer | Acrylic-type | 6 | 5 | 2 | 1 |
| | (B3) Polyvinyl butyral | Vinyl-type | 3 | 5 | 1 | 8 |
| | Isopropyl myristate | Ester oil | 1 | 1 | 1 | 1 |
| | Dipropylene glycol | Polyol | | | | |
| | Polyethylene glycol 400 | Polyol | | | | |
| | Polysorbate 80 | Nonionic surfactant | | | | |
| | Squalane | Non-polar oil | | | | |
| (C) | Diisopropanolamine | Alkanolamine | | | | |
| | Triethanolamine | Alkanolamine | | | | |
| | 2-Amino-2-methylpropanol | Alkanolamine | 0.8 | 0.7 | 0.3 | 0.1 |
| | 1,3-Butylene glycol | Polyol | | | | |
| | Propylene glycol monocaprate | Ester oil | | | | |
| | Polyethylene glycol 4000 | Polyol | | | | |
| Others | Polyvinylpyrrolidone | Water-soluble polymer | | | | |
| | Lactic acid | | | | | |
| (D) | Ethanol | | 61.75 | 61.75 | 57 | 61.75 |
| | Isopropanol | | | | | |
| Water | Purified water | | 23.32 | 22.45 | 34.60 | 23.99 |
| Total | | | 100 | 100 | 100 | 100 |
| Component (A) content (% by mass) | | | 4.13 | 4.13 | 4.13 | 4.13 |
| Component (B) content (% by mass) | | | 9 | 10 | 3 | 9 |
| Component (B1) content (% by mass) | | | 0 | 0 | 0 | 0 |
| Component (B2) content (% by mass) | | | 6 | 5 | 2 | 1 |
| Component (B3) content (% by mass) | | | 3 | 5 | 1 | 8 |
| Component (C) content (% by mass) | | | 1.80 | 1.67 | 1.27 | 1.13 |
| Component (D) content (% by mass) | | | 61.75 | 61.75 | 57 | 61.75 |
| Components (A) to (D) total content (% by mass) | | | 76.68 | 77.55 | 65.40 | 76.01 |
| A/B | | | 0.46 | 0.41 | 1.38 | 0.46 |
| A/C | | | 2.29 | 2.48 | 3.26 | 3.64 |
| C/B | | | 0.20 | 0.17 | 0.42 | 0.13 |
| A/(B + C) | | | 0.38 | 0.35 | 0.97 | 0.41 |
| Water/D | | | 0.38 | 0.36 | 0.61 | 0.39 |
| B/D | | | 0.15 | 0.16 | 0.05 | 0.15 |
| Acrylic/cellulosic B2/B1 | | | - | - | - | - |
| Vinyl/cellulosic B3/B1 | | | - | - | - | - |
| Acrylic/vinyl B2/B3 | | | 2 | 1 | 2 | 0.125 |
| Viscosity (mPa·s) 25°C | | | 18.7 | 24.8 | 5.95 | 102 |
| Evaluation | Presence or absence of crystals in coating film | | 4.0 | 4.0 | 5.0 | 4.0 |
| | Sticky feeling in coating film | | 4.0 | 5.0 | 5.0 | 5.0 |
| | Durability of coating film | | 4.0 | 4.0 | 5.0 | 3.0 |
| | Moisture resistance of coating film | | 4.0 | 4.0 | 5.0 | 3.0 |

**Table 4**

| (% by mass) | | | Examples | | |
|---|---|---|---|---|---|
| | | | 29 | 30 | 31 |
| (A) | Loxoprofen sodium hydrate | | 1.13 | 1.13 | 1.13 |
| | l-Menthol | | 3 | 3 | 3 |
| (B) | (B1) Hypromellose phthalate | Cellulosic-type | 1 | 1 | 1 |
| | (B1) Ethyl cellulose | Cellulosic-type | | | |
| | (B2) Acrylates/diacetoneacrylamide copolymer | Acrylic-type | 2 | 2 | 2 |
| | (B3) Polyvinyl butyral | Vinyl-type | | | |
| | Isopropyl myristate | Ester oil | 1 | 1 | 1 |
| | Dipropylene glycol | Polyol | | | |
| | Polyethylene glycol 400 | Polyol | | | |
| | Polysorbate 80 | Nonionic surfactant | | | |
| | Squalane | Non-polar oil | | | |
| (C) | Diisopropanolamine | Alkanolamine | | | |
| | Triethanolamine | Alkanolamine | | | |
| | 2-Amino-2-methylpropanol | Alkanolamine | 0.3 | 0.3 | 0.3 |
| | 1,3-Butylene glycol | Polyol | | | |
| | Propylene glycol monocaprate | Ester oil | | | |
| | Polyethylene glycol 4000 | Polyol | 1 | 1.5 | 2 |
| Others | Polyvinylpyrrolidone | Water-soluble polymer | 3 | 3 | 3 |
| | Lactic acid | | | | |
| (D) | Ethanol | | 57 | 57 | 57 |
| | Isopropanol | | | | |
| Water | Purified water | | 30.60 | 30.10 | 29.60 |
| Total | | | 100 | 100 | 100 |
| Component (A) content (% by mass) | | | 4.13 | 4.13 | 4.13 |
| Component (B) content (% by mass) | | | 3 | 3 | 3 |
| Component (B1) content (% by mass) | | | 1 | 1 | 1 |
| Component (B2) content (% by mass) | | | 2 | 2 | 2 |
| Component (B3) content (% by mass) | | | 0 | 0 | 0 |
| Component (C) content (% by mass) | | | 2.27 | 2.77 | 3.27 |
| Component (D) content (% by mass) | | | 57 | 57 | 57 |
| Components (A) to (D) total content (% by mass) | | | 66.40 | 66.90 | 67.40 |
| A/B | | | 1.38 | 1.38 | 1.38 |
| A/C | | | 1.82 | 1.49 | 1.26 |
| C/B | | | 0.76 | 0.92 | 1.09 |
| A/(B + C) | | | 0.78 | 0.72 | 0.66 |
| Water/D | | | 0.54 | 0.53 | 0.52 |
| B/D | | | 0.05 | 0.05 | 0.05 |
| Acrylic/cellulosic B2/B1 | | | 2 | 2 | 2 |
| Vinyl/cellulosic B3/B1 | | | 0 | 0 | 0 |
| Acrylic/vinyl B2/B3 | | | - | - | - |
| Viscosity (mPa·s) 25°C | | | 42.9 | 43.4 | 43.6 |
| Evaluation | Presence or absence of crystals in coating film | | 5.0 | 5.0 | 5.0 |
| | Sticky feeling in coating film | | 4.2 | 4.0 | 3.2 |
| | Durability of coating film | | 4.0 | 4.0 | 3.0 |
| | Moisture resistance of coating film | | 5.0 | 4.0 | 3.0 |

**Table 4 (continued)**

| (% by mass) | | | Examples | | |
|---|---|---|---|---|---|
| | | | 32 | 33 | 34 |
| (A) | Loxoprofen sodium hydrate | | 1.13 | 1.13 | 1.13 |
| | 1-Menthol | | 3 | 3 | 3 |
| (B) | (B1) Hypromellose phthalate | Cellulosic-type | 1 | 1 | 1 |
| | (B1) Ethyl cellulose | Cellulosic-type | | | 0.9 |
| | (B2) Acrylates/diacetoneacrylamide copolymer | Acrylic-type | 2 | 2 | 1 |
| | (B3) Polyvinyl butyral | Vinyl-type | | 1 | |
| | Isopropyl myristate | Ester oil | 1 | 1 | |
| | Dipropylene glycol | Polyol | | | |
| | Polyethylene glycol 400 | Polyol | | | |
| | Polysorbate 80 | Nonionic surfactant | | | |
| | Squalane | Non-polar oil | | | |
| (C) | Diisopropanolamine | Alkanolamine | | | |
| | Triethanolamine | Alkanolamine | | | |
| | 2-Amino-2-methylpropanol | Alkanolamine | 0.3 | 0.3 | 0.1 |
| | 1,3-Butylene glycol | Polyol | | | |
| | Propylene glycol monocaprate | Ester oil | | | |
| | Polyethylene glycol 4000 | Polyol | 3 | | |
| Others | Polyvinylpyrrolidone | Water-soluble polymer | 3 | | |
| | Lactic acid | | | | |
| (D) | Ethanol | | 57 | 57 | 57 |
| | Isopropanol | | | | |
| Water | Purified water | | 28.60 | 33.60 | 35.84 |
| Total | | | 100 | 100 | 100 |
| Component (A) content (% by mass) | | | 4.13 | 4.13 | 4.13 |
| Component (B) content (% by mass) | | | 3 | 4 | 2.9 |
| Component (B1) content (% by mass) | | | 1 | 1 | 1.9 |
| Component (B2) content (% by mass) | | | 2 | 2 | 1 |
| Component (B3) content (% by mass) | | | 0 | 1 | 0 |
| Component (C) content (% by mass) | | | 4.27 | 1.27 | 0.13 |
| Component (D) content (% by mass) | | | 57 | 57 | 57 |
| Components (A) to (D) total content (% by mass) | | | 68.40 | 66.40 | 64.16 |
| A/B | | | 1.38 | 1.03 | 1.42 |
| A/C | | | 0.97 | 3.26 | 30.98 |
| C/B | | | 1.42 | 0.32 | 0.05 |
| A/(B + C) | | | 0.57 | 0.78 | 1.36 |
| Water/D | | | 0.50 | 0.59 | 0.63 |
| B/D | | | 0.05 | 0.07 | 0.05 |
| Acrylic/cellulosic B2/B1 | | | 2 | 2 | 0.526 |
| Vinyl/cellulosic B3/B1 | | | 0 | 1 | 0 |
| Acrylic/vinyl B2/B3 | | | - | 2 | - |
| Viscosity (mPa·s) 25°C | | | 45.2 | 45.2 | 7.02 |
| Evaluation | Presence or absence of crystals in coating film | | 5.0 | 5.0 | 5.0 |
| | Sticky feeling in coating film | | 3.0 | 5.0 | 5.0 |
| | Durability of coating film | | 3.0 | 4.0 | 5.0 |
| | Moisture resistance of coating film | | 3.0 | 5.0 | 5.0 |

**Table 5**

| (% by mass) | | | Examples | | | | |
|---|---|---|---|---|---|---|---|
| | | | 35 | 36 | 37 | 38 | 39 |
| (A) | Loxoprofen sodium hydrate | | 1.13 | 1.13 | 1.13 | 1.13 | 1.13 |
| | 1-Menthol | | 3 | 3 | 3 | 3 | 3 |
| (B) | (B1) Hypromellose phthalate | Cellulosic-type | 1 | 1 | 1 | 1 | 1 |
| | (B1) Ethyl cellulose | Cellulosic-type | | | | | |
| | (B2) Acrylates/diacetoneacrylamide copolymer | Acrylic-type | 2 | 2 | 2 | 2 | 2 |
| | (B3) Polyvinyl butyral | Vinyl-type | | | | | |
| | Isopropyl myristate | Ester oil | | | | | |
| | Dipropylene glycol | Polyol | 1 | | | | |
| | Polyethylene glycol 400 | Polyol | | 1 | | | |
| | Polysorbate 80 | Nonionic surfactant | | | 2 | | |
| | Squalane | Non-polar oil | | | 0.1 | | |
| (C) | Diisopropanolamine | Alkanolamine | | | | 0.1 | |
| | Triethanolamine | Alkanolamine | | | | | 0.1 |
| | 2-Amino-2-methylpropanol | Alkanolamine | 0.3 | 0.3 | 0.3 | 0.3 | 0.3 |
| | 1,3-Butylene glycol | Polyol | | | | | |
| | Propylene glycol monocaprate | Ester oil | | | | | |
| | Polyethylene glycol 4000 | Polyol | | | | | |
| Others | Polyvinylpyrrolidone | Water-soluble polymer | | | | | |
| | Lactic acid | | | | | | |
| (D) | Ethanol | | 57 | 57 | 57 | 57 | 57 |
| | Isopropanol | | | | | | |
| Water | Purified water | | 34.60 | 34.60 | 33.50 | 35.50 | 35.50 |
| Total | | | 100 | 100 | 100 | 100 | 100 |
| Component (A) content (% by mass) | | | 4.13 | 4.13 | 4.13 | 4.13 | 4.13 |
| Component (B) content (% by mass) | | | 3 | 3 | 3 | 3 | 3 |
| Component (B1) content (% by mass) | | | 1 | 1 | 1 | 1 | 1 |
| Component (B2) content (% by mass) | | | 2 | 2 | 2 | 2 | 2 |
| Component (B3) content (% by mass) | | | 0 | 0 | 0 | 0 | 0 |
| Component (C) content (% by mass) | | | 1.27 | 1.27 | 2.37 | 0.37 | 0.37 |
| Component (D) content (% by mass) | | | 57 | 57 | 57 | 57 | 57 |
| Components (A) to (D) total content (% by mass) | | | 65.40 | 65.40 | 66.50 | 64.50 | 64.50 |
| A/B | | | 1.38 | 1.38 | 1.38 | 1.38 | 1.38 |
| A/C | | | 3.26 | 3.26 | 1.75 | 11.27 | 11.27 |
| C/B | | | 0.42 | 0.42 | 0.79 | 0.12 | 0.12 |
| A/(B + C) | | | 0.97 | 0.97 | 0.77 | 1.23 | 1.23 |
| Water/D | | | 0.61 | 0.61 | 0.59 | 0.62 | 0.62 |
| B/D | | | 0.05 | 0.05 | 0.05 | 0.05 | 0.05 |
| Acrylic/cellulosic B2/B1 | | | 2 | 2 | 2 | 2 | 2 |
| Vinyl/cellulosic B3/B1 | | | 0 | 0 | 0 | 0 | 0 |
| Acrylic/vinyl B2/B3 | | | - | - | - | - | - |
| Viscosity (mPa·s) 25°C | | | 6.23 | 6.82 | 6.04 | 6.01 | 5.98 |
| Evaluation | Presence or absence of crystals in coating film | | 5.0 | 4.0 | 4.0 | 5.0 | 5.0 |
| | Sticky feeling in coating film | | 4.2 | 4.2 | 4.0 | 3.8 | 4.8 |
| | Durability of coating film | | 5.0 | 5.0 | 5.0 | 5.0 | 5.0 |
| | Moisture resistance of coating film | | 5.0 | 5.0 | 5.0 | 5.0 | 5.0 |

**Table 5 (continued)**

| (% by mass) | | | Examples | | | |
|---|---|---|---|---|---|---|
| | | | 40 | 41 | 42 | 43 |
| (A) | Loxoprofen sodium hydrate | | 1.13 | 1.13 | 1.13 | 1.13 |
| | 1-Menthol | | 3 | 3 | 3 | 3 |
| (B) | (B1) Hypromellose phthalate | Cellulosic-type | 1 | 1 | 1 | 1 |
| | (B1) Ethyl cellulose | Cellulosic-type | | | | |
| | (B2) Acrylates/diacetoneacrylamide copolymer | Acrylic-type | 2 | 2 | 2 | 2 |
| | (B3) Polyvinyl butyral | Vinyl-type | | | | |
| | Isopropyl myristate | Ester oil | | | | |
| | Dipropylene glycol | Polyol | | | | |
| | Polyethylene glycol 400 | Polyol | | | | |
| | Polysorbate 80 | Nonionic surfactant | | | | |
| | Squalane | Non-polar oil | | | | |
| (C) | Diisopropanolamine | Alkanolamine | | | | |
| | Triethanolamine | Alkanolamine | | | | |
| | 2-Amino-2-methylpropanol | Alkanolamine | 0.3 | 0.3 | 0.3 | 0.3 |
| | 1,3-Butylene glycol | Polyol | 5 | 7 | 9 | |
| | Propylene glycol monocaprate | Ester oil | | | | 15 |
| | Polyethylene glycol 4000 | Polyol | | | | |
| Others | Polyvinylpyrrolidone | Water-soluble polymer | | | | |
| | Lactic acid | | | | | |
| (D) | Ethanol | | 57 | 57 | 57 | 57 |
| | Isopropanol | | | | | |
| Water | Purified water | | 30.60 | 28.60 | 26.60 | 20.60 |
| Total | | | 100 | 100 | 100 | 100 |
| Component (A) content (% by mass) | | | 4.13 | 4.13 | 4.13 | 4.13 |
| Component (B) content (% by mass) | | | 3 | 3 | 3 | 3 |
| Component (B1) content (% by mass) | | | 1 | 1 | 1 | 1 |
| Component (B2) content (% by mass) | | | 2 | 2 | 2 | 2 |
| Component (B3) content (% by mass) | | | 0 | 0 | 0 | 0 |
| Component (C) content (% by mass) | | | 5.27 | 7.27 | 9.27 | 15.27 |
| Component (D) content (% by mass) | | | 57 | 57 | 57 | 57 |
| Components (A) to (D) total content (% by mass) | | | 69.40 | 71.40 | 73.40 | 79.40 |
| A/B | | | 1.38 | 1.38 | 1.38 | 1.38 |
| A/C | | | 0.78 | 0.57 | 0.45 | 0.27 |
| C/B | | | 1.76 | 2.42 | 3.09 | 5.09 |
| A/(B + C) | | | 0.50 | 0.40 | 0.34 | 0.23 |
| Water/D | | | 0.54 | 0.50 | 0.47 | 0.36 |
| B/D | | | 0.05 | 0.05 | 0.05 | 0.05 |
| Acrylic/cellulosic B2/B1 | | | 2 | 2 | 2 | 2 |
| Vinyl/cellulosic B3/B1 | | | 0 | 0 | 0 | 0 |
| Acrylic/vinyl B2/B3 | | | - | - | - | - |
| Viscosity (mPa·s) 25°C | | | 6.35 | 6.91 | 6.24 | 6.22 |
| Evaluation | Presence or absence of crystals in coating film | | 5.0 | 5.0 | 4.0 | 5.0 |
| | Sticky feeling in coating film | | 4.2 | 3.2 | 3.0 | 4.2 |
| | Durability of coating film | | 4.0 | 4.0 | 4.0 | 4.0 |
| | Moisture resistance of coating film | | 5.0 | 5.0 | 5.0 | 5.0 |

**Table 5 (continued)**

| (% by mass) | | | Examples | | | |
|---|---|---|---|---|---|---|
| | | | 44 | 45 | 46 | 47 |
| (A) | Loxoprofen sodium hydrate | | 1.13 | 1.13 | 1.13 | 1.13 |
| | 1-Menthol | | 3 | 3 | 3 | 3 |
| (B) | (B1) Hypromellose phthalate | Cellulosic-type | 1 | 1 | 2 | 2 |
| | (B1) Ethyl cellulose | Cellulosic-type | | | | |
| | (B2) Acrylates/diacetoneacrylamide copolymer | Acrylic-type | 2 | 2 | 10 | 10 |
| | (B3) Polyvinyl butyral | Vinyl-type | | | | |
| | Isopropyl myristate | Ester oil | | | | |
| | Dipropylene glycol | Polyol | | | | |
| | Polyethylene glycol 400 | Polyol | | | | |
| | Polysorbate 80 | Nonionic surfactant | | | | |
| | Squalane | Non-polar oil | | | | |
| (C) | Diisopropanolamine | Alkanolamine | | | | |
| | Triethanolamine | Alkanolamine | | | | |
| | 2-Amino-2-methylpropanol | Alkanolamine | 0.3 | 0.3 | 1.3 | 1.3 |
| | 1,3-Butylene glycol | Polyol | | | | |
| | Propylene glycol monocaprate | Ester oil | 20 | 30 | 20 | 30 |
| | Polyethylene glycol 4000 | Polyol | | | | |
| Others | Polyvinylpyrrolidone | Water-soluble polymer | | | | |
| | Lactic acid | | | | | |
| (D) | Ethanol | | 57 | 52.25 | 52.25 | 42.75 |
| | Isopropanol | | | | | |
| Water | Purified water | | 15.60 | 10.35 | 10.29 | 9.79 |
| Total | | | 100 | 100 | 100 | 100 |
| Component (A) content (% by mass) | | | 4.13 | 4.13 | 4.13 | 4.13 |
| Component (B) content (% by mass) | | | 3 | 3 | 12 | 12 |
| Component (B1) content (% by mass) | | | 1 | 1 | 2 | 2 |
| Component (B2) content (% by mass) | | | 2 | 2 | 10 | 10 |
| Component (B3) content (% by mass) | | | 0 | 0 | 0 | 0 |
| Component (C) content (% by mass) | | | 20.27 | 30.27 | 21.33 | 31.33 |
| Component (D) content (% by mass) | | | 57 | 52.25 | 52.25 | 42.75 |
| Components (A) to (D) total content (% by mass) | | | 84.40 | 89.65 | 89.71 | 90.21 |
| A/B | | | 1.38 | 1.38 | 0.34 | 0.34 |
| A/C | | | 0.20 | 0.14 | 0.19 | 0.13 |
| C/B | | | 6.76 | 10.09 | 1.78 | 2.61 |
| A/(B + C) | | | 0.18 | 0.12 | 0.12 | 0.10 |
| Water/D | | | 0.27 | 0.20 | 0.20 | 0.23 |
| B/D | | | 0.05 | 0.06 | 0.23 | 0.28 |
| Acrylic/cellulosic B2/B1 | | | 2 | 2 | 5 | 5 |
| Vinyl/cellulosic B3/B1 | | | 0 | 0 | 0 | 0 |
| Acrylic/vinyl B2/B3 | | | - | - | - | - |
| Viscosity (mPa·s) 25°C | | | 6.12 | 5.92 | 84.4 | 88.2 |
| Evaluation | Presence or absence of crystals in coating film | | 5.0 | 5.0 | 5.0 | 5.0 |
| | Sticky feeling in coating film | | 3.2 | 3.0 | 4.2 | 3.0 |
| | Durability of coating film | | 3.0 | 3.0 | 4.0 | 4.0 |
| | Moisture resistance of coating film | | 4.0 | 3.0 | 5.0 | 4.0 |

**Table 6**

| (% by mass) | | | Examples | | | |
|---|---|---|---|---|---|---|
| | | | 48 | 49 | 50 | 51 |
| (A) | Loxoprofen sodium hydrate | | 1.13 | 3.402 | 5.67 | 1.13 |
| | 1-Menthol | | 3 | 3 | 3 | 3 |
| (B) | (B1) Hypromellose phthalate | Cellulosic-type | 1 | 1 | 1 | 1 |
| | (B1) Ethyl cellulose | Cellulosic-type | | | | |
| | (B2) Acrylates/diacetoneacrylamide copolymer | Acrylic-type | 2 | 2 | 2 | 2 |
| | (B3) Polyvinyl butyral | Vinyl-type | | | | |
| | Isopropyl myristate | Ester oil | 1 | 1 | 1 | 1 |
| | Dipropylene glycol | Polyol | | | | |
| | Polyethylene glycol 400 | Polyol | | | | |
| | Polysorbate 80 | Nonionic surfactant | | | | |
| | Squalane | Non-polar oil | | | | |
| (C) | Diisopropanolamine | Alkanolamine | | | | |
| | Triethanolamine | Alkanolamine | | | | |
| | 2-Amino-2-methylpropanol | Alkanolamine | 0.3 | 0.3 | 0.3 | 0.3 |
| | 1,3-Butylene glycol | Polyol | | | | |
| | Propylene glycol monocaprate | Ester oil | | | | |
| | Polyethylene glycol 4000 | Polyol | | | | |
| Others | Polyvinylpyrrolidone | Water-soluble polymer | | | | |
| | Lactic acid | | 0.18 | 0.18 | 0.18 | |
| (D) | Ethanol | | | 57 | 57 | 88.60 |
| | Isopropanol | | 60 | | | |
| Water | Purified water | | 31.42 | 32.15 | 29.88 | 3 |
| Total | | | 100 | 100 | 100 | 100 |
| Component (A) content (% by mass) | | | 4.13 | 6.402 | 8.67 | 4.13 |
| Component (B) content (% by mass) | | | 3 | 3 | 3 | 3 |
| Component (B1) content (% by mass) | | | 1 | 1 | 1 | 1 |
| Component (B2) content (% by mass) | | | 2 | 2 | 2 | 2 |
| Component (B3) content (% by mass) | | | 0 | 0 | 0 | 0 |
| Component (C) content (% by mass) | | | 1.27 | 1.27 | 1.27 | 1.27 |
| Component (D) content (% by mass) | | | 60 | 57 | 57 | 88.60 |
| Components (A) to (D) total content (% by mass) | | | 68.40 | 67.67 | 69.94 | 97 |
| A/B | | | 1.38 | 2.13 | 2.89 | 1.38 |
| A/C | | | 3.26 | 5.05 | 6.85 | 3.26 |
| C/B | | | 0.42 | 0.42 | 0.42 | 0.42 |
| A/(B + C) | | | 0.97 | 1.50 | 2.03 | 0.97 |
| Water/D | | | 0.52 | 0.56 | 0.52 | 0.03 |
| B/D | | | 0.05 | 0.05 | 0.05 | 0.03 |
| Acrylic/cellulosic B2/B1 | | | 2 | 2 | 2 | 2 |
| Vinyl/cellulosic B3/B1 | | | 0 | 0 | 0 | 0 |
| Acrylic/vinyl B2/B3 | | | - | - | - | - |
| Viscosity (mPa·s) 25°C | | | 6.23 | 6.12 | 6.22 | 5.33 |
| Evaluation | Presence or absence of crystals in coating film | | 5.0 | 5.0 | 5.0 | 5.0 |
| | Sticky feeling in coating film | | 4.3 | 5.0 | 4.3 | 5.0 |
| | Durability of coating film | | 5.0 | 5.0 | 5.0 | 4.0 |
| | Moisture resistance of coating film | | 5.0 | 5.0 | 5.0 | 3.0 |

**Table 6 (continued)**

| (% by mass) | | | Examples | | | |
|---|---|---|---|---|---|---|
| | | | 52 | 53 | 54 | 55 |
| (A) | Loxoprofen sodium hydrate | | 1.13 | 1.13 | 1.13 | 1.13 |
| | 1-Menthol | | 3 | 3 | 3 | 3 |
| (B) | (B1) Hypromellose phthalate | Cellulosic-type | 1 | 1 | 1 | 1 |
| | (B1) Ethyl cellulose | Cellulosic-type | | | | |
| | (B2) Acrylates/diacetoneacrylamide copolymer | Acrylic-type | 2 | 2 | 2 | 2 |
| | (B3) Polyvinyl butyral | Vinyl-type | | | | |
| | Isopropyl myristate | Ester oil | 1 | 1 | 1 | 1 |
| | Dipropylene glycol | Polyol | | | | |
| | Polyethylene glycol 400 | Polyol | | | | |
| | Polysorbate 80 | Nonionic surfactant | | | | |
| | Squalane | Non-polar oil | | | | |
| (C) | Diisopropanolamine | Alkanolamine | | | | |
| | Triethanolamine | Alkanolamine | | | | |
| | 2-Amino-2-methylpropanol | Alkanolamine | 0.3 | 0.3 | 0.3 | 0.3 |
| | 1,3-Butylene glycol | Polyol | | | | |
| | Propylene glycol monocaprate | Ester oil | | | | |
| | Polyethylene glycol 4000 | Polyol | | | | |
| Others | Polyvinylpyrrolidone | Water-soluble polymer | | | | |
| | Lactic acid | | | | | |
| (D) | Ethanol | | 81.60 | 51.60 | 41.60 | 21.60 |
| | Isopropanol | | | | | |
| Water | Purified water | | 10 | 40 | 50 | 70 |
| Total | | | 100 | 100 | 100 | 100 |
| Component (A) content (% by mass) | | | 4.13 | 4.13 | 4.13 | 4.13 |
| Component (B) content (% by mass) | | | 3 | 3 | 3 | 3 |
| Component (B1) content (% by mass) | | | 1 | 1 | 1 | 1 |
| Component (B2) content (% by mass) | | | 2 | 2 | 2 | 2 |
| Component (B3) content (% by mass) | | | 0 | 0 | 0 | 0 |
| Component (C) content (% by mass) | | | 1.27 | 1.27 | 1.27 | 1.27 |
| Component (D) content (% by mass) | | | 81.60 | 51.60 | 41.60 | 21.60 |
| Components (A) to (D) total content (% by mass) | | | 90 | 60 | 50 | 30 |
| A/B | | | 1.38 | 1.38 | 1.38 | 1.38 |
| A/C | | | 3.26 | 3.26 | 3.26 | 3.26 |
| C/B | | | 0.42 | 0.42 | 0.42 | 0.42 |
| A/(B + C) | | | 0.97 | 0.97 | 0.97 | 0.97 |
| Water/D | | | 0.12 | 0.78 | 1.20 | 3.24 |
| B/D | | | 0.04 | 0.06 | 0.07 | 0.14 |
| Acrylic/cellulosic B2/B1 | | | 2 | 2 | 2 | 2 |
| Vinyl/cellulosic B3/B1 | | | 0 | 0 | 0 | 0 |
| Acrylic/vinyl B2/B3 | | | - | - | - | - |
| Viscosity (mPa·s) 25°C | | | 5.22 | 5.08 | 4.26 | 4.01 |
| Evaluation | Presence or absence of crystals in coating film | | 5.0 | 5.0 | 4.0 | 3.0 |
| | Sticky feeling in coating film | | 5.0 | 3.3 | 3.2 | 3.0 |
| | Durability of coating film | | 4.0 | 3.0 | 3.0 | 3.0 |
| | Moisture resistance of coating film | | 4.0 | 5.0 | 4.0 | 3.0 |

**Table 7**

| (% by mass) | | | Examples | | Comparative Example | | |
|---|---|---|---|---|---|---|---|
| | | | 1 | 30 | 1 | 2 | 3 |
| (A) | Loxoprofen sodium hydrate | | 1.13 | 1.13 | 1.13 | 1.13 | 1.13 |
| | 1-Menthol | | 3 | 3 | 3 | 3 | 3 |
| (B) | (B1) Hypromellose phthalate | Cellulosic-type | 1 | 1 | | | 3 |
| | (B1) Ethyl cellulose | Cellulosic-type | | | | | |
| | (B2) Acrylates/diacetoneacrylamide copolymer | Acrylic-type | 2 | 2 | | 3 | |
| | (B2) Alkyl acrylate-vinyl acetate copolymer | Acrylic-type | | | | | |
| | (B2) Alkyl acrylate copolymer | Acrylic-type | | | | | |
| | (B3) Polyvinyl butyral | Vinyl-type | | | | | |
| | Isopropyl myristate | Ester oil | 1 | 1 | 1 | 1 | 1 |
| | Dipropylene glycol | Polyol | | | | | |
| | Polyethylene glycol 400 | Polyol | | | | | |
| | Polysorbate 80 | Nonionic surfactant | | | | | |
| | Squalane | Non-polar oil | | | | | |
| (C) | Diisopropanolamine | Alkanolamine | | | | | |
| | Triethanolamine | Alkanolamine | | | | | |
| | 2-Amino-2-methylpropanol | Alkanolamine | 0.3 | 0.3 | | 0.4 | |
| | 1,3-Butylene glycol | Ester oil | | | | | |
| | Propylene glycol monocaprate | Polyol | | | | | |
| | Polyethylene glycol 4000 | Polyol | | 1.5 | | | |
| Others | Polyvinylpyrrolidone | Water-soluble polymer | | 3 | | | |
| | Lactic acid | | 0.18 | | | | |
| (D) | Ethanol | | 57 | 57 | 57 | 57 | 57 |
| | Isopropanol | | | | | | |
| Water | Purified water | | 34.42 | 30.10 | 37.87 | 34.47 | 34.87 |
| Total | | | 100 | 100 | 100 | 100 | 100 |
| Component (A) content (% by mass) | | | 4.13 | 4.13 | 4.13 | 4.13 | 4.13 |
| Component (B) content (% by mass) | | | 3 | 2 | 0 | 3 | 3 |
| Component (B1) content (% by mass) | | | 1 | 2 | 0 | 0 | 3 |
| Component (B2) content (% by mass) | | | 2 | 0 | 0 | 3 | 0 |
| Component (B3) content (% by mass) | | | 0 | 0 | 0 | 0 | 0 |
| Component (C) content (% by mass) | | | 1.27 | 1.27 | 1 | 1.40 | 1 |
| Component (D) content (% by mass) | | | 57 | 57 | 57 | 57 | 57 |
| Components (A) to (D) total content (% by mass) | | | 65.40 | 64.40 | 62.13 | 65.53 | 65.13 |
| A/B | | | 1.38 | 2.07 | - | 1.38 | 1.38 |
| A/C | | | 3.26 | 3.26 | 4.13 | 2.95 | 4.13 |
| C/B | | | 0.42 | 0.63 | - | 0.47 | 0.33 |
| A/(B + C) | | | 0.97 | 1.26 | 4.13 | 0.94 | 1.03 |
| Water/D | | | 0.60 | 0.53 | 0.66 | 0.60 | 0.61 |
| B/D | | | 0.05 | 0.04 | 0.00 | 0.05 | 0.05 |
| Acrylic/cellulosic B2/B1 | | | 2 | 0 | - | - | 0 |
| Vinyl/cellulosic B3/B1 | | | 0 | 0 | - | - | 0 |
| Acrylic/vinyl B2/B3 | | | - | - | - | - | - |
| Viscosity (mPa·s) 25°C | | | 6.08 | 43.4 | 1.04 | 6.34 | 15.3 |
| Evaluation | Presence or absence of crystals in coating film | | 5.0 | 5.0 | 1.0 | 4.0 | 5.0 |
| | Sticky feeling in coating film | | 5.0 | 4.0 | 5.0 | 1.0 | 5.0 |
| | Durability of coating film | | 5.0 | 4.0 | 1.0 | 2.0 | 4.0 |
| | Moisture resistance of coating film | | 5.0 | 4.0 | 1.0 | 5.0 | 2.0 |

**Table 7 (continued)**

| (% by mass) | | | Comparative Example | | | | |
|---|---|---|---|---|---|---|---|
| | | | 4 | 5 | 6 | 7 | 8 |
| (A) | Loxoprofen sodium hydrate | | 1.13 | 1.13 | 1.13 | 1.13 | 1.13 |
| | 1-Menthol | | 3 | 3 | 3 | 3 | 3 |
| (B) | (B1) Hypromellose phthalate | Cellulosic-type | | | | 1 | 1 |
| | (B1) Ethyl cellulose | Cellulosic-type | | | | | |
| | (B2) Acrylates/diacetoneacrylamide copolymer | Acrylic-type | | | | 2 | 2 |
| | (B2) Alkyl acrylate-vinyl acetate copolymer | Acrylic-type | | 0.225 | 1 | | |
| | (B2) Alkyl acrylate copolymer | Acrylic-type | | 1.8 | 2 | | |
| | (B3) Polyvinyl butyral | Vinyl-type | 3 | | | | |
| | Isopropyl myristate | Ester oil | 1 | 1 | | | 1 |
| | Dipropylene glycol | Polyol | | | 1 | | |
| | Polyethylene glycol 400 | Polyol | | | | | |
| | Polysorbate 80 | Nonionic surfactant | | | | | |
| | Squalane | Non-polar oil | | | | | 60 |
| (C) | Diisopropanolamine | Alkanolamine | | | | | |
| | Triethanolamine | Alkanolamine | | | | | |
| | 2-Amino-2-methylpropanol | Alkanolamine | | | | | 0.3 |
| | 1,3-Butylene glycol | Ester oil | | | | | |
| | Propylene glycol monocaprate | Polyol | | | | | |
| | Polyethylene glycol 4000 | Polyol | | | | | |
| Others | Polyvinylpyrrolidone | Water-soluble polymer | | | | | |
| | Lactic acid | | | 0.18 | 0.18 | | |
| (D) | Ethanol | | 57 | 89.67 | 87.45 | 57 | |
| | Isopropanol | | | | | | |
| Water | Purified water | | 34.87 | 2.995 | 4.242 | 35.87 | 31.60 |
| Total | | | 100 | 100 | 100 | 100 | 100 |
| Component (A) content (% by mass) | | | 4.13 | 4.13 | 4.13 | 4.13 | 4.13 |
| Component (B) content (% by mass) | | | 3 | 2.025 | 3 | 3 | 3 |
| Component (B1) content (% by mass) | | | 0 | 0 | 0 | 1 | 1 |
| Component (B2) content (% by mass) | | | 0 | 2.025 | 3 | 2 | 2 |
| Component (B3) content (% by mass) | | | 3 | 0 | 0 | 0 | 0 |
| Component (C) content (% by mass) | | | 1 | 1 | 1 | 0 | 61.27 |
| Component (D) content (% by mass) | | | 57 | 89.67 | 87.45 | 57 | 0 |
| Components (A) to (D) total content (% by mass) | | | 65.13 | 96.83 | 95.58 | 64.13 | 68.40 |
| A/B | | | 1.38 | 2.04 | 1.38 | 1.38 | 1.38 |
| A/C | | | 4.13 | 4.13 | 4.13 | - | 0.07 |
| C/B | | | 0.33 | 0.49 | 0.33 | 0 | 20.42 |
| A/(B + C) | | | 1.03 | 1.37 | 1.03 | 1 | 0.06 |
| Water/D | | | 0.61 | 0.03 | 0.05 | 1 | - |
| B/D | | | 0.05 | 0.02 | 0.03 | 0.05 | - |
| Acrylic/cellulosic B2/B1 | | | - | - | - | 2 | 2 |
| Vinyl/cellulosic B3/B1 | | | - | - | - | 0 | 0 |
| Acrylic/vinyl B2/B3 | | | 0 | - | - | - | - |
| Viscosity (mPa·s) 25°C | | | 14.2 | 6.22 | 8.34 | 6.1 | 82.3 |
| Evaluation | Presence or absence of crystals in coating film | | 4.0 | 3.0 | 3.0 | 5.0 | 2.0 |
| | Sticky feeling in coating film | | 3.8 | 4.2 | 4.0 | 5.0 | 3.0 |
| | Durability of coating film | | 2.0 | 2.0 | 2.0 | 2.0 | 3.0 |
| | Moisture resistance of coating film | | 1.0 | 1.0 | 1.0 | 2.0 | 1.0 |

**Table 8**

| (% by mass) | | Examples | | | | |
|---|---|---|---|---|---|---|
| | | 56 | 57 | 58 | 59 | 60 |
| | Betamethasone valerate | 0.12 | | | | |
| | dl-Camphor | | 1 | | | |
| | Terbinafine hydrochloride | | | 1 | | |
| | Clotrimazole | | | | 0.4 | |
| | Tolnaftate | | | | | 0.2 |
| | Minoxidil | | | | | |
| (A) | Pyridoxine hydrochloride | | | | | |
| | Glycol salicylate | | | | | |
| | Felbinac | | | | | |
| | 1-Menthol | | | | | |
| | Nonylic acid vanillylamide | | | | | |
| | Isopropylmethylphenol | | | | | |
| | Allantoin | | | | | |
| (B) | (B1) Hypromellose phthalate | 0.5 | 1.0 | 0.3 | 1.0 | 0.3 |
| | (B2) Acrylates/diacetoneacrylamide copolymer | 2.0 | 2.0 | 0.6 | 2.0 | 0.6 |
| (C) | Polysorbate 80 | | 1.0 | 1.0 | 1.0 | 1.0 |
| | Diisopropanolamine | | | | | |
| | 2-Amino-2-methylpropanol | 0.3 | 0.3 | 0.1 | 0.3 | 0.1 |
| | Triethanolamine | | | | | |
| | 1,3-Butylene glycol | 0.2 | | | | |
| (D) | Ethanol | 55.0 | 57.0 | 70.0 | 57.0 | 70.0 |
| Others | Carboxyvinyl polymer | | | | | |
| Water | Purified water | 41.9 | 37.7 | 27.0 | 38.3 | 27.8 |
| Total | | 100 | 100 | 100 | 100 | 100 |
| Component (A) content (% by mass) | | 0.12 | 1.00 | 1.00 | 0.40 | 0.20 |
| Component (B) content (% by mass) | | 2.5 | 3 | 0.9 | 3 | 0.9 |
| Component (B1) content (% by mass) | | 0.5 | 1 | 0.3 | 1 | 0.3 |
| Component (B2) content (% by mass) | | 2.0 | 2 | 0.6 | 2 | 0.6 |
| Component (C) content (% by mass) | | 0.5 | 1.3 | 1.1 | 1.3 | 1.1 |
| Component (D) content (% by mass) | | 41.9 | 37.7 | 27.0 | 38.3 | 27.8 |
| Components (A) to (D) total content (% by mass) | | 45.5 | 44.0 | 30.3 | 44.0 | 30.3 |
| A/B | | 0.05 | 0.33 | 1.11 | 0.13 | 0.22 |
| A/C | | 0.26 | 0.79 | 0.93 | 0.32 | 0.19 |
| C/B | | 0.19 | 0.42 | 1.20 | 0.42 | 1.20 |
| A/(B + C) | | 0.040 | 0.234 | 0.505 | 0.094 | 0.101 |
| Water/D | | 1.00 | 1.00 | 1.00 | 1.00 | 1.00 |
| B/D | | 0.06 | 0.08 | 0.03 | 0.08 | 0.03 |
| B2/B1 | | 4 | 2 | 2 | 2 | 2 |
| Viscosity (mPa·s) 25°C | | 4.28 | 6.95 | 3.02 | 7.98 | 3.11 |
| Evaluation | Presence or absence of crystals in coating film | 4.0 | 5.0 | 3.0 | 5.0 | 3.0 |
| | Sticky feeling in coating film | 4.8 | 5.0 | 5.0 | 5.0 | 5.0 |
| | Durability of coating film | 5.0 | 5.0 | 5.0 | 5.0 | 5.0 |
| | Moisture resistance of coating film | 5.0 | 5.0 | 5.0 | 5.0 | 5.0 |

**Table 8 (continued)**

| (% by mass) | | Examples | | | | |
|---|---|---|---|---|---|---|
| | | 61 | 62 | 63 | 64 | 65 |
| | Betamethasone valerate | | | | | |
| | d1-Camphor | | | | | |
| | Terbinafine hydrochloride | | | | | |
| | Clotrimazole | | | | | |
| | Tolnaftate | | | | | |
| (A) | Minoxidil | 5 | 5 | | | |
| | Pyridoxine hydrochloride | | 0.05 | | | |
| | Glycol salicylate | | | 3.00 | 3.00 | 3.00 |
| | Felbinac | | | | | |
| | 1-Menthol | | | | | |
| | Nonylic acid vanillylamide | | | | | |
| | Isopropylmethylphenol | | | | | |
| | Allantoin | | 0.5 | | | |
| (B) | (B1) Hypromellose phthalate | 0.3 | 0.3 | 1.0 | 1.0 | 1.0 |
| | (B2) Acrylates/diacetoneacrylamide copolymer | 0.6 | 0.6 | 2.0 | 2.0 | 2.0 |
| (C) | Polysorbate 80 | 1.0 | 1.0 | 1.0 | | |
| | Diisopropanolamine | | | | | |
| | 2-Amino-2-methylpropanol | 0.1 | 0.1 | 0.3 | 0.3 | 0.3 |
| | Triethanolamine | | | | 0.3 | 0.6 |
| | 1,3-Butylene glycol | 5.0 | 5.0 | | | |
| (D) | Ethanol | 70.0 | 57.0 | 57.0 | 40.0 | 40.0 |
| Others | Carboxyvinyl polymer | | | | 0.2 | 0.4 |
| Water | Purified water | 18.0 | 30.5 | 35.7 | 53.2 | 52.7 |
| Total | | 100 | 100 | 100 | 100 | 100 |
| Component (A) content (% by mass) | | 5.00 | 5.55 | 3.00 | 3.00 | 3.00 |
| Component (B) content (% by mass) | | 0.9 | 0.9 | 3 | 3 | 3 |
| Component (B1) content (% by mass) | | 0.3 | 0.3 | 1 | 1 | 1 |
| Component (B2) content (% by mass) | | 0.6 | 0.6 | 2 | 2 | 2 |
| Component (C) content (% by mass) | | 6.1 | 6.1 | 1.3 | 0.6 | 0.9 |
| Component (D) content (% by mass) | | 18.0 | 30.5 | 35.7 | 53.2 | 52.7 |
| Components (A) to (D) total content (% by mass) | | 30.3 | 43.3 | 44.0 | 60.8 | 60.6 |
| A/B | | 5.56 | 6.17 | 1.00 | 1.00 | 1.00 |
| A/C | | 0.82 | 0.91 | 2.37 | 5.29 | 3.46 |
| C/B | | 6.76 | 6.76 | 0.42 | 0.19 | 0.29 |
| A/(B + C) | | 0.716 | 0.795 | 0.703 | 0.841 | 0.776 |
| Water/D | | 1.00 | 1.00 | 1.00 | 1.00 | 1.00 |
| B/D | | 0.05 | 0.03 | 0.08 | 0.06 | 0.06 |
| B2/B1 | | 2 | 2 | 2 | 2 | 2 |
| Viscosity (mPa·s) 25°C | | 3.27 | 7.62 | 6.34 | 560 | 4200 |
| Evaluation | Presence or absence of crystals in coating film | 3.0 | 3.0 | 5.0 | 5.0 | 5.0 |
| | Sticky feeling in coating film | 5.0 | 5.0 | 5.0 | 4.2 | 4.0 |
| | Durability of coating film | 5.0 | 5.0 | 5.0 | 4.0 | 4.0 |
| | Moisture resistance of coating film | 5.0 | 5.0 | 5.0 | 4.0 | 3.0 |

**Table 8 (continued)**

| (% by mass) | | Examples | | | |
|---|---|---|---|---|---|
| | | 66 | 67 | 68 | 69 |
| | Betamethasone valerate | | | | |
| | d1-Camphor | | | | |
| | Terbinafine hydrochloride | | | | |
| | Clotrimazole | | | | |
| | Tolnaftate | | | | |
| | Minoxidil | | | | |
| (A) | Pyridoxine hydrochloride | | | | |
| | Glycol salicylate | | | 3.00 | |
| | Felbinac | 3.00 | | | |
| | 1-Menthol | | 6.00 | | |
| | Nonylic acid vanillylamide | | | 0.01 | |
| | Isopropylmethylphenol | | | | 1.00 |
| | Allantoin | | | | |
| (B) | (B1) Hypromellose phthalate | 1.0 | 1.0 | 1.0 | 1.0 |
| | (B2) Acrylates/diacetoneacrylamide copolymer | 2.0 | 2.0 | 2.0 | 2.0 |
| (C) | Polysorbate 80 | 1.0 | | 0.5 | 1.0 |
| | Diisopropanolamine | 0.6 | | | |
| | 2-Amino-2-methylpropanol | 0.3 | 0.3 | 0.3 | 0.3 |
| | Triethanolamine | | | 0.6 | |
| | 1,3-Butylene glycol | | | | |
| (D) | Ethanol | 70.0 | 57.0 | 45.0 | 57.0 |
| Others | Carboxyvinyl polymer | | | 0.5 | |
| Water | Purified water | 22.1 | 33.7 | 47.1 | 37.7 |
| Total | | 100 | 100 | 100 | 100 |
| Component (A) content (% by mass) | | 3.00 | 6.00 | 3.01 | 1.00 |
| Component (B) content (% by mass) | | 3 | 3 | 3 | 3 |
| Component (B1) content (% by mass) | | 1 | 1 | 1 | 1 |
| Component (B2) content (% by mass) | | 2 | 2 | 2.0 | 2 |
| Component (C) content (% by mass) | | 1.9 | 0.3 | 1.4 | 1.3 |
| Component (D) content (% by mass) | | 22.1 | 33.7 | 47.1 | 37.7 |
| Components (A) to (D) total content (% by mass) | | 31.0 | 44.0 | 55.5 | 44.0 |
| A/B | | 1.00 | 2.00 | 1.003 | 0.33 |
| A/C | | 1.61 | 22.51 | 2.203 | 0.79 |
| C/B | | 0.62 | 0.09 | 0.46 | 0.42 |
| A/(B + C) | | 0.616 | 1.837 | 0.689 | 0.234 |
| Water/D | | 1.00 | 1.00 | 1.00 | 1.00 |
| B/D | | 0.14 | 0.09 | 0.06 | 0.08 |
| B2/B1 | | 2 | 2 | 2 | 2 |
| Viscosity (mPa·s) 25°C | | 7.33 | 7.58 | 9900 | 7.50 |
| Evaluation | Presence or absence of crystals in coating film | 5.0 | 5.0 | 5.0 | 5.0 |
| | Sticky feeling in coating film | 5.0 | 5.0 | 3.3 | 5.0 |
| | Durability of coating film | 5.0 | 5.0 | 3.0 | 5.0 |
| | Moisture resistance of coating film | 5.0 | 5.0 | 3.0 | 5.0 |

**Table 9**

| (% by mass) | | Examples | | | |
|---|---|---|---|---|---|
| | | 70 | 71 | 72 | 73 |
| | Isopropylmethylphenol | | | | |
| | Benzalkonium chloride | 0.10 | | | |
| | Ibuprofen piconol | | 3.00 | | |
| | Glycyrrhetinic acid | | | 0.30 | |
| | Dipotassium glycyrrhizinate | | | | 0.50 |
| (A) | Heparinoid | | | | |
| | Tranexamic acid | | | | |
| | Prednisolone valerate acetate | | | | |
| | Salicylic acid | | | | |
| | Ufenamate | | | | |
| | Chlorpheniramine maleate | | | | |
| | Dexamethasone acetate | | | | |
| | Crotamiton | | | | |
| (B) | (B1) Hypromellose phthalate | 0.5 | 1.0 | 1.0 | 1.0 |
| | (B2) Acrylates/diacetoneacrylamide copolymer | 1.0 | 2.0 | 2.0 | 2.0 |
| (C) | Polysorbate 80 | 1.0 | 1.0 | 1.0 | 1.0 |
| | Diisopropanolamine | | | | |
| | 2-Amino-2-methylpropanol | 0.1 | 0.3 | 0.3 | 0.3 |
| | Triethanolamine | | | | |
| | 1,3-Butylene glycol | 0.5 | | | |
| (D) | Ethanol | 57.0 | 57.0 | 57.0 | 57.0 |
| Others | Carboxyvinyl polymer | | | | |
| Water | Purified water | 39.8 | 35.7 | 38.4 | 38.2 |
| Total | | 100 | 100 | 100 | 100 |
| Component (A) content (% by mass) | | 0.10 | 3.00 | 0.30 | 0.50 |
| Component (B) content (% by mass) | | 1.5 | 3 | 3 | 3 |
| Component (B1) content (% by mass) | | 0.5 | 1 | 1 | 1 |
| Component (B2) content (% by mass) | | 1 | 2 | 2 | 2 |
| Component (C) content (% by mass) | | 1.6 | 1.3 | 1.3 | 1.3 |
| Component (D) content (% by mass) | | 39.8 | 35.7 | 38.4 | 38.2 |
| Components (A) to (D) total content (% by mass) | | 43.5 | 44.0 | 44.0 | 44.0 |
| A/B | | 0.07 | 1.00 | 0.10 | 0.17 |
| A/C | | 0.06 | 2.37 | 0.24 | 0.39 |
| C/B | | 1.09 | 0.42 | 0.42 | 0.42 |
| A/(B + C) | | 0.032 | 0.703 | 0.070 | 0.117 |
| Water/D | | 1.00 | 1.00 | 1.00 | 1.00 |
| B/D | | 0.04 | 0.08 | 0.08 | 0.08 |
| B2/B1 | | 2 | 2 | 2 | 2 |
| Viscosity (mPa·s) 25°C | | 6.43 | 7.04 | 6.21 | 6.87 |
| Evaluation | Presence or absence of crystals in coating film | 4.0 | 5.0 | 5.0 | 5.0 |
| | Sticky feeling in coating film | 5.0 | 5.0 | 5.0 | 5.0 |
| | Durability of coating film | 5.0 | 5.0 | 5.0 | 5.0 |
| | Moisture resistance of coating film | 5.0 | 5.0 | 5.0 | 5.0 |

**Table 9 (continued)**

| (% by mass) | | Examples | | | |
|---|---|---|---|---|---|
| | | 74 | 75 | 76 | 77 |
| | Isopropylmethylphenol | | | | |
| | Benzalkonium chloride | | | | |
| | Ibuprofen piconol | | | | |
| | Glycyrrhetinic acid | | | | |
| | Dipotassium glycyrrhizinate | | | | |
| | Heparinoid | 0.30 | | | |
| (A) | Tranexamic acid | | 3.00 | | |
| | Prednisolone valerate acetate | | | 0.30 | |
| | Salicylic acid | | | | 0.50 |
| | Ufenamate | | | | |
| | Chlorpheniramine maleate | | | | |
| | Dexamethasone acetate | | | | |
| | Crotamiton | | | | |
| (B) | (B1) Hypromellose phthalate | 0.3 | 0.3 | 1.0 | 0.3 |
| | (B2) Acrylates/diacetoneacrylamide copolymer | 0.6 | 0.6 | 2.0 | 0.6 |
| (C) | Polysorbate 80 | 1.0 | 1.0 | 1.0 | 1.0 |
| | Diisopropanolamine | | | | |
| | 2-Amino-2-methylpropanol | 0.1 | 0.1 | 0.3 | 0.1 |
| | Triethanolamine | | | | |
| | 1,3-Butylene glycol | | | | |
| (D) | Ethanol | 50.0 | 50.0 | 57.0 | 70.0 |
| Others | Carboxyvinyl polymer | | | | |
| Water | Purified water | 47.7 | 45.0 | 38.4 | 27.5 |
| Total | | 100 | 100 | 100 | 100 |
| Component (A) content (% by mass) | | 0.30 | 3.00 | 0.30 | 0.50 |
| Component (B) content (% by mass) | | 0.9 | 0.9 | 3 | 0.9 |
| Component (B1) content (% by mass) | | 0.3 | 0.3 | 1 | 0.3 |
| Component (B2) content (% by mass) | | 0.6 | 0.6 | 2 | 0.6 |
| Component (C) content (% by mass) | | 1.1 | 1.1 | 1.3 | 1.1 |
| Component (D) content (% by mass) | | 47.7 | 45.0 | 38.4 | 27.5 |
| Components (A) to (D) total content (% by mass) | | 50.3 | 50.3 | 44.0 | 30.3 |
| A/B | | 0.33 | 3.33 | 0.10 | 0.56 |
| A/C | | 0.28 | 2.78 | 0.24 | 0.46 |
| C/B | | 1.20 | 1.20 | 0.42 | 1.20 |
| A/(B + C) | | 0.152 | 1.515 | 0.070 | 0.253 |
| Water/D | | 1.00 | 1.00 | 1.00 | 1.00 |
| B/D | | 0.02 | 0.02 | 0.08 | 0.03 |
| B2/B1 | | 2 | 2 | 2 | 2 |
| Viscosity (mPa·s) 25°C | | 3.41 | 3.62 | 6.88 | 4.12 |
| Evaluation | Presence or absence of crystals in coating film | 4.0 | 4.0 | 5.0 | 3.0 |
| | Sticky feeling in coating film | 4.2 | 4.2 | 5.0 | 4.8 |
| | Durability of coating film | 4.0 | 4.0 | 5.0 | 5.0 |
| | Moisture resistance of coating film | 4.0 | 4.0 | 5.0 | 5.0 |

**Table 9 (continued)**

| (% by mass) | | Examples | | | |
|---|---|---|---|---|---|
| | | 78 | 79 | 80 | 81 |
| | Isopropylmethylphenol | | | 0.1 | |
| | Benzalkonium chloride | | | | |
| | Ibuprofen piconol | | | | |
| | Glycyrrhetinic acid | | | | |
| | Dipotassium glycyrrhizinate | | | | |
| | Heparinoid | | | | |
| (A) | Tranexamic acid | | | | |
| | Prednisolone valerate acetate | | | | |
| | Salicylic acid | | | | |
| | Ufenamate | 5.00 | | | |
| | Chlorpheniramine maleate | | 0.20 | | |
| | Dexamethasone acetate | | | 0.025 | |
| | Crotamiton | | | | 10.00 |
| (B) | (B1) Hypromellose phthalate | 0.3 | 1.0 | 0.5 | 1.0 |
| | (B2) Acrylates/diacetoneacrylamide copolymer | 0.6 | 2.0 | 2.0 | 1.0 |
| (C) | Polysorbate 80 | 1.0 | 1.0 | | 0.1 |
| | Diisopropanolamine | | | | |
| | 2-Amino-2-methylpropanol | 0.1 | 0.3 | 0.3 | 0.1 |
| | Triethanolamine | | | | |
| | 1,3-Butylene glycol | | | 0.1 | |
| (D) | Ethanol | 70.0 | 57.0 | 55.0 | 57.0 |
| Others | Carboxyvinyl polymer | | | | |
| Water | Purified water | 23.0 | 38.5 | 42.0 | 30.8 |
| Total | | 100 | 100 | 100 | 100 |
| Component (A) content (% by mass) | | 5.00 | 0.20 | 0.13 | 10.00 |
| Component (B) content (% by mass) | | 0.9 | 3 | 2.5 | 2 |
| Component (B1) content (% by mass) | | 0.3 | 1 | 0.5 | 1 |
| Component (B2) content (% by mass) | | 0.6 | 2 | 2 | 1 |
| Component (C) content (% by mass) | | 1.1 | 1.3 | 0.4 | 0.2 |
| Component (D) content (% by mass) | | 23.0 | 38.5 | 42.0 | 30.8 |
| Components (A) to (D) total content (% by mass) | | 30.3 | 44.0 | 45.5 | 44.0 |
| A/B | | 5.56 | 0.07 | 0.05 | 5.00 |
| A/C | | 4.63 | 0.16 | 0.34 | 42.86 |
| C/B | | 1.20 | 0.42 | 0.15 | 0.12 |
| A/(B + C) | | 2.525 | 0.047 | 0.044 | 4.478 |
| Water/D | | 1.00 | 1.00 | 1.00 | 1.00 |
| B/D | | 0.04 | 0.08 | 0.06 | 0.07 |
| B2/B1 | | 2 | 2 | 4 | 1 |
| Viscosity (mPa·s) 25°C | | 2.98 | 6.23 | 5.23 | 7.04 |
| Evaluation | Presence or absence of crystals in coating film | 3.0 | 5.0 | 4.0 | 5.0 |
| | Sticky feeling in coating film | 4.8 | 5.0 | 4.8 | 5.0 |
| | Durability of coating film | 5.0 | 5.0 | 5.0 | 5.0 |
| | Moisture resistance of coating film | 5.0 | 5.0 | 5.0 | 5.0 |

**Table 10**

| (% by mass) | | Examples | | | | | |
|---|---|---|---|---|---|---|---|
| | | 82 | 83 | 84 | 85 | 86 | 87 |
| | Panthenol | 1.00 | | | | | |
| | Pantothenyl ethyl ether | | 1.00 | | | | |
| | Tocopherol acetate | | | 2.00 | | | |
| | Allantoin | | | | 0.20 | | |
| | Lidocaine | | | | | 2.00 | |
| | Hinokitiol | | | | | | 0.10 |
| | Homosulfamine hydrochloride | | | | | | |
| | Methyl salicylate | | | | | | |
| | Indometacin | | | | | | |
| (A) | Diclofenac sodium | | | | | | |
| | Ketoprofen | | | | | | |
| | Flurbiprofen | | | | | | |
| | Dibucaine hydrochloride | | | | | | |
| | Fluocinolone acetonide | | | | | | |
| | Butenafine hydrochloride | | | | | | |
| | Tretinoin | | | | | | |
| | Hydrocortisone butyrate | | | | | | |
| | Resorcin | | | | | | |
| | Hydrocortisone | | | | | | |
| | Diphenhydramine | | | | | | |
| | Chlorpheniramine maleate | | | | | | |
| | Loxoprofen sodium hydrate | | | | | | |
| (B) | (B1) Hypromellose phthalate | 1.0 | 1.0 | 0.3 | 1.0 | 1.0 | 0.5 |
| | (B2) Acrylates/diacetoneacrylamide copolymer | 2.0 | 2.0 | 0.6 | 2.0 | 2.0 | 1.0 |
| (C) | Polysorbate 80 | 1.0 | 1.0 | 1.0 | 1.0 | 1.0 | 0.1 |
| | Diisopropanolamine | | | | | | |
| | 2-Amino-2-methylpropanol | 0.3 | 0.3 | 0.1 | 0.3 | 0.3 | 0.1 |
| | Triethanolamine | | | | | | |
| | 1,3-Butylene glycol | | | | | | |
| (D) | Ethanol | 57.0 | 57.0 | 70.0 | 57.0 | 57.0 | 57.0 |
| Others | Carboxyvinyl polymer | | | | | | |
| Water | Purified water | 37.7 | 37.7 | 26.0 | 38.5 | 36.7 | 41.2 |
| Total | | 100 | 100 | 100 | 100 | 100 | 100 |
| Component (A) content (% by mass) | | 1.00 | 1.00 | 2.00 | 0.20 | 2.00 | 0.10 |
| Component (B) content (% by mass) | | 3 | 3 | 0.9 | 3 | 3 | 1.5 |
| Component (B1) content (% by mass) | | 1 | 1 | 0.3 | 1 | 1 | 0.5 |
| Component (B2) content (% by mass) | | 2 | 2 | 0.6 | 2 | 2 | 1 |
| Component (C) content (% by mass) | | 1.3 | 1.3 | 1.1 | 1.3 | 1.3 | 0.2 |
| Component (D) content (% by mass) | | 37.7 | 37.7 | 26.0 | 38.5 | 36.7 | 41.2 |
| Components (A) to (D) total content (% by mass) | | 44.0 | 44.0 | 30.3 | 44.0 | 44.0 | 43.5 |
| A/B | | 0.33 | 0.33 | 2.22 | 0.07 | 0.67 | 0.07 |
| A/C | | 0.79 | 0.79 | 1.85 | 0.16 | 1.58 | 0.43 |
| C/B | | 0.42 | 0.42 | 1.20 | 0.42 | 0.42 | 0.16 |
| A/(B + C) | | 0.234 | 0.234 | 1.010 | 0.047 | 0.469 | 0.058 |
| Water/D | | 1.00 | 1.00 | 1.00 | 1.00 | 1.00 | 1.00 |
| B/D | | 0.08 | 0.08 | 0.03 | 0.08 | 0.08 | 0.04 |
| B2/B1 | | 2 | 2 | 2 | 2 | 2 | 2 |
| Viscosity (mPa·s) 25°C | | 7.34 | 7.23 | 3.12 | 6.98 | 7.35 | 7.78 |
| Evaluation | Presence or absence of crystals in coating film | 5.0 | 5.0 | 3.0 | 5.0 | 5.0 | 4.0 |
| | Sticky feeling in coating film | 5.0 | 5.0 | 5.0 | 5.0 | 5.0 | 5.0 |
| | Durability of coating film | 5.0 | 5.0 | 5.0 | 5.0 | 5.0 | 5.0 |
| | Moisture resistance of coating film | 5.0 | 5.0 | 5.0 | 5.0 | 5.0 | 5.0 |

**Table 10 (continued)**

| (% by mass) | | Examples | | | | | |
|---|---|---|---|---|---|---|---|
| | | 88 | 89 | 90 | 91 | 92 | 93 |
| | Panthenol | | | | | | |
| | Pantothenyl ethyl ether | | | | | | |
| | Tocopherol acetate | | | | | | |
| | Allantoin | | | | | | |
| | Lidocaine | | | | | | |
| | Hinokitiol | | | | | | |
| | Homosulfamine hydrochloride | 0.30 | | | | | |
| | Methyl salicylate | | 0.50 | | | | |
| | Indometacin | | | 1.00 | | | |
| | Diclofenac sodium | | | | 1.00 | | |
| | Ketoprofen | | | | | 3.00 | |
| | Flurbiprofen | | | | | | 3.00 |
| (A) | Dibucaine hydrochloride | | | | | | |
| | Fluocinolone acetonide | | | | | | |
| | Butenafine hydrochloride | | | | | | |
| | Tretinoin | | | | | | |
| | Hydrocortisone butyrate | | | | | | |
| | Resorcin | | | | | | |
| | Hydrocortisone | | | | | | |
| | Diphenhydramine | | | | | | |
| | Chlorpheniramine maleate | | | | | | |
| | Loxoprofen sodium hydrate | | | | | | |
| (B) | (B1) Hypromellose phthalate | 1.0 | 1.0 | 1.0 | 1.0 | 1.0 | 1.0 |
| | (B2) Acrylates/diacetoneacrylamide copolymer | 2.0 | 2.0 | 2.0 | 2.0 | 2.0 | 2.0 |
| (C) | Polysorbate 80 | 1.0 | 1.0 | 1.0 | 1.0 | 1.0 | 1.0 |
| | Diisopropanolamine | | | | | | |
| | 2-Amino-2-methylpropanol | 0.3 | 0.3 | 0.3 | 0.3 | 0.3 | 0.3 |
| | Triethanolamine | | | | | | |
| | 1,3-Butylene glycol | | | | | | |
| (D) | Ethanol | 57.0 | 57.0 | 57.0 | 57.0 | 70.0 | 70.0 |
| Others | Carboxyvinyl polymer | | | | | | |
| Water | Purified water | 38.4 | 38.2 | 37.7 | 37.7 | 22.7 | 22.7 |
| Total | | 100 | 100 | 100 | 100 | 100 | 100 |
| Component (A) content (% by mass) | | 0.30 | 0.50 | 1.00 | 1.00 | 3.00 | 3.00 |
| Component (B) content (% by mass) | | 3 | 3 | 3 | 3 | 3 | 3 |
| Component (B1) content (% by mass) | | 1 | 1 | 1 | 1 | 1 | 1 |
| Component (B2) content (% by mass) | | 2 | 2 | 2 | 2 | 2 | 2 |
| Component (C) content (% by mass) | | 1.3 | 1.3 | 1.3 | 1.3 | 1.3 | 1.3 |
| Component (D) content (% by mass) | | 38.4 | 38.2 | 37.7 | 37.7 | 22.7 | 22.7 |
| Components (A) to (D) total content (% by mass) | | 44.0 | 44.0 | 44.0 | 44.0 | 31.0 | 31.0 |
| A/B | | 0.10 | 0.17 | 0.33 | 0.33 | 1.00 | 1.00 |
| A/C | | 0.24 | 0.39 | 0.79 | 0.79 | 2.37 | 2.37 |
| C/B | | 0.42 | 0.42 | 0.42 | 0.42 | 0.42 | 0.42 |
| A/(B + C) | | 0.070 | 0.117 | 0.234 | 0.234 | 0.703 | 0.703 |
| Water/D | | 1.00 | 1.00 | 1.00 | 1.00 | 1.00 | 1.00 |
| B/D | | 0.08 | 0.08 | 0.08 | 0.08 | 0.13 | 0.13 |
| B2/B1 | | 2 | 2 | 2 | 2 | 2 | 2 |
| Viscosity (mPa·s) 25°C | | 6.36 | 7.22 | 6.77 | 6.44 | 6.54 | 6.98 |
| Evaluation | Presence or absence of crystals in coating film | 5.0 | 5.0 | 5.0 | 5.0 | 5.0 | 5.0 |
| | Sticky feeling in coating film | 5.0 | 5.0 | 5.0 | 5.0 | 5.0 | 5.0 |
| | Durability of coating film | 5.0 | 5.0 | 5.0 | 5.0 | 5.0 | 5.0 |
| | Moisture resistance of coating film | 5.0 | 5.0 | 5.0 | 5.0 | 5.0 | 5.0 |

**Table 11**

| (% by mass) | | Examples | | | | |
|---|---|---|---|---|---|---|
| | | 94 | 95 | 96 | 97 | 98 |
| | Dibucaine hydrochloride | 0.10 | | | | |
| | Fluocinolone acetonide | | 0.25 | | | |
| | Butenafine hydrochloride | | | 1.00 | | |
| | Tretinoin | | | | 0.40 | |
| (A) | Hydrocortisone butyrate | | | | | 0.05 |
| | Resorcin | | | | | |
| | Hydrocortisone | | | | | |
| | Diphenhydramine | | | | | |
| | Chlorpheniramine maleate | | | | | 0.5 |
| | Loxoprofen sodium hydrate | | | | | |
| (B) | (B1) Hypromellose phthalate | 0.5 | 1.0 | 0.5 | 0.3 | 1.0 |
| | (B2) Acrylates/diacetoneacrylamide copolymer | 2.0 | 2.0 | 2.0 | 0.6 | 2.0 |
| (C) | Polysorbate 80 | 0.1 | 1.0 | 1.0 | 1.0 | |
| | Diisopropanolamine | | | | | |
| | 2-Amino-2-methylpropanol | 0.3 | 0.3 | 0.3 | 0.1 | 0.3 |
| | Triethanolamine | | | | | |
| | 1,3-Butylene glycol | | | 5.0 | | 0.5 |
| (D) | Ethanol | 57.0 | 57.0 | 70.0 | 70.0 | 55.0 |
| Others | Carboxyvinyl polymer | | | | | |
| Water | Purified water | 40.0 | 38.5 | 20.2 | 27.6 | 40.7 |
| Total | | 100 | 100 | 100 | 100 | 100 |
| Component (A) content (% by mass) | | 0.10 | 0.25 | 1.00 | 0.40 | 0.55 |
| Component (B) content (% by mass) | | 2.5 | 3 | 2.5 | 0.9 | 3 |
| Component (B1) content (% by mass) | | 0.5 | 1 | 0.5 | 0.3 | 1 |
| Component (B2) content (% by mass) | | 2 | 2 | 2 | 0.6 | 2 |
| Component (C) content (% by mass) | | 0.4 | 1.3 | 6.3 | 1.1 | 0.8 |
| Component (D) content (% by mass) | | 40.0 | 38.5 | 20.2 | 27.6 | 40.7 |
| Components (A) to (D) total content (% by mass) | | 43.5 | 44.0 | 30.5 | 30.3 | 46.0 |
| A/B | | 0.04 | 0.08 | 0.40 | 0.44 | 0.18 |
| A/C | | 0.27 | 0.20 | 0.16 | 0.37 | 0.72 |
| C/B | | 0.15 | 0.42 | 2.51 | 1.20 | 0.26 |
| A/(B + C) | | 0.035 | 0.059 | 0.114 | 0.202 | 0.146 |
| Water/D | | 1.00 | 1.00 | 1.00 | 1.00 | 1.00 |
| B/D | | 0.06 | 0.08 | 0.12 | 0.03 | 0.07 |
| B2/B1 | | 4 | 2 | 4 | 2 | 2 |
| Viscosity (mPa·s) 25°C | | 8.23 | 6.43 | 5.12 | 2.87 | 4.23 |
| Evaluation | Presence or absence of crystals in coating film | 4.0 | 5.0 | 4.0 | 3.0 | 4.0 |
| | Sticky feeling in coating film | 5.0 | 5.0 | 4.8 | 4.7 | 5.0 |
| | Durability of coating film | 5.0 | 5.0 | 5.0 | 5.0 | 5.0 |
| | Moisture resistance of coating film | 5.0 | 5.0 | 5.0 | 5.0 | 5.0 |

**Table 11 (continued)**

| (% by mass) | | Examples | | | |
|---|---|---|---|---|---|
| | | 99 | 100 | 101 | 102 |
| | Dibucaine hydrochloride | | | | |
| | Fluocinolone acetonide | | | | |
| | Butenafine hydrochloride | | | | |
| | Tretinoin | | | | |
| (A) | Hydrocortisone butyrate | | | | |
| | Resorcin | 2.00 | | | |
| | Hydrocortisone | | 1.00 | | |
| | Diphenhydramine | | | 2.00 | |
| | Chlorpheniramine maleate | | | | |
| | Loxoprofen sodium hydrate | | | | 1.13 |
| (B) | (B1) Hypromellose phthalate | 1.0 | 1.0 | 1.0 | 1.0 |
| | (B2) Acrylates/diacetoneacrylamide copolymer | 2.0 | 2.0 | 2.0 | 2.0 |
| (C) | Polysorbate 80 | 1.0 | 1.0 | 1.0 | 1.0 |
| | Diisopropanolamine | | | | |
| | 2-Amino-2-methylpropanol | 0.3 | 0.3 | 0.3 | 0.3 |
| | Triethanolamine | | | | |
| | 1,3-Butylene glycol | | | | |
| (D) | Ethanol | 57.0 | 57.0 | 57.0 | 57.0 |
| Others | Carboxyvinyl polymer | | | | |
| Water | Purified water | 36.7 | 37.7 | 36.7 | 37.6 |
| Total | | 100 | 100 | 100 | 100 |
| Component (A) content (% by mass) | | 2.00 | 1.00 | 2.00 | 1.13 |
| Component (B) content (% by mass) | | 3 | 3 | 3 | 3 |
| Component (B1) content (% by mass) | | 1 | 1 | 1 | 1 |
| Component (B2) content (% by mass) | | 2 | 2 | 2 | 2 |
| Component (C) content (% by mass) | | 1.3 | 1.3 | 1.3 | 1.3 |
| Component (D) content (% by mass) | | 36.7 | 37.7 | 36.7 | 37.6 |
| Components (A) to (D) total content (% by mass) | | 44.0 | 44.0 | 44.0 | 44.0 |
| A/B | | 0.67 | 0.33 | 0.67 | 0.38 |
| A/C | | 1.58 | 0.79 | 1.58 | 0.89 |
| C/B | | 0.42 | 0.42 | 0.42 | 0.42 |
| A/(B + C) | | 0.469 | 0.234 | 0.469 | 0.265 |
| Water/D | | 1.00 | 1.00 | 1.00 | 1.00 |
| B/D | | 0.08 | 0.08 | 0.08 | 0.08 |
| B2/B1 | | 2 | 2 | 2 | 2 |
| Viscosity (mPa·s) 25°C | | 7.34 | 7.77 | 6.89 | 6.32 |
| Evaluation | Presence or absence of crystals in coating film | 5.0 | 5.0 | 5.0 | 5.0 |
| | Sticky feeling in coating film | 5.0 | 5.0 | 5.0 | 5.0 |
| | Durability of coating film | 5.0 | 5.0 | 5.0 | 5.0 |
| | Moisture resistance of coating film | 5.0 | 5.0 | 5.0 | 5.0 |

From Tables 2 to 11, it can be seen that the compositions for external preparation of the present Examples have good durability and moisture resistance of the coating film, and furthermore, the effect of suppressing crystallization of the component (A) and the effect of suppressing stickiness of the coating film are high.

In contrast, Comparative Example 1, which did not contain the component (B), Comparative Examples 2 to 6, which did not contain two or more selected from the group consisting of the components (B1) to (B3) as the component (B), Comparative Example 7, which did not contain the component (C), and Comparative Example 8, which did not contain the component (D), were inferior in any of the items to the effects of the present invention.

In addition, the thicknesses of the coating films formed in Examples 1, 2, 4, 7, 12, and 15 in which a combination of (B1) a cellulosic polymer and (B2) an acrylic polymer was used, Example 19 in which a combination of (B1) a cellulosic polymer and (B3) a vinyl polymer was used, and Example 27 in which a combination of (B2) an acrylic polymer and (B3) a vinyl polymer was used were measured by the following method. The thicknesses of the coating films in Examples 1, 2, 4, 7, 9, 12, 13, 15, 19, and 27 were 0.028 mm, 0.019 mm, 0.008 mm, 0.028 mm, 0.051 mm, 0.029 mm, 0.070 mm, 0.098 mm, 0.031 mm, and 0.020 mm, respectively.

### (Thickness of coating film)

The composition for external preparation was applied to a vinyl tape ("lead-free type J3447" manufactured by Nitoms, Inc.) at 20 µL/cm², and dried on a hotplate warmed to 32°C for 10 minutes. The coating film obtained after drying was peeled off with tweezers, and the thickness of the coating film was measured using a digimatic indicator ("ID-C112XBS" manufactured by Mitutoyo Corporation, minimum display: 0.001 mm).

Furthermore, with respect to Examples 1 to 3 and Comparative Examples 5 and 6, the removability of the coating film formed using the composition for external preparation was evaluated by the following method. The results are shown in Table 12.

### (Removability of coating film)

An expert panelist applied 50 µL of the composition for external preparation to an area of 2 cm × 2 cm on the inner side of the forearm using MICROMAN (manufactured by GILSON), and the composition was naturally dried for 10 minutes to obtain a coating film. Thereafter, the forearm was immersed in a water bath at about 40°C, and the remaining state of the coating film in a case where the coating film was rubbed with a finger 10 times was visually observed. Next, a foamed cleaning agent (Kao Soap White) was brought into contact with the coating film, and then the coating film was rubbed with a finger 10 times, the foam was flushed under a water bath, and the remaining state of the coating film was visually observed. The removability of the coating film was evaluated according to the following evaluation criteria depending on the stage of removal in consideration of the moisture resistance of the coating film. Pass was rated A.
A: The coating film is not removed with tepid water at 40°C, but can be removed when a cleaning agent is used.
B: The coating film is removed with tepid water at 40°C.
C: The coating film cannot be removed even with tepid water at 40°C or by using a cleaning agent.

**Table 12**

| (% by mass) | | Examples | | | Comparative Example | |
|---|---|---|---|---|---|---|
| | | 1 | 2 | 3 | 5 | 6 |
| (A) | Loxoprofen sodium hydrate | 1.13 | 1.13 | 1.13 | 1.13 | 1.13 |
| | 1-Menthol | 3 | 3 | 3 | 3 | 3 |
| (B) | (B1) Hypromellose phthalate | 1 | | 0.01 | | |
| | (B1) Ethyl cellulose | | 0.2 | | | |
| | (B2) Acrylates/diacetoneacrylamide copolymer | 2 | 2 | 0.01 | | |
| | (B2) Alkyl acrylate-vinyl acetate copolymer | | | | 0.225 | 1 |
| | (B2) Alkyl acrylate copolymer | | | | 1.8 | 2 |
| | (B3) Polyvinyl butyral | | | | | |
| | Isopropyl myristate | 1 | 1 | 1 | 1 | |
| | Dipropylene glycol | | | | | 1 |
| | Polyethylene glycol 400 | | | | | |
| | Polysorbate 80 | | | | | |
| | Squalane | | | | | |
| (C) | Diisopropanolamine | | | | | |
| | Triethanolamine | | | | | |
| | 2-Amino-2-methylpropanol | 0.3 | 0.3 | 0.001 | | |
| | 1,3-Butylene glycol | | | | | |
| | Propylene glycol monocaprate | | | | | |
| | Polyethylene glycol 4000 | | | | | |
| Others | Polyvinylpyrrolidone | | | | | |
| | Lactic acid | 0.18 | | | 0.18 | 0.18 |
| (D) | Ethanol | 57 | 57 | 57 | 89.67 | 87.45 |
| | Isopropanol | | | | | |
| Water | Purified water | 34.42 | 35.40 | 37.85 | 2.995 | 4.242 |
| Total | | 100 | 100 | 100 | 100 | 100 |
| Evaluation | Moisture resistance of coating film | 5.0 | 5.0 | 3.0 | 2.0 | 2.0 |
| | Removability of coating film | A | A | A | B | B |

### Examples 103 to 106 (Preparation and Evaluation of Compositions for External Preparation)

The component (B) and other components described in the table were mixed with the component (C), the component (D), and an appropriate amount of water, and then the component (A) was mixed therewith. Thereafter, the remaining water was mixed to prepare a composition for external preparation.

Using the obtained composition for external preparation, evaluation was performed by the method described above. Furthermore, the contact angle and the rate of change thereof, and the application property of the composition were evaluated by the following methods. The main components used are shown in Table 1, and the compositions and evaluation results of the compositions for external preparation are shown in Table 13. The blending amount described in the table is the active ingredient amount (% by mass) of each component.

### (Contact angle of composition and rate of change thereof)

At a temperature of 25°C, 2 µL of the composition for external preparation described in Table 13 was dropped onto artificial leather SUPPLALE (manufactured by IDEATEX JAPAN Co., Ltd.) using an automatic contact angle meter (DM-501i manufactured by Kyowa Chemical Industry Co., Ltd.), and the contact angle after 10 seconds after droplet deposition and the contact angle after 60 seconds after droplet deposition were measured. In addition, the rate of change in the contact angle in 10 seconds to 60 seconds after droplet deposition was calculated from the following formula (1). Rate of change in contact angle = 1 - (contact angle in 60 seconds after droplet deposition)/(contact angle in 10 seconds after droplet deposition)

### (Application property)

A mark was made on the inner side of the forearm of an expert panelist so that the area of 2 cm × 2 cm could be known, and then 24 µL of the composition for external preparation shown in Table 13 was applied using MICROMAN (manufactured by GILSON). The spread of the composition 30 seconds after the application was visually confirmed, and the application property was determined according to the following criteria.
A: The composition could be applied so as to fall within the range of 2 cm × 2 cm.
B: Bleeding occurred around the range of 2 cm × 2 cm.
C: Dripping occurred from the range of 2 cm × 2 cm.

**Table 13**

| (% by mass) | | Examples | | | | |
|---|---|---|---|---|---|---|
| | | 1 | 2 | 7 | 9 | 12 |
| (A) | Loxoprofen sodium hydrate | 1.13 | 1.13 | 1.13 | 1.13 | 1.13 |
| | 1-Menthol | 3 | 3 | 3 | 3 | 3 |
| (B) | (B1) Hypromellose phthalate | 1 | | 2 | 2 | 10 |
| | (B1) Ethyl cellulose | | 0.2 | | | |
| | (B2) Acrylates/diacetoneacrylamide copolymer | 2 | 2 | 4 | 8 | 2 |
| | (B3) Polyvinyl butyral | | | | | |
| | Isopropyl myristate | 1 | 1 | 1 | 1 | 1 |
| | Dipropylene glycol | | | | | |
| | Polyethylene glycol 400 | | | | | |
| | Polysorbate 80 | | | | | |
| (C) | Squalane | | | | | |
| | Diisopropanolamine | | | | | |
| | Triethanolamine | | | | | |
| | 2-Amino-2-methylpropanol | 0.3 | 0.3 | 0.5 | 1.1 | 0.3 |
| | 1,3-Butylene glycol | | | | | |
| | Propylene glycol monocaprate | | | | | |
| | Polyethylene glycol 4000 | | | | | |
| Others | Polyvinylpyrrolidone | | | | | |
| | Lactic acid | 0.18 | | | | |
| (D) | Ethanol | 57 | 57 | 57 | 57 | 57 |
| | Isopropanol | | | | | |
| Water | Purified water | 34.42 | 35.40 | 31.34 | 26.80 | 25.60 |
| Total | | 100 | 100 | 100 | 100 | 100 |
| Component (A) content (% by mass) | | 4.13 | 4.13 | 4.13 | 4.13 | 4.13 |
| Component (B) content (% by mass) | | 3 | 2.2 | 6 | 10 | 12 |
| Component (B1) content (% by mass) | | 1 | 0.2 | 2 | 2 | 10 |
| Component (B2) content (% by mass) | | 2 | 2 | 4 | 8 | 2 |
| Component (B3) content (% by mass) | | 0 | 0 | 0 | 0 | 0 |
| Component (C) content (% by mass) | | 1.3 | 1.3 | 1.5 | 2.1 | 1.3 |
| Component (D) content (% by mass) | | 57 | 57 | 57 | 57 | 57 |
| Components (A) to (D) total content (% by mass) | | 65.40 | 64.60 | 68.66 | 73.20 | 74.40 |
| A/B | | 1.38 | 1.88 | 0.69 | 0.41 | 0.34 |
| A/C | | 3.26 | 3.26 | 2.69 | 2.00 | 3.26 |
| C/B | | 0.42 | 0.58 | 0.26 | 0.21 | 0.11 |
| A/(B + C) | | 0.97 | 1.19 | 0.55 | 0.34 | 0.31 |
| Water/D | | 0.60 | 0.62 | 0.55 | 0.47 | 0.45 |
| B/D | | 0.05 | 0.04 | 0.11 | 0.18 | 0.21 |
| Acrylic/cellulosic B2/B1 | | 2 | 10 | 2 | 4 | 0.2 |
| Vinyl/cellulosic B3/B1 | | 0 | 0 | 0 | 0 | 0 |
| Acrylic/vinyl B2/B3 | | - | - | - | - | - |
| Viscosity (mPa·s) 25°C | | 6.08 | 5.51 | 11.2 | 24.0 | 121 |
| Evaluation | Contact angle (°) in 10 seconds after droplet deposition | 28.7 | 36.3 | 46.5 | 45.8 | 53.0 |
| | Contact angle (°) in 60 seconds after droplet deposition | 19.8 | 30.1 | 33.4 | 35.4 | 40.8 |
| | Rate of change in contact angle in 10 seconds to 60 seconds after droplet deposition | 0.31 | 0.17 | 0.28 | 0.23 | 0.23 |
| | Application property | A | A | A | A | A |
| | Presence or absence of crystals in coating film | 5.0 | 5.0 | 4.0 | 5.0 | 5.0 |
| | Sticky feeling in coating film | 5.0 | 4.2 | 5.0 | 5.0 | 5.0 |
| | Durability of coating film | 5.0 | 4.0 | 5.0 | 5.0 | 3.0 |
| | Moisture resistance of coating film | 5.0 | 5.0 | 5.0 | 5.0 | 3.0 |

**Table 13 (continued)**

| (% by mass) | | Examples | | | | |
|---|---|---|---|---|---|---|
| | | 13 | 15 | 22 | 28 | 29 |
| (A) | Loxoprofen sodium hydrate | 1.13 | 1.13 | 1.13 | 1.13 | 1.13 |
| | 1-Menthol | 3 | 3 | 3 | 3 | 3 |
| (B) | (B1) Hypromellose phthalate | 1 | 1 | 1 | | 2 |
| | (B1) Ethyl cellulose | | | | | |
| | (B2) Acrylates/diacetoneacrylamide copolymer | 12 | 20 | | 1 | 10 |
| | (B3) Polyvinyl butyral | | | 10 | 8 | |
| | Isopropyl myristate | 1 | 1 | 1 | 1 | |
| | Dipropylene glycol | | | | | |
| | Polyethylene glycol 400 | | | | | |
| | Polysorbate 80 | | | | | |
| | Squalane | | | | | |
| (C) | Diisopropanolamine | | | | | |
| | Triethanolamine | | | | | |
| | 2-Amino-2-methylpropanol | 1.6 | 2.7 | | 0.1 | 1.3 |
| | 1,3-Butylene glycol | | | | | |
| | Propylene glycol monocaprate | | | | | 30 |
| | Polyethylene glycol 4000 | | | | | |
| Others | Polyvinylpyrrolidone | | | | | |
| | Lactic acid | | | | | |
| (D) | Ethanol | 57 | 57 | 63 | 61.75 | 42.75 |
| | Isopropanol | | | | | |
| Water | Purified water | 23.27 | 14.20 | 20.87 | 23.99 | 9.79 |
| Total | | 100 | 100 | 100 | 100 | 100 |
| Component (A) content (% by mass) | | 4.13 | 4.13 | 4.13 | 4.13 | 4.13 |
| Component (B) content (% by mass) | | 13 | 21 | 11 | 9 | 12 |
| Component (B1) content (% by mass) | | 1 | 1 | 1 | 0 | 2 |
| Component (B2) content (% by mass) | | 12 | 20 | 0 | 1 | 10 |
| Component (B3) content (% by mass) | | 0 | 0 | 10 | 8 | 0 |
| Component (C) content (% by mass) | | 2.6 | 3.7 | 1.0 | 1.1 | 31.3 |
| Component (D) content (% by mass) | | 57 | 57 | 63 | 61.75 | 42.75 |
| Components (A) to (D) total content (% by mass) | | 76.73 | 85.80 | 79.13 | 76.01 | 90.21 |
| A/B | | 0.32 | 0.20 | 0.38 | 0.46 | 0.34 |
| A/C | | 1.59 | 1.13 | 4.13 | 3.64 | 0.13 |
| C/B | | 0.20 | 0.17 | 0.09 | 0.13 | 2.61 |
| A/(B + C) | | 0.26 | 0.17 | 0.34 | 0.41 | 0.10 |
| Water/D | | 0.41 | 0.25 | 0.33 | 0.39 | 0.23 |
| B/D | | 0.23 | 0.37 | 0.17 | 0.15 | 0.28 |
| Acrylic/cellulosic B2/B1 | | 12 | 20 | 0 | - | 5 |
| Vinyl/cellulosic B3/B1 | | 0 | 0 | 10 | - | 0 |
| Acrylic/vinyl B2/B3 | | - | - | 0 | 0.125 | - |
| Viscosity (mPa·s) 25°C | | 82.5 | 135 | 234 | 102 | 88.2 |
| Evaluation | Contact angle (°) in 10 seconds after droplet deposition | 43.4 | 59.1 | 73.0 | 46.9 | 40.8 |
| | Contact angle (°) in 60 seconds after droplet deposition | 32.0 | 51.0 | 66.7 | 35.7 | 32.6 |
| | Rate of change in contact angle in 10 seconds to 60 seconds after droplet deposition | 0.26 | 0.14 | 0.09 | 0.24 | 0.20 |
| | Application property | A | A | A | A | A |
| | Presence or absence of crystals in coating film | 5.0 | 5.0 | 5.0 | 4.0 | 5.0 |
| | Sticky feeling in coating film | 3.8 | 3.0 | 5.0 | 5.0 | 3.0 |
| | Durability of coating film | 4.0 | 3.0 | 3.0 | 3.0 | 4.0 |
| | Moisture resistance of coating film | 5.0 | 5.0 | 3.0 | 3.0 | 4.0 |

**Table 13 (continued)**

| (% by mass) | | Examples | | | |
|---|---|---|---|---|---|
| | | 103 | 104 | 105 | 106 |
| (A) | Loxoprofen sodium hydrate | 1.13 | 1.13 | 1.13 | 1.13 |
| | 1-Menthol | 3 | 3 | 3 | 3 |
| (B) | (B1) Hypromellose phthalate | | | | |
| | (B1) Ethyl cellulose | 0.2 | 0.2 | 0.2 | 0.2 |
| | (B2) Acrylates/diacetoneacrylamide copolymer | 2 | 2 | 2 | 2 |
| | (B3) Polyvinyl butyral | | | | |
| | Isopropyl myristate | 1 | 1 | 1 | 1 |
| | Dipropylene glycol | | | | |
| | Polyethylene glycol 400 | | | | |
| | Polysorbate 80 | | | | |
| | Squalane | | | | |
| (C) | Diisopropanolamine | | | | |
| | Triethanolamine | | | | |
| | 2-Amino-2-methylpropanol | 0.3 | 0.3 | 0.3 | 0.3 |
| | 1,3-Butylene glycol | | | | |
| | Propylene glycol monocaprate | | | | |
| | Polyethylene glycol 4000 | | | | |
| Others | Polyvinylpyrrolidone | | | | |
| | Lactic acid | | | | |
| (D) | Ethanol | 92.4 | 91.4 | 89.4 | 82.4 |
| | Isopropanol | | | | |
| Water | Purified water | 0 | 1 | 3 | 10 |
| Total | | 100 | 100 | 100 | 100 |
| Component (A) content (% by mass) | | 4.13 | 4.13 | 4.13 | 4.13 |
| Component (B) content (% by mass) | | 2.2 | 2.2 | 2.2 | 2.2 |
| Component (B1) content (% by mass) | | 0.2 | 0.2 | 0.2 | 0.2 |
| Component (B2) content (% by mass) | | 2 | 2 | 2 | 2 |
| Component (B3) content (% by mass) | | 0 | 0 | 0 | 0 |
| Component (C) content (% by mass) | | 1.3 | 1.3 | 1.3 | 1.3 |
| Component (D) content (% by mass) | | 92.4 | 91.4 | 89.4 | 82.4 |
| Components (A) to (D) total content (% by mass) | | 100.00 | 99.00 | 97.00 | 90.00 |
| A/B | | 1.88 | 1.88 | 1.88 | 1.88 |
| A/C | | 3.26 | 3.26 | 3.26 | 3.26 |
| C/B | | 0.58 | 0.58 | 0.58 | 0.58 |
| A/(B + C) | | 1.19 | 1.19 | 1.19 | 1.19 |
| Water/D | | 0.00 | 0.01 | 0.03 | 0.12 |
| B/D | | 0.02 | 0.02 | 0.02 | 0.03 |
| Acrylic/cellulosic B2/B1 | | 10 | 10 | 10 | 10 |
| Vinyl/cellulosic B3/B1 | | 0 | 0 | 0 | 0 |
| Acrylic/vinyl B2/B3 | | - | - | - | - |
| Viscosity (mPa·s) 25°C | | 2.71 | 2.98 | 3.05 | 4.26 |
| Evaluation | Contact angle (°) in 10 seconds after droplet deposition | 18.6 | 20.1 | 23.9 | 30.3 |
| | Contact angle (°) in 60 seconds after droplet deposition | 9.1 | 14.5 | 17.0 | 24.9 |
| | Rate of change in contact angle in 10 seconds to 60 seconds after droplet deposition | 0.51 | 0.28 | 0.29 | 0.18 |
| | Application property | C | B | B | A |
| | Presence or absence of crystals in coating film | 4.0 | 5.0 | 5.0 | 5.0 |
| | Sticky feeling in coating film | 4.5 | 4.5 | 4.5 | 4.5 |
| | Durability of coating film | 4.0 | 5.0 | 5.0 | 5.0 |
| | Moisture resistance of coating film | 3.5 | 3.5 | 3.5 | 3.5 |

From Table 13, it can be seen that a composition for external preparation having a high contact angle with respect to artificial leather at 10 seconds and 60 seconds after droplet deposition and a small change rate of the contact angle at 10 seconds to 60 seconds after droplet deposition has better application property to the skin.

### Prescription Examples 1 to 26 (Preparation of Composition for External Preparation)

According to the prescriptions shown in Tables 14 and 15, the component (B) and other components were mixed with the component (C), the component (D), and an appropriate amount of water, and then the component (A) was mixed therewith. Thereafter, the remaining water was mixed to prepare a composition for external preparation.

### Prescription Examples 27 to 30 (Preparation of Aerosol Formulation)

According to the prescriptions shown in Table 16, the component (B) and other components were mixed with the component (C), the component (D), and an appropriate amount of water, and then the component (A) was mixed therewith. Thereafter, the remaining water was mixed to prepare a composition for external preparation serving as an aerosol stock solution. This was filled in an aerosol container (manufactured by Toyo Seikan Co., Ltd.), and then dimethyl ether (DME) was sealed therein so that the mass ratio of the aerosol stock solution to DME as a propellant was as shown in the table, thereby preparing an aerosol formulation.

**Table 14**

| (% by mass) | Prescription Example | | | | |
|---|---|---|---|---|---|
| | 1 | 2 | 3 | 4 | 5 |
| Loxoprofen sodium hydrate | | | | | |
| Felbinac | | | | | |
| Glycyrrhetinic acid | | | | | |
| Isopropylmethylphenol | | | | | |
| Crotamiton | | | | | |
| Butenafine hydrochloride | | | | | |
| Dibucaine hydrochloride | | | | | |
| Chlorpheniramine maleate | | | | | |
| l-Menthol | | | | | |
| Aluminum chloride | | | | | |
| Glycopyrronium tosilate hydrate | | | | 2.5 | |
| Sofpironium bromide | | | | | 5 |
| Miconazole nitrate | 1 | | | | |
| Zinc oxide | | 2 | | | |
| Capsicum tincture | | | 0.35 | | |
| Hypromellose phthalate | 1 | 1 | 1 | 1 | 1 |
| Acrylates/diacetoneacrylamide copolymer | 2 | 2 | 2 | 2 | 2 |
| Acrylic resin alkanolamine solution | | | | | |
| Isopropyl myristate | | | | 1 | 1 |
| Polysorbate 80 | 1 | 1 | 1 | | |
| 2-Amino-2-methylpropanol | 0.3 | 0.3 | 0.3 | 0.3 | 0.3 |
| 1,3-Butylene glycol | 1 | 1 | | | |
| Diisopropanolamine | | | | | |
| Ethanol | 50 | 50 | 50 | 60 | 60 |
| Antiseptic | q.s. | q.s. | q.s. | q.s. | q.s. |
| pH adjuster | q.s. | q.s. | q.s. | q.s. | q.s. |
| Perfume | q.s. | q.s. | q.s. | q.s. | q.s. |
| Purified water | balance | balance | balance | balance | balance |
| Total | 100 | 100 | 100 | 100 | 100 |

**Table 14 (continued)**

| (% by mass) | Prescription Example | | | |
|---|---|---|---|---|
| | 6 | 7 | 8 | 9 |
| Loxoprofen sodium hydrate | | | 1.13 | |
| Felbinac | | | | 3 |
| Glycyrrhetinic acid | 0.2 | | | |
| Isopropylmethylphenol | | 0.1 | | |
| Crotamiton | 1 | | | |
| Butenafine hydrochloride | 1 | | | |
| Dibucaine hydrochloride | 0.2 | | | |
| Chlorpheniramine maleate | 0.5 | | | |
| 1-Menthol | 2 | | 3 | 3 |
| Aluminum chloride | | 4 | | |
| Glycopyrronium tosilate hydrate | | | | |
| Sofpironium bromide | | | | |
| Miconazole nitrate | | | | |
| Zinc oxide | | | | |
| Capsicum tincture | | | | |
| Hypromellose phthalate | 1 | 1 | 1 | 3 |
| Acrylates/diacetoneacrylamide copolymer | 2 | 2 | | |
| Acrylic resin alkanolamine solution | | | 2 | 0.2 |
| Isopropyl myristate | 1 | | 1 | 1 |
| Polysorbate 80 | | 1 | | |
| 2-Amino-2-methylpropanol | 0.3 | 0.3 | | |
| 1,3-Butylene glycol | | 1 | | |
| Diisopropanolamine | | | | 1 |
| Ethanol | 60 | 60 | 57 | 57 |
| Antiseptic | q.s. | q.s. | q.s. | q.s. |
| pH adjuster | q.s. | q.s. | q.s. | q.s. |
| Perfume | q.s. | q.s. | q.s. | q.s. |
| Purified water | balance | balance | balance | balance |
| Total | 100 | 100 | 100 | 100 |

**Table 15**

| (% by mass) | Prescription Example | | | | | |
|---|---|---|---|---|---|---|
| | 10 | 11 | 12 | 13 | 14 | 15 |
| Prednisolone valerate acetate | | | | | | |
| Diphenhydramine hydrochloride | | | | | | |
| Dexamethasone acetate | | | | | | |
| Glycyrrhetinic acid | | | | | | |
| Isopropylmethylphenol | | | | | | |
| Crotamiton | | | | | | |
| Benzethonium chloride | | | | | | |
| Allantoin | | | | | | |
| Salicylic acid | | | | | | |
| 1-Menthol | | | | | | |
| dl-Camphor | | | | | | |
| Urea | 20 | | | | | |
| Sulfur | | 8 | | | | |
| Carpronium chloride hydrate | | | 2.18 | | | |
| Chlorhexidine hydrochloride | | | | 0.2 | | |
| Homosulfamine | | | | | 0.3 | |
| Acyclovir | | | | | | 5 |
| Vidarabine | | | | | | |
| Trichlorocarbanilide | | | | | | |
| Bifonazole | | | | | | |
| Lidocaine | | | | | | |
| Oxiconazole nitrate | | | | | | |
| Lanoconazole | | | | | | |
| Chlorpheniramine maleate | | | | | | |
| Hydrocortisone acetate | | | | | | |
| Adapalene | | | | | | |
| Tacrolimus | | | | | | |
| Hypromellose phthalate | 0.3 | 0.3 | 1 | 1 | 0.3 | 1 |
| Acrylates/diacetoneacrylamide copolymer | 2 | 2 | 2 | 2 | 2 | 2 |
| Acrylic resin alkanolamine solution | | | | | | |
| Isopropyl myristate | | | | | | |
| Polysorbate 80 | 1 | 1 | 1 | 1 | 1 | 1 |
| 2-Amino-2-methylpropanol | 0.3 | 0.3 | 0.3 | 0.3 | 0.3 | 0.3 |
| 1,3-Butylene glycol | 3 | 3 | | | | |
| Triethanolamine | | | | | 0.6 | |
| Carboxyvinyl polymer | | | | | 0.5 | |
| Ethanol | 40 | 40 | 57 | 50 | 50 | 50 |
| Antiseptic | q.s. | q.s. | q.s. | q.s. | q.s. | q.s. |
| pH adjuster | q.s. | q.s. | q.s. | q.s. | q.s. | q.s. |
| Perfume | q.s. | q.s. | q.s. | q.s. | q.s. | q.s. |
| Purified water | balance | balance | balance | balance | balance | balance |
| Total | 100 | 100 | 100 | 100 | 100 | 100 |

**Table 15 (continued)**

| (% by mass) | Prescription Example | | | | | |
|---|---|---|---|---|---|---|
| | 16 | 17 | 18 | 19 | 20 | 21 |
| Prednisolone valerate acetate | | | 0.015 | | | |
| Diphenhydramine hydrochloride | | 1 | | | | |
| Dexamethasone acetate | | 0.025 | | | | |
| Glycyrrhetinic acid | | | 1 | | | |
| Isopropylmethylphenol | | 0.1 | 0.015 | | | |
| Crotamiton | | | 5 | | | |
| Benzethonium chloride | | | | 0.05 | | |
| Allantoin | | | | 0.2 | | |
| Salicylic acid | | | | 0.5 | | |
| 1-Menthol | | 3 | 3.5 | | | |
| dl-Camphor | | 2 | | | | |
| Urea | | | | | | |
| Sulfur | | | | | | |
| Carpronium chloride hydrate | | | | | | |
| Chlorhexidine hydrochloride | | | | | | |
| Homosulfamine | | | | | | |
| Acyclovir | | | | | | |
| Vidarabine | 3 | | | | | |
| Trichlorocarbanilide | | | | | 0.1 | |
| Bifonazole | | | | | | 1 |
| Lidocaine | | | | | | |
| Oxiconazole nitrate | | | | | | |
| Lanoconazole | | | | | | |
| Chlorpheniramine maleate | | | | | | |
| Hydrocortisone acetate | | | | | | |
| Adapalene | | | | | | |
| Tacrolimus | | | | | | |
| Hypromellose phthalate | 1 | 1 | 1 | 1 | 0.3 | 1 |
| Acrylates/diacetoneacrylamide copolymer | 2 | 2 | 2 | 2 | 2 | 2 |
| Acrylic resin alkanolamine solution | | | | | | |
| Isopropyl myristate | | | 1 | | 0.5 | |
| Polysorbate 80 | 1 | 1 | | 1 | | 1 |
| 2-Amino-2-methylpropanol | 0.3 | 0.3 | 0.3 | 0.3 | 0.3 | 0.3 |
| 1,3-Butylene glycol | | 1 | 1 | | | 1 |
| Triethanolamine | | | | | 0.6 | |
| Carboxyvinyl polymer | | | | | 0.5 | |
| Ethanol | 50 | 50 | 50 | 50 | 50 | 50 |
| Antiseptic | q.s. | q.s. | q.s. | q.s. | q.s. | q.s. |
| pH adjuster | q.s. | q.s. | q.s. | q.s. | q.s. | q.s. |
| Perfume | q.s. | q.s. | q.s. | q.s. | q.s. | q.s. |
| Purified water | balance | balance | balance | balance | balance | balance |
| Total | 100 | 100 | 100 | 100 | 100 | 100 |

**Table 15 (continued)**

| (% by mass) | Prescription Example | | | | |
|---|---|---|---|---|---|
| | 22 | 23 | 24 | 25 | 26 |
| Prednisolone valerate acetate | | | | | |
| Diphenhydramine hydrochloride | | | 1 | | |
| Dexamethasone acetate | | | | | |
| Glycyrrhetinic acid | 0.1 | 0.5 | 0.5 | | |
| Isopropylmethylphenol | | | 0.1 | | |
| Crotamiton | | 5 | 5 | | |
| Benzethonium chloride | | | | | |
| Allantoin | | | 0.2 | | |
| Salicylic acid | | | | | |
| 1-Menthol | | 1 | | | |
| dl-Camphor | | | | | |
| Urea | | | | | |
| Sulfur | | | | | |
| Carpronium chloride hydrate | | | | | |
| Chlorhexidine hydrochloride | | | | | |
| Homosulfamine | | | | | |
| Acyclovir | | | | | |
| Vidarabine | | | | | |
| Trichlorocarbanilide | | | | | |
| Bifonazole | | | | | |
| Lidocaine | 2 | | | | |
| Oxiconazole nitrate | 1 | | | | |
| Lanoconazole | | 1 | | | |
| Chlorpheniramine maleate | | 0.5 | | | |
| Hydrocortisone acetate | | | 0.25 | | |
| Adapalene | | | | 0.1 | |
| Tacrolimus | | | | | 0.1 |
| Hypromellose phthalate | 1 | 1 | 1 | 1 | 0.3 |
| Acrylates/diacetoneacrylamide copolymer | 2 | 2 | | | 2 |
| Acrylic resin alkanolamine solution | | | 2 | 2 | |
| Isopropyl myristate | | | 0.5 | | |
| Polysorbate 80 | 1 | 1 | 1 | 1 | |
| 2-Amino-2-methylpropanol | 0.3 | 0.3 | | | 0.3 |
| 1,3-Butylene glycol | 1 | | | 1 | 1 |
| Triethanolamine | | | | | 0.6 |
| Carboxyvinyl polymer | | | | | 0.5 |
| Ethanol | 50 | 50 | 50 | 50 | 50 |
| Antiseptic | q.s. | q.s. | q.s. | q.s. | q.s. |
| pH adjuster | q.s. | q.s. | q.s. | q.s. | q.s. |
| Perfume | q.s. | q.s. | q.s. | q.s. | q.s. |
| Purified water | balance | balance | balance | balance | balance |
| Total | 100 | 100 | 100 | 100 | 100 |

**Table 16**

| (% by mass) | Prescription Example | | | |
|---|---|---|---|---|
| | 27 | 28 | 29 | 30 |
| Felbinac | 3 | 3 | | |
| Loxoprofen sodium hydrate | | | 1.13 | |
| Glycol salicylate | | | | 3 |
| 1-Menthol | 3 | 3 | 3 | 3 |
| Hypromellose phthalate | 0.3 | 0.3 | 0.3 | 0.3 |
| Acrylates/diacetoneacrylamide copolymer | 2.7 | 2.7 | 2.7 | 2.7 |
| Isopropyl myristate | 2 | 2 | 2 | 2 |
| Polysorbate 80 | 1 | 1 | 1 | 1 |
| 2-Amino-2-methylpropanol | 0.4 | 0.4 | 0.4 | 0.4 |
| Diisopropanolamine | 1 | 1 | | |
| Ethanol | 60 | 60 | 60 | 60 |
| Antiseptic | q.s. | q.s. | q.s. | q.s. |
| pH adjuster | q.s. | q.s. | q.s. | q.s. |
| Perfume | q.s. | q.s. | q.s. | q.s. |
| Purified water | balance | balance | balance | balance |
| Total | 100 | 100 | 100 | 100 |
| Mass ratio of aerosol stock solution and DME (stock solution:DME) | 70:30 | 80:20 | 80:20 | 80:20 |

### Industrial Applicability

According to the present invention, it is possible to provide a composition for external preparation which contains a medicinal ingredient and is capable of forming a coating film having excellent durability and moisture resistance, and a method for using the same.

## Claims

1. A composition for external preparation, comprising the following components (A) to (D):
(A) a medicinal ingredient;
(B) a water-insoluble polymer;
(C) a non-volatile base; and
(D) a volatile solvent,
wherein the component (B) comprises two or more selected from the group consisting of (B1) a cellulosic polymer, (B2) an acrylic polymer, and (B3) a vinyl polymer, and the composition for external preparation has a viscosity at 25°C of 1.0 mPa·s or more and 10,000 mPa·s or less.

2. The composition for external preparation according to claim 1, wherein a mass ratio [(C)/(B)] of the component (C) to the component (B) in the composition for external preparation is 0.01 or more and 50 or less.

3. The composition for external preparation according to claim 1 or 2, wherein the component (B) comprises the component (B1) and the component (B2), and a mass ratio [(B2)/(B1)] of the component (B2) to the component (B1) is 0.05 or more and 30 or less.

4. The composition for external preparation according to claim 1 or 2, wherein the component (B) comprises the component (B1) and the component (B3), and a mass ratio [(B3)/(B1)] of the component (B3) to the component (B1) is 0.1 or more and 20 or less.

5. The composition for external preparation according to claim 1 or 2, wherein the component (B) comprises the component (B2) and the component (B3), and a mass ratio [(B2)/(B3)] of the component (B2) to the component (B3) is 0.05 or more and 30 or less.

6. The composition for external preparation according to any one of claims 1 to 5, wherein the composition for external preparation further comprises water, and a content of water in the composition for external preparation is 1% by mass or more and 70% by mass or less.

7. The composition for external preparation according to any one of claims 1 to 6, wherein the component (A) comprises one or more selected from the group consisting of an anti-inflammatory analgesic component, a local irritation component, a blood circulation promoting component, an antihistaminic component, a crude drug component, an antipruritic component, a local anesthetic component, a keratin softening component, a bactericidal component, an antibacterial and antifungal component, an immunosuppressive agent, an antiviral component, a hair growth and hair restoration component, a sebum inhibitory component, an antiperspirant component, and a whitening component.

8. The composition for external preparation according to any one of claims 1 to 7, wherein the component (C) is one or more selected from the group consisting of a polar oil, a non-polar oil, a polyol, an alkanolamine, and a nonionic surfactant.

9. The composition for external preparation according to any one of claims 1 to 8, wherein the component (D) is an alcohol having 4 or less carbon atoms.

10. The composition for external preparation according to any one of claims 1 to 9, wherein a content of the component (A) in the composition for external preparation is 0.001% by mass or more and 30% by mass or less.

11. The composition for external preparation according to any one of claims 1 to 10, wherein a content of the component (B) in the composition for external preparation is 0.01% by mass or more and 30% by mass or less.

12. The composition for external preparation according to any one of claims 1 to 11, wherein a content of the component (C) in the composition for external preparation is 0.001% by mass or more and 40% by mass or less.

13. The composition for external preparation according to any one of claims 1 to 12, wherein a content of the component (D) in the composition for external preparation is 10% by mass or more and 95% by mass or less.

14. A method for using the composition for external preparation according to any one of claims 1 to 13, comprising the following steps (I) and (II) in this order:
step (I): applying the composition for external preparation to an object and then drying the composition to form a coating film composed of the composition for external preparation; and
step (II): bringing the coating film into contact with a cleaning agent and then washing with water to remove the coating film.
